(19) Europäisches Patentamt
European Patent Office
Office européen des brevets

(11) EP 4 563 573 A1

(12) EUROPEAN PATENT APPLICATION
published in accordance with Art. 153(4) EPC

(43) Date of publication:
**04.06.2025 Bulletin 2025/23**

(21) Application number: **23928433.4**

(22) Date of filing: **15.12.2023**

(51) International Patent Classification (IPC):
***C07D 277/34*** (2006.01) ***C07D 417/12*** (2006.01)
***C07D 401/12*** (2006.01) ***C07C 275/34*** (2006.01)
***C07C 275/26*** (2006.01) ***C07C 273/18*** (2006.01)
***A61P 29/00*** (2006.01) ***A61P 25/28*** (2006.01)
***A61P 25/24*** (2006.01) ***A61P 1/16*** (2006.01)
***A61P 13/12*** (2006.01) ***A61P 11/00*** (2006.01)
***A61P 9/12*** (2006.01) ***A61P 3/10*** (2006.01)
***A61P 31/00*** (2006.01)

(86) International application number:
**PCT/CN2023/139144**

(87) International publication number:
**WO 2024/193129 (26.09.2024 Gazette 2024/39)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.03.2023 CN 202310282598**

(71) Applicants:
- **Shenyang Pharmaceutical University**
  **Shenyang, Liaoning 110000 (CN)**
- **Wang, Gaohua**
  **Beijing 100070 (CN)**

(72) Inventors:
- **CHEN, Guoliang**
  **Shenyang, Liaoning 110000 (CN)**
- **WANG, Gaohua**
  **Beijing 100070 (CN)**
- **CAO, Ruolin**
  **Shenyang, Liaoning 110000 (CN)**
- **LIU, Zhongbo**
  **Shenyang, Liaoning 110000 (CN)**
- **ZHANG, Maoying**
  **Shenyang, Liaoning 110000 (CN)**
- **QI, Minggang**
  **Shenyang, Liaoning 110000 (CN)**
- **CHEN, Lu**
  **Shenyang, Liaoning 110000 (CN)**

(74) Representative: **Bryn Aarflot AS**
**Patent**
**Stortingsgata 8**
**0161 Oslo (NO)**

# (54) COMPOUND, AND PREPARATION METHOD THEREFOR AND USE THEREOF IN PREPARATION OF SEH INHIBITOR AND PPARS AGONIST

(57) The present application relates to the technical field of medicines, and in particular to a compound, and a preparation method therefor and a use thereof in the preparation of a soluble epoxide hydrolase (sEH) inhibitor and a peroxisome proliferators-activated receptors (PPARs) agonist. The present application provides a compound, having a structure represented by formula I, II, III, IV, V or VI. The compound provided by the present application has a typical urea structure as the primary pharmacophore of an sEH, and the thiazolidinedione part serves as the primary pharmacophore of PPARs. The sEH inhibitor and PPARs agonist compound provided by the present application has high inhibitory activity against human-derived HsEH and high agonistic activity against a PPAR, and can be used for the preparation of a drug for treating soluble epoxide enzyme and PPARs-mediated diseases.



Processed by Luminess, 75001 PARIS (FR)

## Description

The present application claims priority to the Chinese Patent Application CN202310282598.7 filed to the China National Intellectual Property Administration (CNIPA) on March 22, 2023 and entitled "COMPOUND, AND PREPARATION METHOD THEREOF AND USE THEREOF IN PREPARATION OF sEH INHIBITOR AND PPARs AGONIST", which is incorporated herein by reference in its entirety.

## TECHNICAL FIELD

The disclosure pertains to the technical field of medicines, and particularly to a compound, and a preparation method thereof and use thereof in preparation of a soluble epoxide hydrolase (sEH) inhibitor and a peroxisome proliferator-activated receptors (PPARs) agonist.

## BACKGROUND

The sensation of pain is mediated through the action of a specialized subset of sensory afferent neurons (also known as nociceptors) that are activated in response to thermal, mechanical, and chemical stimuli through a variety of mechanisms. Studies have shown that ion channel regulations including transient receptor potential (TRP) channels, G protein-coupled receptor (GPCR) activation, and cell membrane changes all indicate the mechanism of signal conducting by lipid mediator in nociceptors (Nature, 2001, 413(6852): 203-210).

Studies have also shown that prostaglandin and leukotriene, metabolites of cyclooxygenase and lipoxygenase, can cause pain and inflammation, thus proving the role of lipid mediators in conducting pain signals. Specific long-chain polyunsaturated fatty acids (PUFAs) are metabolized by cytochrome P450 enzyme (CYP450) to form an epoxide metabolite, namely epoxy fatty acid (EpFA). Researchers have found that these metabolites mediate analgesia in several types of pain pathologies, such as acute pain, chronic pain, cancerous pain, or intractable pain.

Arachidonic acid (ARA) is a 20-carbon PUPA with four unsaturated double bonds, and could be metabolized by the CYP450 enzyme into epoxide metabolites (epoxyeicosatrienoic acids, EETs) with any one or more of the four double bonds, including 5,6-EET, 8,9-EET, 11,12-EET, and 14,15-EET. EpFAs, including EETs, limit pain and inflammation through multiple direct and indirect mechanisms, including nuclear receptor agonism, limiting endoplasmic reticulum stress, and blocking mitochondrial dysfunction. Small molecule inhibitors of soluble epoxide hydrolase (sEH) show great analgesic effects in animal inflammatory pain and diabetic neuropathic pain models (Neurotherapeutics, 2020, 17, 900-916). EETs are easily metabolized by sEH *in vivo,* resulting in inactivation, and the dihydroxy metabolites of EETs, dihydroxyeicosatrienoic acids (DHETs), have inflammatory effects. The small molecule inhibitors of sEH, which could stabilize EpFA *in vivo,* increasing the amount of EETs *in vivo* by inhibiting sEH activity, has become a novel approach for treating EETs-related diseases.

EpFA exerts analgesic effects through multiple mechanisms, such as alleviating endoplasmic reticulum (ER) stress, preventing or reversing endothelial cell dysfunction (ECD), and stabilizing mitochondrial functions (Cell Physiol Biochem, 2015, 36, 474-486). EpFA could modulate cellular stress caused by reactive oxygen species (ROS) and shift the ER stress response to maintain homeostasis rather than activate inflammatory pathways to cause cellular senescence and apoptosis. EpFA could reduce ER stress response and limit ROS, indirectly maintaining the stability of mitochondrial functions. EpFA could also directly block the impairment of mitochondrial functions. Inhibition of sEH activity could stabilize EpFA and also limit the production of some pro-inflammatory diol metabolites. Accordingly, the EpFA mediates beneficial effects in all these processes, shifting the ER stress response toward homeostasis and alleviating pain.

There are a lot of evidences suggesting a role for EpFA in nociception including blocking inflammatory and neuropathic pain, and thus sEH inhibitors and EpFA mimetics exhibit a great potential for alleviating pain in human beings.

Epidemiological studies have confirmed that inflammatory biomarkers are associated with the development of type II diabetes mellitus (T2DM) and its complications. The triggering mechanisms of inflammation still remain unclear in T2DM. Inflammatory responses may contribute to the development of T2DM by causing insulin resistance, while an obesity environment leads to adipose tissue dysfunction, macrophage infiltration, and greater release of cytokines such as IL-6 and TNF-$\alpha$. Chronically elevated levels of these molecules promote insulin resistance in skeletal muscle and endothelial dysfunction in the vascular system, as well as release of acute phase proteins from the liver. Chronic elevation of specific inflammatory markers such as IL-6 and TNF-$\alpha$ appears to be associated with metabolic disturbances and could alter insulin sensitivity by triggering different key steps in the insulin signaling pathway. Hyperglycemia could also induce endothelial cells and macrophages to produce IL-6. Furthermore, the hyperglycemia enhances the effects of suppressors of cytokine signaling (SOCS), thereby impairing insulin release and signaling and then promoting long-term complications of diabetes. Targeting inflammatory pathways may be a part of strategies to prevent and control diabetes and related complications thereof.

PPARs agonists have been shown to inhibit the expression of cytokines, such as resistin, tumor necrosis factor $\alpha$

(TNF-α), and interleukin 6, which promote insulin resistance. PPARs agonists induce an increase in adiponectin concentration in plasma, a hormone that is secreted from adipose tissues and present at a low level in the plasma of T2DM patients. Adiponectin increases fatty acid oxidation in liver and skeletal muscle. Overall, adiponectin improves insulin sensitivity in skeletal muscle and liver and reduces glucose production in the liver, thereby lowering circulating free fatty acids (FFAs) and triglycerides (TG) as well as glucose levels. Macrophage infiltration in obesity adipose tissues participates in local inflammation that enhances insulin resistance. PPARs in macrophages are recently proved to partially mediate the antidiabetic effects of thiazolidinediones (TZDs). Inactivation of PPARs in macrophages results in impairment of alternative macrophage activation, glucose intolerance, and insulin resistance in skeletal muscle and liver.

At present, the analgesics used in clinical practice mainly include opioid analgesics and non-steroidal anti-inflammatory drugs, both of which have certain side effects. For example, traditional opioid analgesics have strong effects, but they also show strong addictiveness as well as side effects such as respiratory depression, hypotension, nausea, vomiting, constipation and dysuria. Non-steroidal anti-inflammatory drugs are divided into non-selective non-steroidal anti-inflammatory drugs and selective cyclooxygenase-2 (COX-2) inhibitors. Although showing desirable analgesic effects, the non-selective non-steroidal anti-inflammatory drugs could lead to relatively serious gastrointestinal irritation that easily results in gastric ulcers, and generally have adverse reactions to the coagulation and hematopoietic systems. Although the selective COX-2 inhibitors do not result in adverse reactions of gastrointestinal irritation, they are prone to cause an imbalance of prostacyclin and thromboxane, thereby leading to cardiovascular diseases, and they also generally have little effect on neuropathic pain.

Moreover, the hypoglycemic drugs currently used in clinical practice mainly include insulin, insulin secretion enhancers, insulin sensitizing agents, α-glucosidase inhibitors, glucagon-like peptide (GLP)-1 agonists, and dipeptidyl peptidase-4 inhibitors, all of which have certain disadvantages. For example, the insulin and α-glucosidase inhibitors cannot be used alone to treat diabetes, and always need to be combined with other drugs for blood glucose control. The insulin secretion enhancers would cause gastrointestinal symptoms (such as nausea and upper abdominal distension), headaches, poor compliance of patients who must be injected with insulin for treatment, difficulty in combating diabetic inflammatory complications with other drugs, and concurrent neuropathic pain.

A dual-target compound RB394 for sEH inhibitor and PPARs agonist has been reported so far, showing sEH $IC_{50}$ = 0.3 μM and PPARγ $EC_{50}$ = 0.3 μM, and has exhibited certain therapeutic effects in the fields of diabetic nephropathy and fatty liver hepatitis, but still needs further development and exploration. The dual-target compound for sEH inhibitor and PPARs agonist could not only lower blood glucose, but also be effective for diabetic inflammatory complications, nonalcoholic steatohepatitis (NASH), and neuropathic pain, thereby reducing the drug-drug interactions when patients previously need to take multiple medications at the same time. As a result, it is urgent and necessary to develop a novel and more efficient dual-target compound for sEH inhibitor and PPARs agonist for the treatment of diabetic NASH, pain, and depression.

## SUMMARY

An object of the present disclosure is to provide a compound, and a preparation method thereof and use thereof in preparation of an sEH inhibitor and a PPARs agonist. In the present disclosure, the compound has high activity against both sEH and PPARs and shows small side effects, and could be used as a dual-target compound for the sEH inhibitor and the PPARs agonist in preparation of a drug for treating diabetes and inflammatory complications thereof, neuropathic pain, and depression.

The present disclosure provides a compound having a structure shown in one selected from the group consisting of Formulas I, II, III, IV, V, and VI:

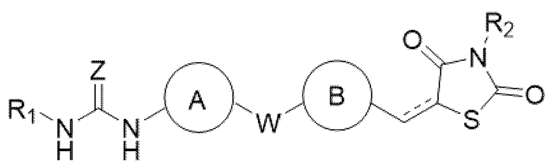

Formula I;

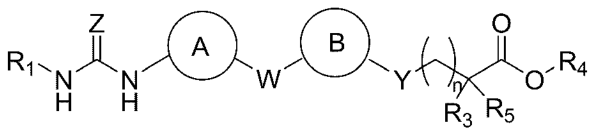

Formula II;

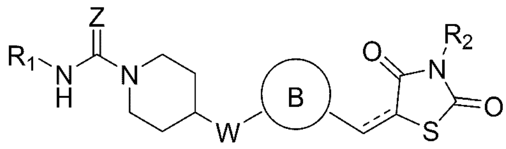 Formula III;

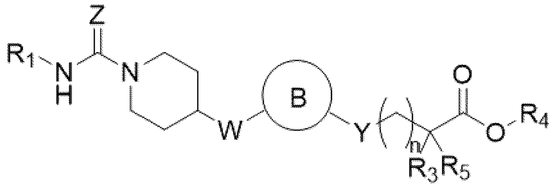 Formula IV;

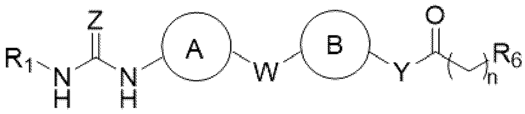 Formula V;

and

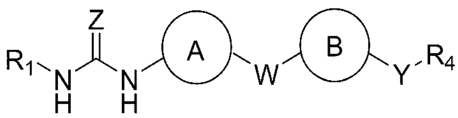 Formula VI;

where

$R_1$ is selected from the group consisting of adamantyl, aryl, alkyl-substituted aryl, halogenated aryl, haloalkyl-substituted aryl, haloalkoxy-substituted aryl, and haloaryloxy-substituted aryl; $R_2$ is selected from the group consisting of hydrogen, hydroxyl, alcoholic hydroxyl, amino, carboxyl, acyl, amido, and ester group; $R_3$ is selected from the group consisting of hydrogen, alkyl, alkoxy, hydroxyl, cyano, and carboxyl; $R_4$ is selected from the group consisting of hydrogen and alkyl; $R_5$ is selected from the group consisting of hydrogen, alkyl, alkoxy, hydroxyl, cyano, and carboxyl; $R_6$ is selected from the group consisting of hydrogen, hydroxyl, alkyl, ester group, and amino; A is selected from the group consisting of cycloalkyl, a heterocyclic group, and aryl, and B is selected from the group consisting of a single bond, cycloalkyl, a heterocyclic group, and aryl;

W is selected from the group consisting of a single bond, $-CH_2-$, -O-, -S-, -NH-, and

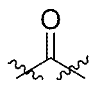 ;

Y is selected from the group consisting of a single bond, $-CH_2-$, -O-, -S-, and -NH-; Z is selected from the group consisting of $=CH_2$, =O, =S, and =NH; and n is an integer of 0 to 12.

The present disclosure provides a compound having a structure shown in Formula I, II, III, IV, V, or VI. The compound has a typical urea structure as a primary pharmacophore of sEH and a thiazolidinedione moiety as a primary pharmacophore of PPARs. The compound for the sEH inhibitor and the PPARs agonist shows a high inhibitory activity against human soluble epoxide hydrolase (HsEH) and a high agonist activity against the PPARs. Therefore, the compound for the sEH inhibitor and the PPARs agonist could be prepared into a drug for treating sEH-mediated and PPARs-mediated diseases.

## BRIEF DESCRIPTION OF THE DRAWINGS

FIG. 1 shows a reaction route for preparing a compound having a structure shown in Formula I for an sEH inhibitor and a PPARs agonist in the present disclosure.

FIG. 2 shows a reaction route for preparing a compound having a structure shown in Formula II for an sEH inhibitor and

a PPARs agonist in the present disclosure.

FIG. 3 shows a reaction route for preparing a compound having a structure shown in Formula III for an sEH inhibitor and a PPARs agonist in the present disclosure.

FIG. 4 shows a reaction route for preparing a compound having a structure shown in Formula IV where $R_3$ is halogen for an sEH inhibitor and a PPARs agonist in the present disclosure.

FIG. 5 shows a reaction route for preparing a compound having a structure shown in Formula V for an sEH inhibitor and a PPARs agonist in the present disclosure.

FIG. 6 shows an influence of the compound SP-B07 prepared in an example on the body weight of KM mice.

FIG. 7 shows an influence of the compound SP-C01 prepared in an example on the body weight of KM mice.

FIG. 8 shows an influence of the compound SP-C01 prepared in an example on a visceral index of KM mice.

FIG. 9 shows representative H&E staining sections in the influence of the compound SP-C01 prepared in an example on the organs of KM mice.

FIG. 10 shows evaluation of the compound SP-B07 prepared in an example for treating arthritis induced by complete Freund's adjuvant (CFA) in an adjuvant-induced arthritis (AIA) mouse model.

FIG. 11 shows evaluation of the compound SP-C01 prepared in an example for treating arthritis induced by CFA in an AIA mouse model.

FIG. 12 shows evaluation of the compound SP-C01 prepared in an example for treating arthritis induced by CFA in an AIA mouse model.

FIG. 13 shows a survival rate of C57Bl/6 mice in lipopolysaccharide (LPS)-induced inflammation post treatment with the compound SP-B07 prepared in an example.

FIG. 14 shows expression levels of COX-2, sEH, NOS2, and VCAM in mouse plasma detected by Western blot post treatment with the compound SP-B07 prepared in an example.

FIG. 15 shows IL-6, MCP-5, and TNF-$\alpha$ inflammatory factors in mouse plasma detected by Elisa post treatment with the compound SP-B07 prepared in an example.

FIG. 16 shows that SP-C01 has blocked pain measured by the von Frey test (mechanical withdrawal threshold) in a chronic constriction injury model of neuropathy in male SD rats.

FIG. 17 shows results of histological analysis on pancreas of mice treated with control, Mod, celecoxib, ulinastatin, and the compound SP-C01, where representative H&E staining sections of the pancreas are shown in the *in vivo* efficacy study.

FIG. 18 shows results of histological analysis on pancreas of mice treated with control, Mod, celecoxib, ulinastatin, and the compound SP-C01.

FIG. 19 shows representative H&E staining sections in the influence of the compound SP-B07 prepared in an example on the organs of KM mice.

FIG. 20 shows results of blood glucose analysis of mice treated with control, Mod, pioglitazone, the compound SP-B07, and the compound SP-C01.

FIG. 21 shows results of diabetic neuralgia analysis in mice treated with control, Mod, pioglitazone, the compound SP-B07, and the compound SP-C01.

FIG. 22 shows a reaction route for preparing a compound having a structure shown in Formula VI for an sEH inhibitor and a PPARs agonist in the present disclosure.

FIG. 23 shows a reaction route for preparing a compound having a structure shown in Formula V where Y is -NH- for an sEH inhibitor and a PPARs agonist in the present disclosure.

DETAILED DESCRIPTION OF THE EMBODIMENTS

The present disclosure provides a compound having a structure shown in Formula I, II, III, IV, V, or VI:

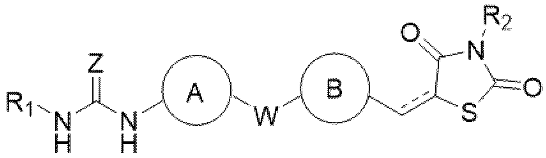

Formula I;

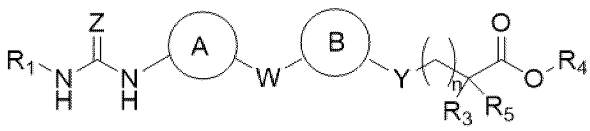

Formula II;

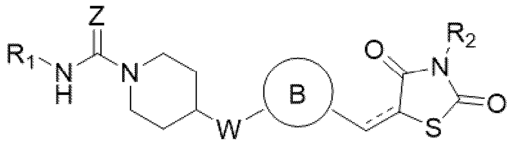
Formula III;

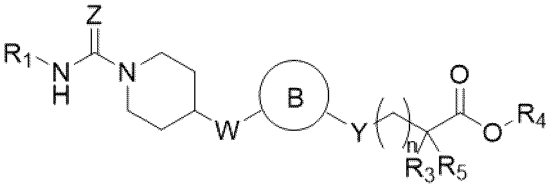
Formula IV;

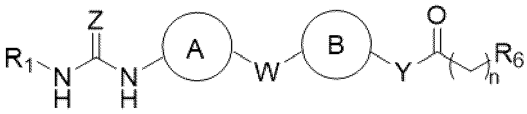
Formula V;

and

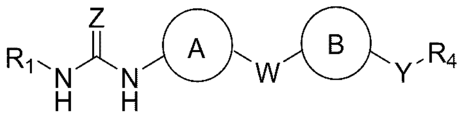
Formula VI;

where

$R_1$ is selected from the group consisting of adamantyl, aryl, alkyl-substituted aryl, halogenated aryl, haloalkyl-substituted aryl, haloalkoxy-substituted aryl, and haloaryloxy-substituted aryl; $R_2$ is selected from the group consisting of hydrogen, hydroxyl, alcoholic hydroxyl, amino, carboxyl, acyl, amido, and ester group; $R_3$ is selected from the group consisting of hydrogen, alkyl, alkoxy, hydroxyl, cyano, and carboxyl; $R_4$ is selected from the group consisting of hydrogen and alkyl; $R_5$ is selected from the group consisting of hydrogen, alkyl, alkoxy, hydroxyl, cyano, and carboxyl; $R_6$ is selected from the group consisting of hydrogen, hydroxyl, alkyl, ester group, and amino; A is selected from the group consisting of cycloalkyl, a heterocyclic group, and aryl, and B is selected from the group consisting of a single bond, cycloalkyl, a heterocyclic group, and aryl;

W is selected from the group consisting of a single bond, $-CH_2-$, -O-, -S-, -NH-, and

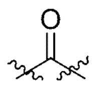
;

Y is selected from the group consisting of a single bond, $-CH_2-$, -O-, -S-, and -NH-; Z is selected from the group consisting of $=CH_2$, =O, =S, and =NH; and n is an integer of 0 to 12.

In some embodiments, the present disclosure, alkyl in the alkyl-substituted aryl and the haloalkyl-substituted aryl is independently selected from the group consisting of methyl, ethyl, propyl, butyl, pentyl, isobutyl, isopropyl, isopentyl, and tert-butyl. In some embodiments, alkoxy in the haloalkoxy-substituted aryl is selected from the group consisting of methoxy, ethoxy, propoxy, isopropoxy, butoxy, cyclopentyloxy, cyclohexyloxy, phenoxy, and benzyloxy. In some embodiments, halogen in the halogenated aryl, the haloalkyl-substituted aryl, and the haloalkoxy-substituted aryl is preferably independently selected from the group consisting of -F, -Cl, and -Br.

In some embodiments of the present disclosure, the cycloalkyl is unsubstituted or substituted C3 to C8 cycloalkyl; preferably, a substituent of the substituted C3 to C8 cycloalkyl is independently selected from the group consisting of -F, -Cl, -Br, -OH, $-NH_2$, $-NHCH_3$, - $N(CH_3)_2$, and C1 to C6 alkyl; the heterocyclic group is independently an unsubstituted or substituted 3- to 10-membered heterocyclic group; a substituent of the substituted 3- to 10-membered heterocyclic group is preferably independently selected from the group consisting of - F, -Cl, -Br, -OH, $-NH_2$, $-NHCH_3$, $-N(CH_3)_2$, and C1 to C6 alkyl; the aryl is independently selected from the group consisting of substituted or unsubstituted phenyl, substituted or unsubstituted pyridyl, and substituted or unsubstituted naphthyl; and a substituent of the substituted phenyl, the substituted pyridyl, or the substituted naphthyl is preferably independently selected from the group consisting of -F,

-Cl, -Br, -OH, $-NH_2$, $-NHCH_3$, $-N(CH_3)_2$, and C1 to C6 alkyl.

In some embodiments of the present disclosure, $R_1$ is selected from the group consisting of adamantyl, halogenated aryl, haloalkyl-substituted aryl, and haloalkoxy-substituted aryl; $R_2$ is preferably selected from the group consisting of hydrogen and ester group; $R_3$ is preferably selected from the group consisting of hydrogen and alkyl; $R_4$ is preferably selected from the group consisting of hydrogen, methyl, and ethyl; $R_5$ is preferably selected from the group consisting of hydrogen and alkyl; $R_6$ is preferably selected from the group consisting of hydroxyl, amino, alkyl, and ester group; A is preferably selected from the group consisting of cyclohexyl and phenyl, and B is preferably selected from the group consisting of single bond and phenyl; W is preferably selected from the group consisting of single bond and -O-; Y is preferably selected from the group consisting of single bond, -O-, and -NH-; Z is =O; and n is preferably an integer of 0 to 2.

In some embodiments of the present disclosure, $R_1$ is selected from the group consisting of adamantyl,

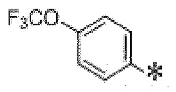

,

and

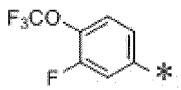

;

$R_2$ is preferably selected from the group consisting of hydrogen and $-CH_2-C(O)-CH_3$; $R_3$ is preferably selected from the group consisting of hydrogen and methyl; $R_4$ is preferably hydrogen; $R_5$ is preferably selected from the group consisting of hydrogen and methyl; and $R_6$ is preferably selected from the group consisting of hydroxyl, $-NH_2$, methyl, $-C(O)-O-CH_2-CH_3$, and sec-butyl.

In some embodiments of the present disclosure,

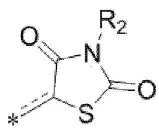

in Formula 1 and Formula 3 is

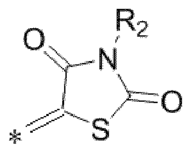

or

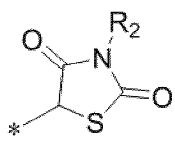

.

In some embodiments of the present disclosure, the compound has any one of the following structures:

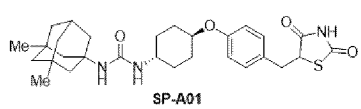

SP-A01

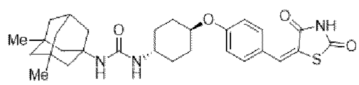

SP-B01

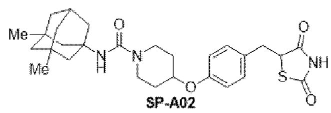

SP-A02

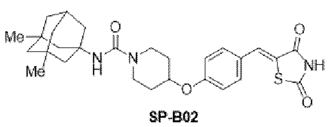

SP-B02

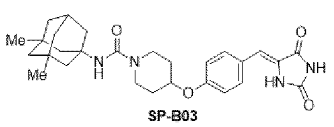

SP-B03

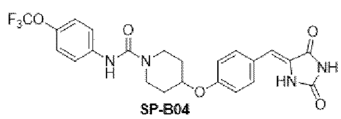

SP-B04

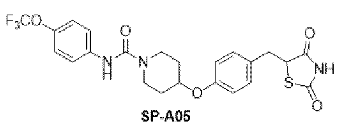

SP-A05

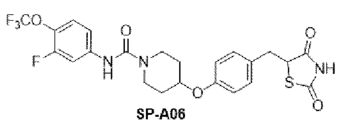

SP-A06

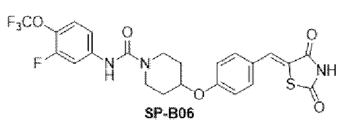

SP-B06

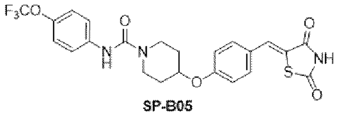

SP-B05

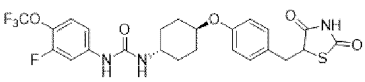

SP-A07

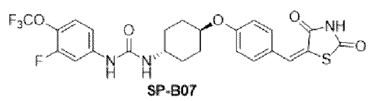

SP-B07

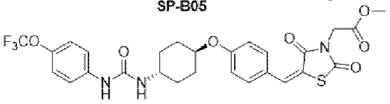

SP-D01

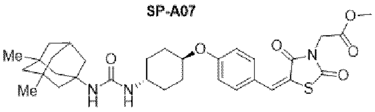

SP-D04

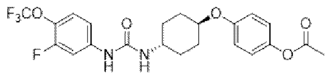

SP-C01

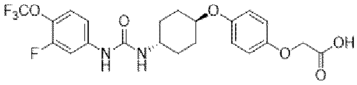

SP-C02

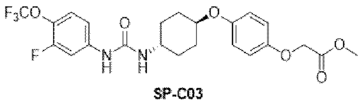

SP-C03

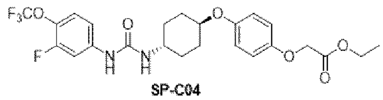

SP-C04

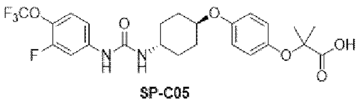

SP-C05

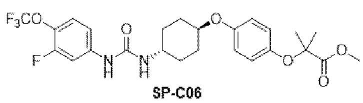

SP-C06

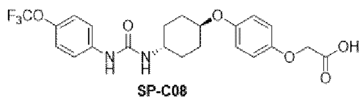

SP-C08

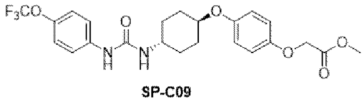

SP-C09

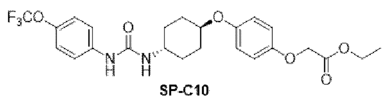

SP-C10

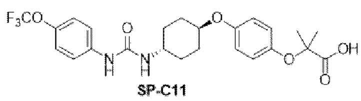

SP-C11

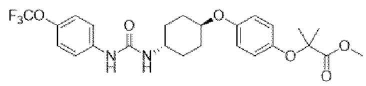

SP-C12

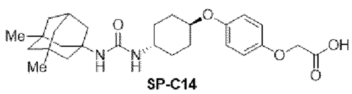

SP-C14

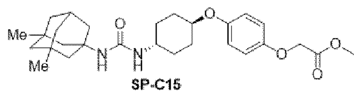

SP-C15

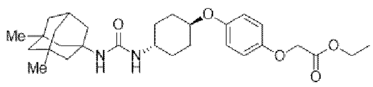

SP-C16

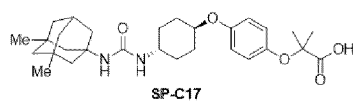

SP-C17

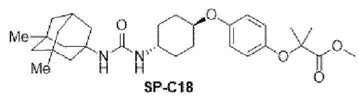

SP-C18

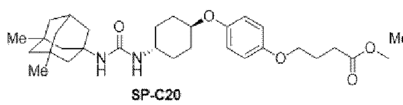

SP-C20

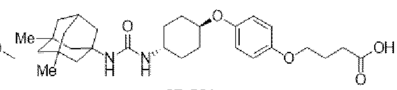

SP-C21

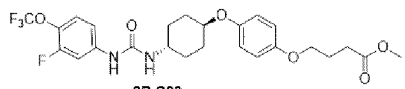

SP-C23

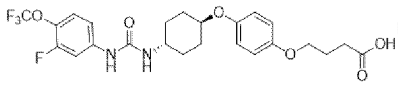

SP-C24

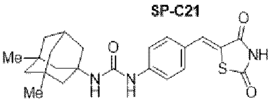

SP-E01

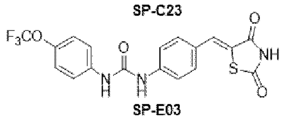

SP-E03

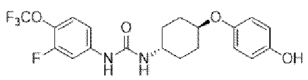

SP-C01b

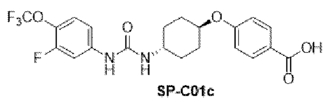

SP-C01c

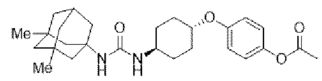

SP-C01d

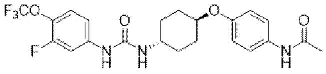

SP-C01e

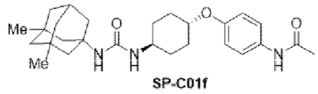

SP-C01f

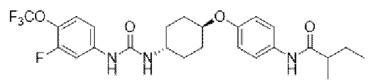

SP-C01g

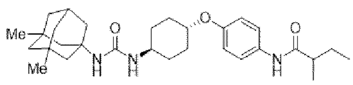

SP-C01h

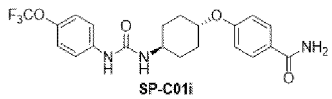

SP-C01i

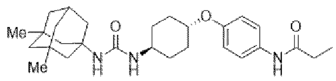

SP-C01j

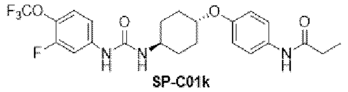

SP-C01k

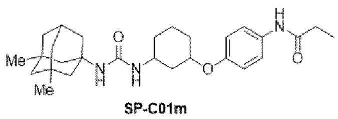

SP-C01m

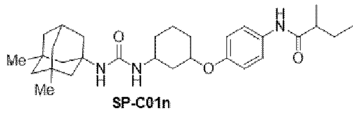

SP-C01n

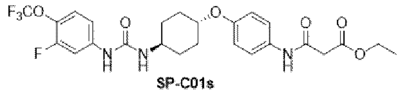

SP-C01s

In the present disclosure, chemical names of the compounds of the specific structures correspond to: 1-[(1r,3R,5S,7R)-3,5-dimethyladamantan-1-yl]-3-((1r,4R)-4-{4-[(2,4-dioxythiazolidin-5-yl)methyl]phenoxy}cyclohexyl) urea, 1-[(1r,3R,5S,7R)-3,5-dimethyladamantan-1-yl]-3-((1r,4R)-4-(4-{[(E)-(2,4-dioxythiazolin-5-ylidene)methyl]phenoxy}cyclohexyl)urea, *N*-[(1r,3R,5S,7r)-3,5-dimethyladamantan-1-yl]-4-{4-[(Z)-(2,4-dioxythiazolidin-5-ylidene)methyl] phenoxy}piperidin-1-carboxamide, *N*-[(1r,3R,5S,7r)-3,5-dimethyladamantan-1-yl]-4-{4-[(2,4-dioxothiazolidin-5-yl) methyl]phenoxy}piperidin-1-carboxamide, *N*-[(1r,3R,5S,7r)-3,5-dimethyladamantan-1-yl]-4-{4-[(Z)-(2,5-dioxyimidazolin-4-ylidene)methyl[phenoxy}piperidin-1-carboxamide, (Z)-4-[4-(2,5-dioxoimidazolin-4-ylidene)methyl]phenoxy-N-[4-(trifluoromethoxy)phenyl]piperidin-1-carboxamide, (Z)-4-{4-[(2,4-dioxothiazolidin-5-ylidene)methyl]phenoxy}-N-[4-(trifluoromethoxy)phenyl]piperidin-1-carboxamide, 4-[4-(2,4-dioxothiazolidin-5-yl)methyl[phenoxy-N-[4-(trifluoromethoxy)phenyl]piperidin-1-carboxamide, (Z)-4-4-{[2, 4-dioxothiazolidin-5-ylidene)methyl]phenoxy}-N-[3-fluoro-4-(trifluoromethoxy)phenyl]piperidin-1-carboxamide, 4-{4-[(2,4-dioxothiazolidin-5-yl)methyl]phenoxy}-N-[3-fluoro-4-(trifluoromethoxy)phenyl]piperidin-1-carboxamide, 1-((1r,4r)-4-(4-{(E)-[(2,4-dioxothiazolidin-5-ylidene)methyl]phenoxy }cyclohexyl)-3-[3-fluoro-4-(trifluoromethoxy)phenyl urea, 1-((1r,4r)-4-{4-[(2,4-dioxothiazolidin-5-yl)methyl]phenoxy}cyclohexyl)-3-[3-fluoro-4-(trifluoromethoxy)phenyl urea, 2-(4-{(1r,4r)-4-[3-(3-fluoro-4-trifluoromethoxyphenyl)ureido]cyclohexyl} oxy)phenoxyacetic acid, methyl 2-(4-{(1r,4r)-4-[3-(3-fluoro-4-trifluoromethoxyphenyl)ureido]cyclohexyl}oxy)phenoxy) acetate, ethyl 2-(4-{(1r,4r)-4-[3-(3-fluoro-4-trifluoromethoxyphenyl)ureido]cyclohexyl}oxy)phenoxy)acetate, 2-(4-{(1r,4r)-4-[3-(3-fluoro-4-trifluoromethoxyphenyl)ureido] cyclohexyl } oxy)phenoxy)-2-methylpropanoic acid, methyl 2-(4-{(1r,4r)-4-[3-(3-fluoro-4-trifluoromethoxyphenyl)ureido]cyclohexyl}oxy)phenoxy)-2-methylpropanoate, 2-(4-{(1r,4r)-4-[3-(4-trifluoromethoxyphenyl)ureido]cyclohexyl)oxy}phenoxy)acetic acid, methyl 2-(4-{(1r,4r)-4-[3-(4-trifluoromethoxyphenyl)ureido]cyclohexyl}oxy)phenoxy) acetate, ethyl 2-(4-{(1r,4r)-4-[3-(4-trifluoromethoxyphenyl)ureido]cyclohexyl}oxy)phenoxy)acetate, 2-methyl-2-(4-((1r,4r)-4{[3-(4-trifluoromethoxyphenylureido)cyclohexyl]oxy}phenoxy)propanoic acid, methyl 2-methyl-2-(4-{(1r,4r)-4-[3-(4-trifluoromethoxyphenyl)ureido]cyclohexyl}oxy)phenoxy)propionate, 2-[4-((1R,4r)-4-{3-[(1R,3R,5S,7R)-3,5-dimethyladamantan-1-yl]ureido}cyclohexyl)oxy]phenoxy)acetic acid, methyl 2-[4-((1R,4r)-4{3-[(1R,3R,5S,7R)-3,5-dimethyladamantan-1-yl]ureido}cyclohexyl)oxy]phenoxy)acetate, ethyl 2-[4-((1R,4r)-4{3-[(1R,3R,5S,7R)-3,5-dimethyladamantan-1-yl]ureido}cyclohexyl)oxy]phenoxy)acetate, 2-[4-((1R,4r)-4-{3-[(1R,3R,5S,7R)-3,5-dimethyladamantan-1-yl]ureido}cyclohexyl)oxy]phenoxy)-2-methylpropanoic acid, methyl 2-[4-((1R,4r)-4-{3-[(1R,3R,5S,7R)-3,5-dimethyladamantan-1-yl]ureido}cyclohexyl)oxy]phenoxy)-2-methylpropanoate, methyl 2-(2,4-dioxo-5-((E)-4-(((1r,4r)-4-)3-(4-(trifluoromethoxy)phenyl)ureido)cyclohexyl)oxy)benzylidene) thiazolidin-3-yl)acetate, methyl 2-(5-((E)-4-(((1R,4r)-4-)(3-((1R,3R,5S,7R)-3,5-dimethyladamantan-1-yl)ureido)cyclohexyl)oxy)benzylidene)-2,4-dioxothiazolidin-3-yl)acetate, phenyl 4-(((1r,4r)-4-(3-(3-fluoro-4-(trifluoromethoxy)phenyl) ureido)cyclohexyl)oxy)acetate, methyl 4-(4-(((1R,4r)-4-(3-((1R,3R,5S,7R)-3,5-dimethyladamantan-1-yl)ureido)cyclohexyl)oxy)phenoxy)butyrate, 4-(4-(((1R,4r)-4-(3-((1R,3R,5S,7R)-3,5-dimethyladamantan-1-yl)ureido)cyclohexyl)oxy) phenoxy)butyric acid, methyl 4-(4-(((1r,4r)-4-(3-(3-fluoro-4-(trifluoromethoxy)phenyl)ureido)cyclohexyl)oxy)phenoxy)butyrate, 4-(4-(((1r,4r)-4-(3-(3-fluoro-4-(trifluoromethoxy)phenyl)ureido)cyclohexyl)oxy)phenoxy)butyric acid, 1-((1r,3R,5S,7r)-3,5-dimethyladamantan-1-yl)-3-(4-((Z)-(2,4-dioxothiazolidin-5-ylidene)methyl)phenyl)urea, (Z)-1-(4-((2,4-dioxothiazolidin-5-ylidene)methyl)phenyl)-3-(4-(trifluoromethoxy)phenyl)urea, 1-(3-fluoro-4-(trifluoromethoxy)phenyl)-3-((1r,4r)-4-(4-hydroxyphenoxy)cyclohexyl)urea, 4-(((1r,4r)-4-(3-(3-fluoro-4-(trifluoromethoxy)phenyl) ureido)cyclohexyl)oxy)benzoic acid, phenyl 4-(((1R,4r)-4-(3-((1R,3R,5S,7R)-3,5-dimethyladamantan-1-yl)ureido)cyclohexyl)oxy)acetate, N-(4-(((1r,4r)-4-(3-(3-fluoro-4-(trifluoromethoxy)phenyl)ureido)cyclohexyl)oxy)phenyl)acetamide, N-(4-(((1R,4r)-4-(3-((1R,3R,5S,7R)-3,5-dimethyladamantan-1-yl)ureido)cyclohexyl)oxy)phenyl)acetamide, *N*-(4-(((1r,4r)-4-(3-(3-fluoro-4-(trifluoromethoxy)phenyl)ureido)cyclohexyl)oxy)phenyl)-2-methylbutanamide, *N*-(4-(((1r,4r)-4-(3-(3-fluoro-4-(trifluoromethoxy)phenyl)ureido)cyclohexyl)oxy)phenyl)-2-methylbutanamide, 4-(((1r,4r)-4-(3-(4-(trifluoromethoxy)phenyl)ureido)cyclohexyl)oxy)benzamide, *N*-(4-(((1R,4r)-4-(3-((1R,3R,5S,7R)-3,5-dimethyladamantan-1-yl)ureido)cyclohexyl)oxy)phenyl)propanamide, *N*-(4-(((1r,4r)-4-(3-(3-fluoro-4-(trifluoromethoxy) phenyl)ureido)cyclohexyl)oxy)phenyl)propanamide, N-(4-((3-(3-((1r,3R,5S,7r)-3,5-dimethyladamantan-1-yl)ureido)cyclohexyl)oxy)phenyl)propanamide, *N*-(4-((3-(3-((1r,3R,5S,7r)-3,5-dimethyladamantan-1-yl)ureido)cyclohexyl)oxy)phenyl)-2-methylbutanamide, ethyl 3-((4-(((1r,4r)-4-(3-(3-fluoro-4-(trifluoromethoxy)phenyl)ureido)cyclohexyl)oxy)phenyl) amino)-3-oxo-propionate, 3-((4-(((1r,4r)-4-(3-(3-fluoro-4-(trifluoromethoxy)phenyl)ureido)cyclohexyl)oxy)phenyl)amino)-3-oxo-propanoic acid.

The present disclosure further provides a method for preparing the compound as described in above technical solutions, where

(1) A method for preparing the compound having a structure shown in Formula I includes the following steps:

subjecting a compound a and a compound b to a first substitution to obtain a compound c;
subjecting the compound c and a compound d to a first condensation to obtain a compound e;
subjecting the compound e to a first hydrolysis to obtain a compound f;
subjecting the compound f, a compound w, and a compound af to a first nucleophilic substitution to obtain a compound g; where the compound g is the compound having a structure shown in Formula I where a thiazolidinedione group is linked to a double bond; and
subjecting the compound g to a first reduction to obtain the compound having a structure shown in Formula I where the thiazolidinedione group is linked to a single bond; where
the compound a, the compound b, the compound c, the compound d, the compound e, the compound f, the compound g, the compound w, and the compound af have the following structural formulas:

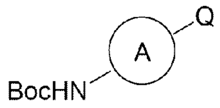
Compound a

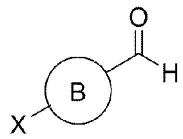
Compound b

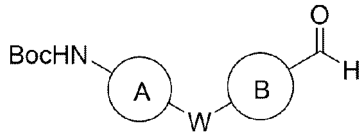
Compound c

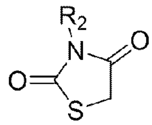
Compound d

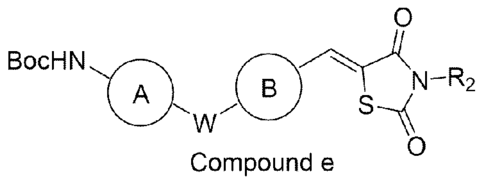
Compound e

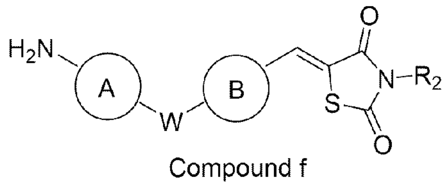
Compound f

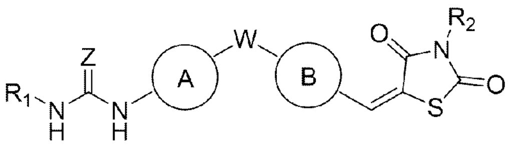
Compound g

$R_1-NH_2$
Compound w

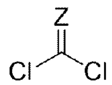
Compound af

;

Q in the compound a is selected from the group consisting of H, hydroxyl, amino, sulfhydryl, carboxyl, and acyl chloride; and X in the compound b is selected from the group consisting of H, hydroxyl, halogen, and haloalkyl.

As shown in the reaction route of FIG. 1, the compound a and the compound b are subjected to nucleophilic substitution to obtain a compound c. In some embodiments, the nucleophilic substitution is conducted in the presence of triphenylphosphine (TPP) and diisopropyl azodicarboxylate (DIAD). In some embodiments, the nucleophilic substitution is conducted in an ice-salt bath at -10°Cfor preferably 8 h to 12 h. In some embodiments, a molar ratio of the compound a, the compound b, the TPP, and the DIAD is 1: 1: 1.5: 1.5. In some embodiments, a solvent for the nucleophilic substitution is tetrahydrofuran (THF), and a product obtained after the nucleophilic substitution is purified by column chromatography.

In the present disclosure, the compound c and the compound d are subjected to Claisen-Schmidt condensation to obtain the compound e. In some embodiments, the condensation is conducted in the presence of pyridine (or piperidine) and acetic acid (or benzoic acid). In some embodiments, a molar ratio of the compound c, the compound d, the alkali, and the acid is 1: 1: 0.5: 0.5. In some embodiments, the nucleophilic substitution is conducted at 110°C, a solvent for the condensation is preferably toluene, and the condensation is conducted for preferably 4 h to 8 h. After the condensation, a resulting reaction mixture is cooled to room temperature and subjected to filtration suction.

In the present disclosure, after obtaining the compound e, the compound e is subjected to deprotection under an acidic condition to obtain the compound f. In some embodiments, a reagent providing the acidic condition is a solution of trifluoroacetic acid (TFA) or HCl in ethyl acetate, and the deprotection is preferably conducted in the presence of dichloromethane (DCM). In some embodiments, the deprotection is conducted at room temperature, preferably for 1.5 h to 2.5 h. In some embodiments, a reaction solution obtained by the deprotection is subjected to vacuum distillation, and water and DCM are added thereto; a pH value of the obtained system is adjusted to 10 with solid sodium hydroxide in

an ice-water bath; an organic layer therein is separated and removed, and an aqueous layer is extracted with DCM (100 mL×2), followed by washing with water, washing with saturated NaCl solution, and drying over anhydrous sodium sulfate, filtering by suction in sequence, and a resulting filtrate is concentrated under reduced pressure to obtain the compound f.

In the present disclosure, after obtaining the compound f, the compound f and the compound af undergo nucleophilic substitution to obtain the compound g. In some embodiments, the nucleophilic substitution is conducted in the presence of triethylamine (TEA). In some embodiments, a molar ratio of the compound f, solid phosgene, and an alkali is in a range of 1 : (0.33-0.6) : 3, and preferably 1 : (0.4-0.5) : 3. In some embodiments, the nucleophilic substitution is conducted in an ice bath for preferably 10 min to 50 min, more preferably 30 min. After the nucleophilic substitution is completed, a resulting product is purified by column chromatography to obtain the compound g.

In the present disclosure, after obtaining the compound g, the compound g is subjected to a first reduction to obtain the compound having a structure shown in Formula I where the thiazolidinedione group is linked to a single bond. In some embodiments, the first reduction is conducted in the presence of hydrogen gas and palladium/carbon. In some embodiments, a molar ratio of the compound g to the palladium/carbon is 1 : 0.1. In some embodiments, the catalytic hydrogenation is conducted at room temperature preferably for 240 min to obtain the compound having a structure shown in Formula I for an sEH inhibitor and a PPARs agonist.

(2) A method for the compound having a structure shown in Formula II includes the following steps:

subjecting the compound a and a compound h to a first substitution to obtain a compound i;
subjecting the compound i to a first hydrolysis to obtain a compound j;
subjecting the compound j and a compound aa to a second substitution to obtain a compound k;
subjecting the compound k to a second hydrolysis to obtain a compound l; and
subjecting the compound l, the compound w, and the compound af to nucleophilic substitution to obtain the compound having a structure shown in Formula II; where
the compound h, the compound i, the compound j, the compound k, the compound l, and the compound aa have the following structural formulas:

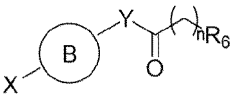
Compound h

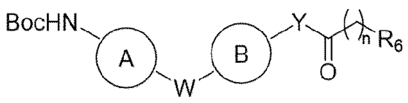
Compound i

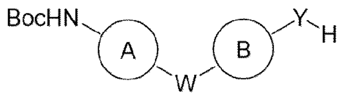
Compound j

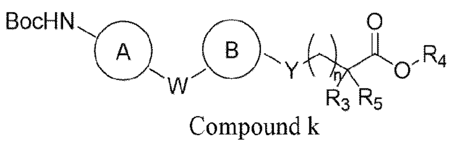
Compound k

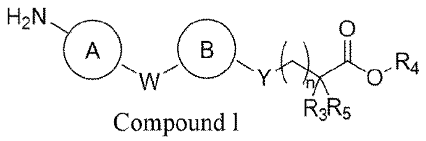
Compound l

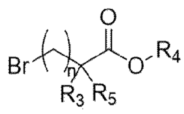
Compound aa

;

wherein X in the compound h is selected from the group consisting of H, hydroxyl, halogen, and haloalkyl;

As shown in the reaction route of FIG. 2, the compound a and the compound h are subjected to nucleophilic substitution to obtain a compound i. In some embodiments, the nucleophilic substitution is conducted in the presence of triphenylphosphine (TPP) and DIAD. In some embodiments, a molar ratio of the compound a, the compound h, the TPP, and the DIAD is 1 : 1 : 1.5 : 1.5. In some embodiments, the nucleophilic substitution is conducted in an ice-salt bath for preferably 8 h to 12 h. In some embodiments, a solvent for the nucleophilic substitution is THF, and a product obtained after the nucleophilic substitution is purified by column chromatography.

In the present disclosure, the compound i is subjected to esterolysis under an alkaline condition to obtain a compound j. In some embodiments, a reagent providing the alkaline condition is LiOH, and the esterolysis is preferably conducted in THF/$H_2O$. In some embodiments, the esterolysis is conducted at room temperature for preferably 1.5 h to 2.5 h. In some embodiments, a reaction solution obtained by the esterolysis is subjected to vacuum distillation, and water and DCM are added thereto; a pH value of the obtained system is adjusted to 2 with 6N HCl in an ice-water bath; a resulting mixture was extracted with DCM (40 mL×3), followed by washing with water, washing with saturated NaCl solution, and drying over anhydrous sodium sulfate, filtering by suction, and a resulting filtrate is concentrated under reduced pressure to obtain the compound j.

In the present disclosure, the compound j and a compound aa are subjected to nucleophilic substitution to obtain a compound k. In some embodiments, the nucleophilic substitution is conducted in the presence of $K_2CO_3$. In some embodiments, a molar ratio of the compound j, a nucleophile, and an alkali in the nucleophilic substitution is 1 : 1 : 3. In some

embodiments, the nucleophilic substitution is conducted at 80°C for preferably 5 h to 6 h; a solvent for the nucleophilic substitution is preferably acetonitrile. After the nucleophilic substitution is completed, column chromatography is conducted for purification.

In the present disclosure, the compound k is subjected to a second hydrolysis to obtain a compound, where the second hydrolysis is deprotection under an acidic condition; a reagent providing the acidic condition is preferably a solution of TFA or HCl in ethyl acetate (EA), and the deprotection is preferably conducted in the presence of dichloromethane (DCM). In some embodiments, the deprotection is conducted at room temperature for preferably 1.5 h to 2.5 h. In some embodiments, a reaction solution obtained by the deprotection is subjected to vacuum distillation, and water and DCM are added thereto; a pH value of the obtained system is adjusted to 7 with solid sodium hydroxide in an ice-water bath, an organic layer therein is separated and removed, and an aqueous layer is extracted with DCM (100 mL×2), followed by washing with water, washing with saturated NaCl solution, and drying over anhydrous sodium sulfate, and filtering by suction, and a resulting filtrate is concentrated under reduced pressure to obtain the compound l.

In the present disclosure, the compound l and the compound af are subjected to nucleophilic substitution to obtain the compound having a structure shown in Formula II for an sEH inhibitor and a PPARs agonist. In some embodiments, the nucleophilic substitution is conducted in the presence of triethylamine (TEA). In some embodiments, a molar ratio of the compound l, the compound af, and an alkali is in a range of 1 : (0.33-0.6) : 3, more preferably 1 : (0.4-0.5) : 3. In some embodiments, the nucleophilic substitution is conducted in an ice bath for preferably 10 min to 50 min, more preferably 30 min, a solvent for the nucleophilic substitution is preferably DCM. After the nucleophilic substitution is completed, column chromatography is conducted for purification.

(3) A method for preparing the compound having a structure shown in Formula III includes the following steps:

subjecting a compound m and the compound b to a first substitution to obtain a compound n;
subjecting the compound n and the compound d to a first condensation to obtain a compound o;
subjecting the compound o to a first hydrolysis to obtain a compound p;
subjecting the compound p, the compound w, and the compound af to a first nucleophilic substitution to obtain a compound q; where the compound q is the compound having a structure shown in Formula III where a thiazolidinedione group is linked to a double bond; and
subjecting the compound q to a first reduction to obtain the compound having a structure shown in Formula III where the thiazolidinedione group is linked to a single bond; where
the compound m, the compound n, the compound o, the compound p, and the compound q have the following structural formulas:

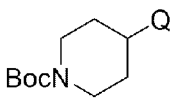
Compound m
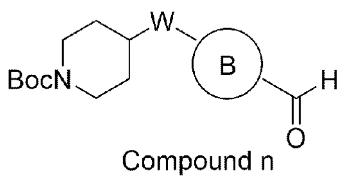
Compound n
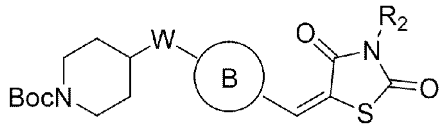
Compound o
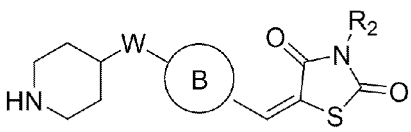
Compound p
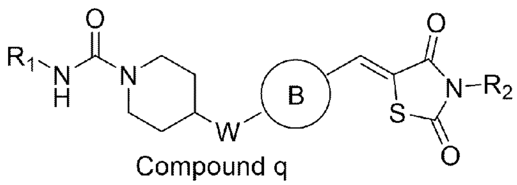
Compound q
;

wherein Q in the compound m is selected from the group consisting of H, hydroxyl, amino, sulfhydryl, carboxyl, and acyl chloride.

As shown in the reaction route of FIG. 3, the compound m the compound b are subjected to nucleophilic substitution to obtain a compound n. In some embodiments, a molar ratio of the compound m to the compound b is 1 : 1. In some embodiments, the nucleophilic substitution is conducted in the presence of triphenylphosphine (TPP) and DIAD. In some embodiments, a molar ratio of the compound m, the compound b, the TPP, and the DIAD is 1: 1 : 1.5 : 1.5. In some embodiments, a solvent for the nucleophilic substitution is THF, and the nucleophilic substitution is preferably conducted in an ice-salt bath for preferably 8 h to 12 h. After the nucleophilic substitution is completed, column chromatography is conducted for purification.

In the present disclosure, the compound n and the compound d are subjected to Claisen-Schmidt condensation

to obtain the compound o. In some embodiments, the condensation is conducted in the presence of pyridine (or piperidine) and acetic acid (or benzoic acid). In some embodiments, a molar ratio of the compound n, the compound d, the alkali, and the acid is 1 : 1 : 0.5 : 0.5. In some embodiments, the nucleophilic substitution is conducted at 110°C, a solvent for the condensation is preferably toluene, and the condensation is conducted for preferably 4 h to 8 h. After the condensation is completed, a product is cooled to room temperature and subjected to filtration suction.

After obtaining the compound o, the compound o is subjected to a first hydrolysis to obtain the compound p, where the first hydrolysis is deprotection under an acidic condition; a reagent providing the acidic condition is preferably TFA, and the deprotection is preferably conducted in the presence of DCM. In some embodiments, the deprotection is conducted at room temperature for preferably 1.5 h to 2.5 h. In some embodiments, a reaction solution obtained by the deprotection is subjected to vacuum distillation, and water and DCM are added thereto; a pH value of the obtained system is adjusted to 12 with solid sodium hydroxide in an ice-water bath, an organic layer is separated and removed, and an aqueous layer is extracted with DCM (100 mL×2), followed by washing with water, washing with saturated NaCl solution, and drying over anhydrous sodium sulfate, and filtering by suction, and a resulting filtrate is concentrated under reduced pressure to obtain the compound p.

After obtaining the compound p, the compound p and the compound af undergo nucleophilic substitution to obtain the compound q. The compound q is the compound having a structure shown in Formula III where a thiazolidinedione group is linked to a double bond; the compound q is subjected to a first reduction to obtain the compound having a structure shown in Formula III where the thiazolidinedione group is linked to a single bond. In some embodiments, a molar ratio of the compound p, solid phosgene, and an alkali is in a range of 1 : (0.33-0.6) : 3, more preferably 1 : (0.4-0.5) : 3. In some embodiments, the nucleophilic substitution is conducted in the presence of TEA using DCM as a solvent. In some embodiments, the nucleophilic substitution is conducted in an ice bath for preferably 10 min to 50 min, more preferably 30 min. After the nucleophilic substitution is completed, column chromatography is conducted for purification.

In some embodiments of the present disclosure, under the condition that $R_4$ is H, the above compound is subjected to esterolysis under an alkaline condition. In some embodiments, a reagent providing the alkaline condition is LiOH, and the esterolysis is preferably conducted in $THF/H_2O$. In some embodiments, the esterolysis is conducted at room temperature for preferably 1.5 h to 2.5 h. In some embodiments, a reaction solution obtained by the esterolysis is subjected to vacuum distillation, and water and DCM are then added thereto; a pH value of the obtained system is adjusted to 2 with 6N HCl in an ice-water bath; a resulting mixture was extracted with DCM (40 mL×3),followed by washing with water, washing with saturated NaCl solution, and drying over anhydrous sodium sulfate, and filtering by suction, and a resulting filtrate is concentrated under reduced pressure.

(4) A method for preparing the compound having a structure shown in Formula IV includes the following steps:

subjecting the compound m and the compound h to a first substitution to obtain a compound r;
subjecting the compound r to a first hydrolysis to obtain a compound s;
subjecting the compound s to a second substitution to obtain a compound t;
subjecting the compound t to a second hydrolysis to obtain a compound u; and
subjecting the compound u, the compound w, and the compound af to a first nucleophilic substitution for forming urea to obtain the compound having a structure shown in Formula IV; where
the compound r, the compound s, the compound t, and the compound u have the following structural formulas:

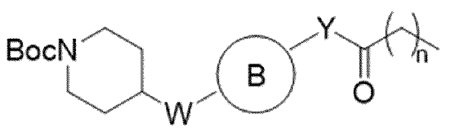

Compound r

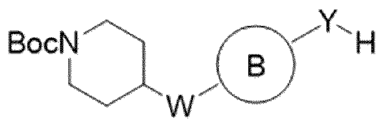

Compound s

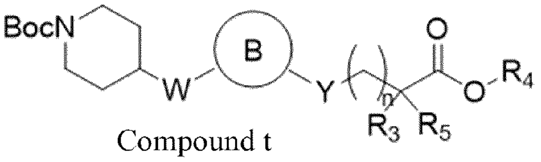

Compound t

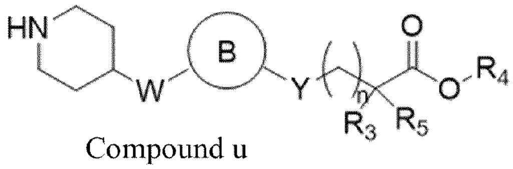

Compound u

;

As shown in the reaction route of FIG. 4, the compound m and the compound h are subjected to a first substitution to obtain a compound r. In some embodiments, the nucleophilic substitution is conducted in the presence of triphenylphosphine (TPP) and DIAD. In some embodiments, a molar ratio of the compound m, the compound h, the TPP, and the DIAD is 1 : 1 : 1.5 : 1.5. In some embodiments, a solvent for the nucleophilic substitution is THF, and the nucleophilic substitution

is preferably conducted in an ice-salt bath for preferably 8 h to 12 h. After the nucleophilic substitution is completed, column chromatography is conducted for purification.

In the present disclosure, the compound r is subjected to esterolysis under an alkaline condition to obtain a compound s. In some embodiments, a reagent providing the alkaline condition is LiOH, and the esterolysis is preferably conducted in THF/$H_2O$. In some embodiments, the esterolysis is conducted at room temperature for preferably 1.5 h to 2.5 h. In some embodiments, a reaction solution obtained by the esterolysis is subjected to vacuum distillation, and water and DCM are then added thereto; a pH value of the obtained system is adjusted to 2 with 6N HCl in an ice-water bath; a resulting system is extracted with DCM (40 mL×3),following by washing with water, washing with saturated NaCl solution, and drying over anhydrous sodium sulfate, and filtering by suction, and a resulting filtrate is concentrated under reduced pressure to obtain the compound s.

In the present disclosure, the compound s is subjected to nucleophilic substitution to obtain a compound t. In some embodiments, the nucleophilic substitution is conducted in the presence of $K_2CO_3$. In some embodiments, a molar ratio of the compound j, a nucleophile, and an alkali in the nucleophilic substitution is 1: 1 : 3. In some embodiments, the nucleophilic substitution is conducted at 80°C for preferably 5 h to 6 h, and a solvent for the nucleophilic substitution is acetonitrile. After the nucleophilic substitution is completed, column chromatography is conducted for purification.

After obtaining the compound t, the compound t is subjected to deprotection under an acidic condition to obtain the compound u; a reagent providing the acidic condition is preferably TFA, and the deprotection is preferably conducted in the presence of DCM. In some embodiments, the deprotection is conducted at room temperature for preferably 1.5 h to 2.5 h. In some embodiments, a reaction solution obtained by the deprotection is subjected to vacuum distillation, and water and DCM are then added thereto; a pH value of the obtained system is adjusted to 14 with solid sodium hydroxide in an ice-water bath; an organic layer therein is separated and removed, and an aqueous layer is extracted with DCM (100 mL×2), followed by washing with water, washing with saturated NaCl solution, and drying over anhydrous sodium sulfate, and filtering by suction, and a resulting filtrate is concentrated under reduced pressure to obtain the compound u.

After obtaining the compound u, the compound u and the compound af are subjected to nucleophilic substitution to obtain the compound having a structure shown in Formula IV for an sEH inhibitor and a PPARs agonist. In some embodiments, a molar ratio of the compound I to the compound af is in a range of 1 : (0.33-0.6), more preferably 1 : (0.4-0.5). In some embodiments, the nucleophilic substitution is conducted in the presence of triethylamine (TEA). In some embodiments, the nucleophilic substitution is conducted in an ice bath for preferably 10 min to 50 min, more preferably 30 min. After the nucleophilic substitution is completed, column chromatography is conducted for purification.

In some embodiments of the present disclosure, under the condition that $R_4$ is H, the above compound is subjected to esterolysis under an alkaline condition. In some embodiments, a reagent providing the alkaline condition is LiOH, and the esterolysis is preferably conducted in THF/$H_2O$. In some embodiments, the esterolysis is conducted at room temperature for preferably 1.5 h to 2.5 h. In some embodiments, a reaction solution obtained by the esterolysis is subjected to vacuum distillation, and water and DCM are then added thereto; a pH value of the obtained system is adjusted to 2 with 6N HCl in an ice-water bath; a resulting system is extracted with DCM (40 mL×3),followed by washing with water, washing with saturated NaCl solution, and drying over anhydrous sodium sulfate, and filtering by suction, and a resulting filtrate is concentrated under reduced pressure.

(5) A method for preparing the compound having a structure shown in Formula V includes the following steps:

subjecting the compound a and the compound h to a first substitution to obtain a compound i;
subjecting the compound i to a first hydrolysis to obtain a compound v; and
subjecting the compound v, the compound w, and the compound af to a first nucleophilic substitution to obtain the compound having a structure shown in Formula V or VI; where
the compound a, the compound h, the compound i, and the compound v have the following structural formulas:

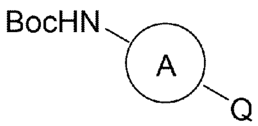
Compound a

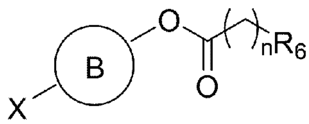
Compound h

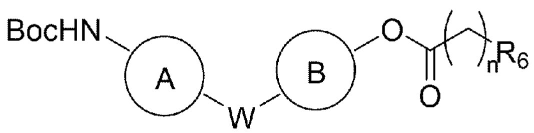
Compound i

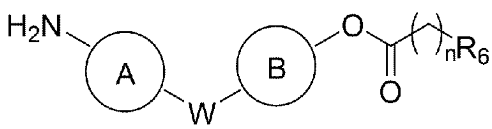
Compound v

.

As shown in the reaction route of FIG. 5, the compound a and the compound h are subjected to nucleophilic substitution to obtain a compound i. In some embodiments, the nucleophilic substitution is conducted in the presence of triphenylphosphine (TPP) and DIAD. In some embodiments, a molar ratio of the compound a, the compound h, the TPP, and the DIAD is 1 : 1 : 1.5 : 1.5. In some embodiments, a solvent for the nucleophilic substitution is THF, and the nucleophilic substitution is preferably conducted in an ice-salt bath for preferably 8 h to 12 h. After the nucleophilic substitution is completed, column chromatography is conducted for purification.

After obtaining the compound i, the compound i is subjected to deprotection under an acidic condition to obtain the compound v; a reagent providing the acidic condition is preferably TFA, and the deprotection is preferably conducted in the presence of DCM. In some embodiments, the deprotection is conducted at room temperature for preferably 1.5 h to 2.5 h. In some embodiments, a reaction solution obtained by the deprotection is subjected to vacuum distillation, and water and DCM are then added thereto; a pH value of the obtained system is adjusted to 14 with solid sodium hydroxide in an ice-water bath; an organic layer therein is separated and removed, and an aqueous layer is extracted with DCM (100 mL×2), followed by washing with water, washing with saturated NaCl solution, and drying over anhydrous sodium sulfate, and filtering by suction, and a resulting filtrate is concentrated under reduced pressure to obtain the compound v.

After obtaining the compound v, the compound v, the compound w, and the compound af are subjected to a first nucleophilic substitution to obtain the compound having a structure shown in Formula V for an sEH inhibitor and a PPARs agonist. In some embodiments, a molar ratio of the compound v to the compound af is in a range of 1 : (0.33-0.6), more preferably 1 : (0.4-0.5). In some embodiments, the nucleophilic substitution is conducted in the presence of triethylamine (TEA). In some embodiments, the nucleophilic substitution is conducted in an ice bath for preferably 10 min to 50 min, more preferably 30 min. After the nucleophilic substitution is completed, column chromatography is conducted for purification.

(6) A method for preparing the compound having a structure shown in Formula VI includes the following steps:

subjecting the compound a and a compound x to a first substitution to obtain a compound y;
subjecting the compound y to a first hydrolysis to obtain a compound z; and
subjecting the compound z, the compound w, and the compound af to a first nucleophilic substitution to obtain the compound having a structure shown in Formula V; where
the compound a, the compound x, the compound y, and the compound z have the following structural formulas:

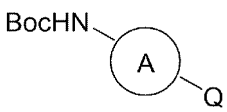

Compound a

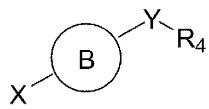

Compound x

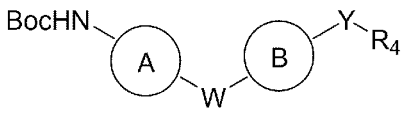

Compound y

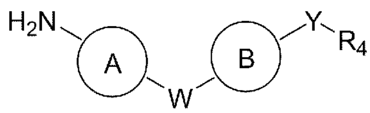

Compound z ;

wherein X in the compound x is selected from the group consisting of H, hydroxyl, halogen, and haloalkyl; and
As shown in the reaction route of FIG. 22, the compound a and a compound x are subjected to nucleophilic substitution to obtain a compound y. In some embodiments, the nucleophilic substitution is conducted in the presence of triphenylphosphine (TPP) and DIAD. In some embodiments, a molar ratio of the compound a, the compound x, the TPP, and the DIAD is 1 : 1 : 1.5 : 1.5. In some embodiments, a solvent for the nucleophilic substitution is THF, and the nucleophilic substitution is preferably conducted in an ice-salt bath for preferably 8 h to 12 h. After the nucleophilic substitution is completed, column chromatography is conducted for purification.

After obtaining the compound y, the compound y is subjected to deprotection under an acidic condition to obtain the compound z; a reagent providing the acidic condition is preferably TFA, and the deprotection is preferably conducted in the presence of DCM. In some embodiments, the deprotection is conducted at room temperature for preferably 1.5 h to 2.5 h. In some embodiments, a reaction solution obtained by the deprotection is subjected to vacuum distillation, and water and DCM are then added thereto; a pH value of the obtained system is adjusted to 14 with solid sodium hydroxide in an ice-water bath; an organic layer therein is separated and removed, and an aqueous layer is extracted with DCM (100 mL×2), followed by washing with water, washing with saturated NaCl solution, and drying over anhydrous sodium sulfate, and filtering by suction, and a resulting filtrate is concentrated under reduced pressure to obtain the compound z.

After obtaining the compound z, the compound z, the compound w, and the compound af are subjected to a first

nucleophilic substitution to obtain the compound having a structure shown in Formula VI for an sEH inhibitor and a PPARs agonist. In some embodiments, a molar ratio of the compound z to the compound af is in a range of 1 : (0.33-0.6), more preferably 1 : (0.4-0.5). In some embodiments, the nucleophilic substitution is conducted in the presence of triethylamine (TEA). In some embodiments, the nucleophilic substitution is conducted in an ice bath for preferably 10 min to 50 min, more preferably 30 min. After the nucleophilic substitution is completed, column chromatography is conducted for purification.

(7) under the condition that Y in the compound having a structure shown in Formula V or VI is -NH-, a method for preparing the compound having the structure shown in Formula V or VI includes the following steps:

subjecting the compound a and a compound ab to a first substitution to obtain a compound ac;
subjecting the compound ac to a first hydrolysis to obtain a compound ad;
subjecting the compound ad, the compound w, and the compound af to nucleophilic substitution to obtain a compound ae;
subjecting the compound ae to a first reduction to obtain the compound having a structure shown in Formula VI; and
subjecting the compound having a structure shown in Formula VI and a compound ag to condensation to obtain the compound having a structure shown in Formula V:

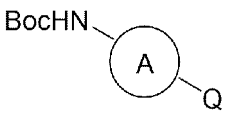
Compound a

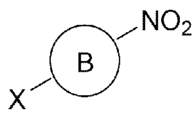
Compound ab

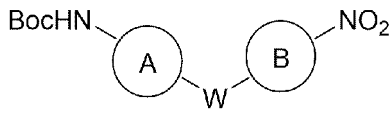
Compound ac

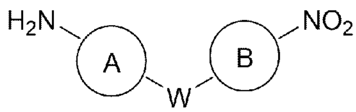
Compound ad

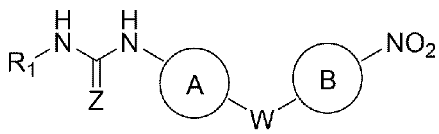
Compound ae

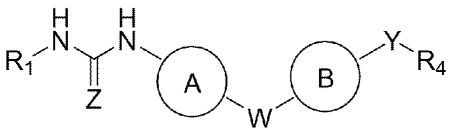
Formula VI

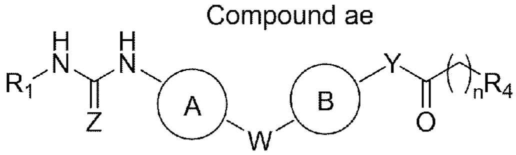
Formula V

;

wherein X in the compound ab is selected from the group consisting of H, hydroxyl, halogen, and haloalkyl.

As shown in the reaction route of FIG. 23, the compound a and a compound ab are subjected to a first nucleophilic substitution to obtain a compound ac. In some embodiments, the nucleophilic substitution is conducted in the presence of triphenylphosphine (TPP) and DIAD. In some embodiments, a molar ratio of the compound a, the compound ab, the TPP, and the DIAD is 1 : 1 : 1.5 : 1.5. In some embodiments, a solvent for the nucleophilic substitution is THF, and the nucleophilic substitution is preferably conducted in an ice-salt bath for preferably 8 h to 12 h. After the nucleophilic substitution is completed, column chromatography is conducted for purification.

After obtaining the compound ac, the compound ac is subjected to a first hydrolysis to obtain the compound ad, where the first hydrolysis is deprotection under an acidic condition; a reagent providing the acidic condition is preferably TFA, and the deprotection is preferably conducted in the presence of DCM. In some embodiments, the deprotection is conducted at room temperature for preferably 1.5 h to 2.5 h. In some embodiments, a reaction solution obtained by the deprotection is subjected to vacuum distillation, and water and DCM are then added thereto; a pH value of the obtained system is adjusted to 14 with solid sodium hydroxide in an ice-water bath; an organic layer therein is separated and removed, and an aqueous layer is extracted with DCM (100 mL×2), followed by washing with water, washing with saturated NaCl solution, and drying over anhydrous sodium sulfate, and filtering by suction, and a resulting filtrate is concentrated under reduced pressure to obtain the compound ad.

After obtaining the compound ad, the compound ad, the compound w, and the compound af are subjected to nucleophilic substitution to obtain a compound ae. In some embodiments, a molar ratio of the compound ad to the compound af is in a range of 1 : (0.33-0.6), and preferably 1 : (0.4-0.5). In some embodiments, the nucleophilic substitution is conducted in the presence of triethylamine (TEA). In some embodiments, the nucleophilic substitution is conducted in an ice bath for preferably 10 min to 50 min, more preferably 30 min. After the nucleophilic substitution is completed, column chromatography is conducted for purification.

After obtaining the compound ae, the compound ae is subjected to a first reduction to obtain the compound having a structure shown in Formula VI. In some embodiments, the first reduction is conducted with a reducing agent preferably hydrogen, a catalyst of Pd-C, and a solvent of methanol. In some embodiments, a mass ratio of the compound ae to the Pd-C is 1 : 0.1. In some embodiments, the first reduction is conducted under reflux for preferably 4 h to 8 h. After the reduction is completed, an obtained reaction solution is subjected to filtration suction and evaporation to dryness.

After obtaining the compound having a structure shown in Formula VI, the compound having a structure shown in Formula VI and the compound ag are subjected to condensation to obtain the compound having a structure shown in Formula V; a condensing agent is preferably 2-(7-azabenzotriazol-1-yl)-N,N,N',N'-tetramethyluronium hexafluorophosphate (HATU), an alkali is preferably diisopropylethylamine (DIEA), and a solvent is preferably THF. In some embodiments, the condensation is conducted at room temperature for preferably 4 h to 8 h. In some embodiments, a molar ratio of the compound having the structure shown in formula VI, the HATU, and the DIEA is 1 : 1.5 : 3. In some embodiments, an obtained reaction solution from the condensation reaction is subjected to vacuum distillation, and water and DCM are then added thereto; a resulting system is extracted, followed by washing with 1N HCl, and an organic layer is dried over anhydrous sodium sulfate. After suction filtration, a resulting filtrate is concentrated to dryness under reduced pressure, followed by purification by column chromatography to obtain the compound having the structure shown in Formula V.

In the present disclosure, unless otherwise specified, all raw materials/components are commercially available products well known to those skilled in the art.

The present disclosure further provides use of the compound and a pharmaceutically acceptable deuterated product, salt, or hydrate thereof, or a compound prepared by the method in preparation of a PPARs agonist and/or an sEH inhibitor.

In the present disclosure, the PPARs agonist and the sEH inhibitor are used to treat sEH-mediated and PPAR-mediated diseases; in some embodiments, the sEH-mediated and PPARs-mediated diseases include an inflammatory disease, a pain, sepsis, a cardiovascular disease, a neurodegenerative disease, diabetes, a diabetic complication, depression, liver fibrosis, renal failure, a chronic obstructive pulmonary disease, or pulmonary arterial hypertension (PAH).

In some embodiments of the present disclosure, the inflammatory disease includes nonalcoholic steatohepatitis (NASH) or chronic nephritis; and the pain includes a neuropathic pain.

The present disclosure provides a compound for an sEH inhibitor and a PPARs agonist. In the present disclosure, the compound for an sEH inhibitor and a PPARs agonist has a typical urea structure as a primary pharmacophore of sEH and a thiazolidinedione moiety as a primary pharmacophore of PPARs. The compound for the sEH inhibitor and the PPARs agonist shows a high inhibitory activity against human soluble epoxide hydrolase (hsEH) and a high agonist activity against the PPARs. Therefore, the compound for the sEH inhibitor and the PPARs agonist can be used to prepare into a drug for treating sEH-mediated and PPARs-mediated diseases.

The present disclosure provides a compound, and a preparation method thereof and use thereof in preparation of an sEH inhibitor and a PPARs agonist, belonging to the technical field of medicines. The compound for an sEH inhibitor and a PPARs agonist has a structure shown in Formula I, Formula II, Formula III, Formula IV, Formula V, or Formula VI. The sEH inhibitor could stabilize the endogenous substance epoxy fatty acid (EpFA) with a wide range of physiological activities, has a great inhibitory effect on human recombinant sEH, and could regulate the production of multiple proinflammatory cytokines, reduce endoplasmic reticulum (ER) stress, prevent or reverse endothelial dysfunction, and stabilize mitochondrial functions through multiple mechanisms of action. The PPAR agonist could synergize with the action of sEH inhibitor to inhibit the expression of cytokines such as resistin, tumor necrosis factor $\alpha$ (TNF-$\alpha$), and interleukin 6, which promote insulin resistance. The PPAR agonist triggers an increase in the concentration of adiponectin in plasma, which is a hormone secreted from adipose tissues and has a low content in the plasma of patients with type II diabetes mellitus. Adiponectin increases fatty acid oxidation in the liver and skeletal muscle. Therefore, the compound according to the present disclosure could be used to treat sEH-mediated and PPARs-mediated diseases including an inflammatory disease, a pain, sepsis, a cardiovascular disease, a neurodegenerative disease, diabetes, a diabetic complication, depression, NASH, liver fibrosis, chronic nephritis, renal failure, a chronic obstructive pulmonary disease, or PAH.

In order to further illustrate the present disclosure, the technical solutions provided by the present disclosure are described in detail below in conjunction with accompanying drawings and examples, but these examples should not be understood as limiting the claimed scope of the present disclosure.

**Example 1 Synthesis of tert-butyl ((1r,4r)-4-hydroxycyclohexyl)carbamate**

Trans-4-aminocyclohexanol hydrochloride (5.00 g, 32.98 mmol), sodium carbonate (10.49 g, 98.98 mmol), and water (25 mL) were added sequentially to a 100 mL three-necked flask, cooled to 0°C in an ice bath, and a solution (5 mL) of $Boc_2O$ (7.92 g, 36.28 mmol) in DCM was added dropwise within 3 min, and a resulting mixture was naturally warmed to room temperature. After 4 h, as monitored by TLC (EA: PE=1 : 3, phosphomolybdic acid color development), the reaction occurred. The reaction was stopped. A resulting reaction solution was poured into 50 mL of water, and a resulting system

was extracted with DCM (30 mL×3),and extracted with EA (30 mL×3); the organic layers were combined, washed with water (30 mL), washed with saturated NaCl (30 mL), and dried over anhydrous magnesium sulfate, followed by filtration suction and rinsing with EA. A resulting filtrate was concentrated to dryness under reduced pressure to obtain 8.0 g of a crude product of white powdery solid. The crude product was directly used in the next step without purification.

**Example 2 Synthesis of 4-aminophenyl-4-nitrobenzoate**

Cis-4-Boc-aminocyclohexanol (7.10 g, 32.98 mmol), *p*-nitrobenzoic acid (5.51 g, 98.98 mmol), TPP (12.98 g, 49.47 mol), and THF (20 mL) were added sequentially into a 100 mL three-necked flask, a resulting mixture was cooled to below -10°C in an ice-salt bath, and a solution (20 mL) of DIAD (10 g, 49.47 mol) in THF was added dropwise thereto. After 2 h, as monitored by TLC, the reaction was completed, and the solvent was evaporated under reduced pressure to obtain 35.6 g of a crude product. The crude product was purified by silica gel column chromatography, with silica gel 1.5 times the amount of the sample for mixing with the sample and 5 times the amount of the sample for silica gel column loading, using an eluent of EA: PE=1: 10, to obtain 5.53 g of a white solid.

**Example 3 Tert-butyl (4-hydroxyphenyl) carbamate**

4-Aminophenyl-4-nitrobenzoate (5.53 g, 14.88 mmol), NaOH (1.79 g, 44.65 mmol), THF (20 mL), and $H_2O$ (20 mL) were added sequentially into a 100 mL three-necked flask and reacted at room temperature. After 4 h, the reaction was completed as monitored by TLC. The reaction mixture was concentrated to dryness under reduced pressure, and EA (30 mL) was added thereto. The mixture was extracted with saturated sodium carbonate (20 mL×2), organic layers were combined, dried over anhydrous sodium sulfate, followed by filtering, and concentrating to dryness under reduced pressure to obtain 3.01 g of a white solid with a yield of 94.08%.

**Example 4 Tert-butyl ((1r,4r)-4-(4-formylphenoxy)cyclohexyl)carbamate**

Cis-*N*-BOC-4-aminocyclohexanol (2.50 g, 11.61 mmol, 1 eq), *p*-hydroxybenzaldehyde (1.42 g, 11.61 mmol,1eq), TPP (4.57 g, 17.42 mmol, 1.5 eq), and THF (10 mL) were added sequentially into a 500 mL three-necked flask, cooled to below -10°C in an ice-salt bath, and a solution (5 mL) of DIAD (3.52 g, 17.42 mmol, 1.5 eq) in THF was added dropwise at one drop/two seconds. After 72 h, the reaction was mostly completed as monitored by TLC (EA: PE=1: 1), and the reaction was stopped. The THF was removed by concentration under reduced pressure to obtain 12.21 g of a crude product of claybank oil, which was purified by silica gel column chromatography, with silica gel 1.5 times the amount of sample for mixing with the sample and 10 times the amount of sample for silica gel column loading, using an eluent of EA: PE=1 : 30, to obtain 2.85 g of white solid with a yield of 76.82%.

**Example 5 Synthesis of tert-butyl ((1r,4r)-4-{4-[(*Z*)-(2,4-dioxothiazolidin-5-ylidene)methyl]phenoxy}cyclohexyl) carbamate**

Tert-butyl[(1r,4r)-4-(4-formylphenoxy)cyclohexyl]carbamate (2.85 g, 8.93 mmol, 1 eq), 2,4-thiazolidinedione (1.05 g, 8.93 mmol, 1 eq), pyridine (0.35 g, 4.46 mmol, 0.5 eq), glacial acetic acid (0.27 g, 4.46 mmol, 0.5 eq), and toluene (10 mL) were added sequentially to a 100 mL round-bottom flask and heated to reflux. As the reaction proceeded, solids precipitated. After 8 h, the reaction was completed as monitored by TLC, and the reaction was stopped. The reaction solution was cooled to room temperature and subjected to filtration suction, and the resulting filter cake was rinsed with a small amount of toluene to obtain 2.03 g of a white solid. ESI-MS (m/z): 424.1 $[M+H]^+$

**Example 6 Synthesis of 5-{(*Z*)-4-[(1r,4r)-4-aminocyclohexyl]oxybenzylidene}thiazolidin-2,4-dione**

Tert-butyl ((1r,4r)-4-{4-[(*Z*)-(2,4-dioxothiazolidin-5-ylidene)methyl]phenoxy} cyclohexyl)carbamate (2.03 g, 4.85 mmol) and DCM (15 mL) were added sequentially into a 500 mL three-necked flask, a mixture was cooled to 0°C in an ice bath, and TFA (4 ml) was added dropwise thereto. After 0.5 h, the reaction was completed as monitored by TLC, and the reaction was stopped. TFA in the reaction solution was removed by vacuum distillation. Water (30 mL) was added and the mixture was extracted with DCM (30 mL×3). The organic layers were combined and concentrated to dryness under reduced pressure to obtain 1.17 g of a crude product of brown solid. The crude product was directly used for the next step without purification.

**Example 7 Synthesis of 1-[(1r,3R,5S,7R)-3,5-dimethyladamantan-1-yl]-3-((1r,4R)-4-{4-[(*E*)-(2,4-dioxothiazolidin-5-ylidene)methyl]phenoxy}cyclohexyl)urea (SP-B01)**

Memantine, dry DCM, and BTC solution in DCM (2 mL) were added to a 50 mL single-necked flask, cooled to below -10°C in an ice-salt bath, and TEA solution in DCM was slowly added dropwise thereto. The resulting solution was evaporated to dryness, and 20 mL of DCM was added thereto, followed by adding 5-{(Z)-4-[(1r,4r)-4-aminocyclohexyl] oxybenzylidene}thiazolidin-2,4-dione, and the mixture was refluxed for reaction. After 3 h, the reaction was completed as monitored by TLC, and the reaction was stopped. The reaction mixture was poured into 30 mL of water, followed by filtration suction to obtain a small amount of white solid without fluorescence. The filtrate was extracted with 30 mL of DCM 3 times, the resulting organic phase was washed once with water and once with saturated NaCl solution, dried over anhydrous magnesium sulfate, and evaporated to dryness to obtain 1.2 g of a white solid. The solid was mixed with 1.2 g of silica gel and loaded into a column with 10 g of silica gel, with an eluent of EA : PE=1 : 7 to obtain 0.15 g of a yellow solid. ESI-MS (m/z): 522.0 $[M-H]^-$.

**Example 8 Synthesis of 1-[(1r,3R,5S,7R)-3,5-dimethyladamantan-1-yl]-3-((1r,4R)-4-{4-[(2,4-dioxothiazolidin-5-ylidene)methyl]phenoxy}cyclohexyl)urea (SP-A01)**

1-[(1r,3R,5S,7R)-3,5-dimethyladamantan-1-yl]-3-((1r,4R)-4-{4-[(*E*)-(2,4-dioxothiazolidin-5-ylidene)methyl] phenoxy}cyclohexyl)urea and 10% Pd/C were added into a 50 mL single-necked flask, and the atmosphere was replaced with hydrogen 3 times and with Ar 3 times. The reaction was conducted at 30°C for 3.5 h before the reaction was completed and stopped, a reaction mixture was subjected to filtration suction and the filter cake was washed with a small amount of water and dried to obtain 0.15 g of a white solid. ESI-MS (m/z): 524.1 $[M-H]^-$. $^1$H NMR (400 MHz, DMSO-$d_6$) δ 11.98 (s, 1H), 7.13 (d, *J* = 8.4 Hz, 2H), 6.90 - 6.83 (m, 2H), 5.58 (d, *J* = 7.6 Hz, 1H), 5.41 (s, 1H), 4.85 (dd, *J* = 9.1, 4.3 Hz, 1H), 4.25 (tt, *J* = 9.3, 3.8 Hz, 1H), 3.28 (d, *J* = 4.4 Hz, 1H), 3.04 (dd, *J* = 14.2, 9.1 Hz, 1H), 2.04 (p, *J* = 3.1 Hz, 1H), 1.98 (dd, *J* = 12.9, 4.1 Hz, 2H), 1.87 - 1.78 (m, 2H), 1.67 (d, *J* = 3.2 Hz, 2H), 1.50 (s, 3H), 1.36 (ddd, *J* = 12.8, 9.8, 3.2 Hz, 2H), 1.32 - 1.24 (m, 2H), 1.26 - 1.19 (m, 3H), 1.19 - 1.12 (m, 1H), 1.07 (s, 2H), 0.80 (s, 6H).

**Example 9 Synthesis of 1-((1r,4r)-4-{4-[(*E*)-(2,4-dioxothiazolidin-5-ylidene)methyl]phenoxy}cyclohexyl)-3-[3-fluoro-4-(trifluoromethoxy)phenyl]urea (SP-B07)**

3-Fluoro-4-trifluoromethoxyaniline (1.21 g, 4.72 mmol, 1.5 eq), $Et_3N$ (0.95 g, 18.70 mmol, 3 eq), and dry DCM (10 mL) were added sequentially into a 100 mL three-necked flask, cooled to -15°C in a cold trap, and a solution (15 mL) of BTC (0.48 g, 1.60 mmol, 0.51 eq) in DCM was added dropwise within 3 min, and a mixture was naturally heated to room temperature. After 2 h, the reaction was completed as monitored by TLC, and the reaction was stopped.

5-{(*Z*)-4-[(1r,4r)-4-aminocyclohexyl]oxybenzylidene}thiazolidin-2,4-dione (1.17 g, 3.68 mmol, 1 eq), $Et_3N$ (0.95 g, 18.7 mmol, 3 eq), DCM (6 mL), and a solution of 3-fluoro-4-trifluoromethoxyaniline isocyanate in DCM (20 mL) were added sequentially into a 100 mL three-necked flask, a mixture was reacted at room temperature. After 6 h, as monitored by TLC, the reaction was completed and then stopped. The reaction mixture was concentrated under reduced pressure to remove DCM, 30 mL of 1N hydrochloric acid was added thereto and a resulting mixture was stirred for 5 min, followed by filtration suction. The resulting filter cake was rinsed with 20 mL of water to obtain 1.46 g of a crude product in white solid. The crude product was purified by silica gel column chromatography, with silica gel 1.5 times the amount of the sample for mixing with the sample and 10 times the amount of the sample for silica gel column loading, using an eluent of EA: PE=1 : 7, to obtain 0.34 g of white solid. $^1$H NMR (400 MHz, DMSO-$d_6$): δ (ppm) 12.49 (s, 1H), 8.71 (s, 1H),7.75 (s, 1H), 7.69 (d, *J* = 2.4 Hz, 1H), 7.54 (d, *J* = 8.8 Hz, 2H),7.55-7.36 (m, 1H), 7.13-7.08 (m, 3H), 6.31 (d, *J* = 7.6 Hz, 1H), 4.50-4.44 (m, 1H), 3.58-3.51 (m, 1H), 2.08-2.05 (m, 2H), 1.96-1.91 (m, 2H), 1.54-1.34 (m, 4H). $^{13}$C NMR (100 MHz, DMSO-$d_6$): δ (ppm) 168.4, 167.9, 159.7, 155.4, 154.6, 152.9, 141.8, 132.6, 125.7, 124.7, 120.6, 116.7, 114.0, 106.2, 106.0, 74.9, 47.7, 30.3, 30.1. ESI MS: m/z 540.0 $[M + H]^+$.

**Example 10 Synthesis of 1-((1r,4r)-4-{4-[(2,4-dioxothiazolidin-5-ylidene)methyl]|phenoxy}cyclohexyl)-3-[3-fluoro-4-(trifluoromethoxy)phenyl]urea (SP-A07)**

1-((1r,4r)-4-{4-[(*E*)-(2,4-dioxothiazolidin-5-ylidene)methyl]phenoxy}cyclohexyl)-3-[3-fluoro-4-(trifluoromethoxy)phenyl]urea (1.65 g, 3.06 mmol, 1 eq), 10% Pd/C (0.17 g), and anhydrous methanol (10 mL) were added sequentially into a 100 mL single-necked flask, and stirred at 30°C. After 3 h, the reaction was completed as monitored by TLC, and the reaction was terminated. A reaction mixture was subjected to filtration suction, and a filtrate was concentrated to dryness under reduced pressure to obtain 40 mg of a white solid. $^1$H NMR (400 MHz, DMSO-$d_6$): δ (ppm) 12.01 (s, 1H), 8.73(s, 1H), 7.68 (d, *J* = 2.4 Hz, 1H),7.65-7.40 (m, 1H), 7.38-7.09 (m, 3H), 6.88 (d, *J* = 8.6 Hz, 2H), 6.30 (d, *J* = 7.6 Hz, 1H),4.88-4.84 (m, 1H), 4.32-4.27 (m, 1H), 3.56-3.49 (m, 1H), 3.28 (s, 1H), 3.07-3.02 (m, 1H), 2.50-2.01 (m, 4H), 1.93-1.41

(m, 4H). $^{13}C$ NMR (100 MHz, DMSO-$d_6$): δ (ppm) 168.4, 167.9, 159.7, 155.4, 154.6, 152.9, 141.8, 132.6, 125.7, 124.7, 120.6, 116.7, 114.0, 106.0, 74.9, 47.7, 30.3, 30.1. ESI MS: *m/z* 542.1 [M + H]$^+$.

**Example 11 Synthesis of tert-butyl 4-(4-formylphenoxy)piperidin-1-carboxylate**

*N*-Boc-4-hydroxypiperidine (5.00 g, 0.25 mol), *p*-hydroxybenzaldehyde (15.18 g, 0.25 mol), TPP (48.92 g, 0.37 mol), and THF (75 mL) were added sequentially into a 500 mL three-necked flask, cooled to below -10°C in an ice-salt bath, and a solution (80 mL) of DIAD (37.74 mg, 0.37 mol) in THF was added dropwise at one drop/two seconds. After 5 h, the reaction was completed as monitored by TLC, and the reaction was stopped. The THF was removed by concentration under reduced pressure to obtain 127.01 g of claybank oil, which was directly used for the next step without purification. $^1H$ NMR (400 MHz, $CDCl_3$): δ (ppm) 9.88 (s, 1H), 7.82 (d, *J* = 2.0 Hz, 2H), 7.01 (d, *J* = 2.0 Hz, 2H), 4.64-4.59 (m, 1H), 3.73-3.67 (m, 2H), 3.42-3.35 (m, 2H), 1.99-1.92 (m, 2H), 1.82-1.74 (m, 2H), 1.77 (s, 9H).

**Example 12 Synthesis of 4-(piperidin-4-oxy)benzaldehyde**

Tert-butyl 4-(4-formylphenoxy)piperidin-1-carboxylate (127.01 g) and DCM (180 mL) were added sequentially into a 500 mL three-necked flask, cooled to 0°C in an ice bath, and TFA (119.04 g, 1.04 mol) was added dropwise thereto. After 37 h, the reaction was completed as monitored by TLC, and the reaction was stopped. TFA in the reaction solution was removed by vacuum distillation. DCM (50 mL) was added thereto, and the resulting mixture was extracted with 1 N HCl (200 mL×6), aqueous layers were combined, and the pH value thereof was adjusted to 10 with solid NaOH, and then extracted with n-butanol (200 mL×12). The organic layers were combined and concentrated to dryness under reduced pressure, and acetone (12 mL) was added thereto to make a slurry, followed by filtration suction. The filter cake was washed with acetone (2 mL), and dried to obtain 17.45 g of a crude product as a brown-red solid. The yield of the two steps was 34%. The crude product was directly used for the next step without purification.

**Example 10 Synthesis of (*Z*)-5-[4-(piperidin-4-oxy)benzylidene]thiazolidine-2,4-dione**

4-(Piperidin-4-oxy)benzaldehyde (4.00 g, 19.50 mmol), 2,4-thiazolidinedione (2.28 g, 19.50 mmol), pyridine (0.77 g, 9.75 mmol), glacial acetic acid (0.59 g, 9.75 mmol), and toluene (15 mL) were added sequentially into a 250 mL round-bottom flask and heated to reflux. As the reaction proceeded, solids precipitated. After 8 h, the reaction was completed as monitored by TLC, and the reaction was stopped. The reaction mixture was cooled to room temperature and subjected to filtration suction, and the filter cake was rinsed with a small amount of toluene to obtain 3 g of a pale yellow solid. ESI MS: *m/z* 304.10 [M + H]$^+$.

**Example 11 Synthesis of *N*-[(1r,3R,5S,7r)-3,5-dimethyladamantan-1-yl[-4-{4-[(*Z*)-(2,4-dioxothiazolidin-5-ylidene)methyl]phenoxy}piperidin-1-carboxamide (SP-B02)**

Memantine (16.51 g, 0.031 mol), $Et_3N$ (10.71 g, 0.11 mol), and dry DCM (50 mL) were added sequentially into a 250 mL three-necked flask, cooled to -10°C in an ice-salt bath, and a solution (50 mL) of BTC (8.01 g, 0.027 mol) in DCM was added dropwise within 30 min. A resulting mixture was naturally heated to room temperature, and the reaction was continued for 4 h and stopped, and the solvent therein was evaporated under reduced pressure.

(*Z*)-5-[4-(piperidin-4-oxy)benzylidene]thiazolidine-2,4-dione (6.33 g, 0.021 mol), $Et_3N$ (6.33 g, 0.062 mol), and DCM (30 mL) were added sequentially to a 250 mL three-necked flask, the above solution of memantine isocyanate (30 mL) in DCM was added dropwise at room temperature within 30 min and the reaction was monitored by TLC. The reaction solution was poured into water (30 mL), and a resulting system was extracted with DCM (30 mL×3). A resulting organic layers were combined, washed with water (30 mL), washed with saturated NaCl solution (30 mL), and dried over anhydrous sodium sulfate, followed by filtration suction, and concentrating under reduced pressure to obtain 12.5 g of a yellow oily liquid. The crude product was purified by silica gel column chromatography, with silica gel 1.5 times the amount of the sample for mixing with the sample and 5 times the amount of the sample for silica gel column loading, using an eluent of EA: PE=1 : 3, to obtain 6.11 g of pale yellow solid with a melting pointing of 83°C to 83°C. $^1H$ NMR (400 MHz, $CDCl_3$): δ (ppm) 9.07 (s, 1H), 7.80 (s,1H), 7.45 (d, *J* = 8.6 Hz, 2H), 6.98 (d, *J* = 8.6 Hz, 2H), 4.57 (s, 1H), 3.57 (d, *J* = 8.3 Hz, 2H), 3.30-3.29 (m, 2H), 2.16 (d, *J* = 10.0 Hz, 2H),1.98(d, *J* = 4.2 Hz, 4H), 1.83 (s,5H), 1.38 (d, *J* = 12.1 Hz, 2H), 1.28 (d, *J* = 12.0 Hz, 2H), 1.20-1.11(m, 2H), 0.85 (s, 6H). ESI MS: m/z 510.3 [M + H]$^+$.

**Example 12 Synthesis of *N*-[(1r,3R,5S,7r)-3,5-dimethyladamantan-1-yl[-4-{4-[(2,4-dioxothiazolidin-5-ylidene) methyl]phenoxy} piperidine-1-carboxamide (SP-A02)**

*N*-[(1r,3R,5S,7r)-3,5-dimethyladamantan-1-yl[-4-{4-[(Z)-(2,4-dioxothiazolidin-5-ylidene)methyl]phenoxy}piperidine-1-carboxamide (0.2 g, 0.39 mmol), 10% Pd/C (0.02 g), and anhydrous methanol (10 mL) were added sequentially into a 50 mL single-necked flask, and stirred at 30°C. After 3 h, the reaction was completed as monitored by TLC, and the reaction was terminated. The reaction mixture was subjected to filtration suction and concentrated under reduced pressure to obtain 0.18 g of a white solid. $^1$H NMR (400 MHz, Chloroform-d) δ 8.38 (s, 1H), 7.14 (d, *J* = 8.3 Hz, 2H), 6.85 (d, *J* = 8.3 Hz, 2H), 4.46 (ddt, *J* = 17.9, 7.2, 3.7 Hz, 2H), 4.26 (s, 1H), 3.57 (ddd, *J* = 12.5, 7.7, 3.7 Hz, 2H), 3.44 (dd, *J* = 14.2, 4.0 Hz, 1H), 3.24 (ddd, *J* = 13.0, 7.8, 4.1 Hz, 2H), 3.11 (dd, *J* = 14.2, 9.4 Hz, 1H), 2.14 (p, *J* = 3.3 Hz, 1H), 1.92 (ddd, *J* = 11.9, 8.0, 3.7 Hz, 2H), 1.85 - 1.71 (m, 3H), 1.77 (s, 1H), 1.38 (d, *J* = 12.4 Hz, 2H), 1.27 (dd, *J* = 15.7, 9.6 Hz, 4H), 1.14 (t, *J* = 10.4 Hz, 2H), 0.85 (s, 6H).

**Example 13 Synthesis of (*Z*)-4-{4-[(2,4-dioxothiazolidin-5-ylidene)methyl]phenoxy}-N-[4-(trifluoromethoxy)phenyl]piperidin-1-carboxamide (SP-B05)**

p-Trifluoromethoxyaniline (0.58 g, 3.28 mmol), $Et_3N$ (0.66 g, 6.56 mmol), and dry DCM (10 mL) were added sequentially to a 100 mL three-necked flask, cooled to -10°C in an ice-salt bath, and a solution (10 mL) of BTC (0.34 g, 1.12 mmol) in DCM was added dropwise within 3 min, and a mixture was naturally heated to room temperature. After 2 h, the reaction was completed as monitored by TLC, and the reaction was stopped.

5-{(*Z*)-4-[(1r,4r)-4-aminocyclohexyl]oxybenzylidene}thiazolidin-2,4-dione (1.00 g, 3.28 mmol), $Et_3N$ (0.66 g, 6.56 mmol), DCM (6 mL), p-trifluoromethoxyaniline isocyanate solution (20 mL) in DCM were added sequentially into a 100 mL three-necked flask, a resulting mixture was reacted at room temperature for half an hour, DMSO (6 mL) was added thereto, and the reaction temperature was raised to reflux. After 6 h, as monitored by TLC, the reaction was stopped. The reaction mixture was concentrated under reduced pressure to remove DCM, 30 mL of 1N hydrochloric acid was added thereto and stirred for 5 min, followed by filtration suction. A resulting filter cake was rinsed with 20 mL of water to obtain 1.46 g of a crude product as a white solid. The crude product was purified by silica gel column chromatography, with silica gel 1.5 times the amount of the sample for mixing the sample and 5 times the amount of the sample for silica gel column loading, using an eluent of EA: PE=1 : 5, to obtain 0.46 g of white solid. $^1$H NMR (400 MHz, DMSO-$d_6$): δ (ppm) 12.48 (s, 1H), 8.75 (s, 1H), 7.73 (s, 1H), 7.56 (s, 4H), 7.23-7.15 (m, 4H), 4.73 (s, 1H), 3.82 (s, 2H), 3.30 (s, 2H), 2.00 (s, 2H), 1.62 (s, 2H). $^{13}$C NMR (100 MHz, DMSO-$d_6$): δ (ppm) 168.8, 159.2, 155.2, 143.0, 140.4, 132.6, 131.8, 126.1, 121.6, 121.1, 119.8, 116.9, 72.8, 30.9.

**Example 14 Synthesis of 4-{4-[(2,4-dioxothiazolidin-5-ylidene)methyl]phenoxy}-N-[4-(trifluoromethoxy)phenyl]piperidin-1-carboxamide (SP-A05)**

(*Z*)-4-{4-[(2,4-dioxothiazolidin-5-ylidene)methyl]phenoxy}-N-[4-(trifluoromethoxy) phenyl]piperidin-1-carboxamide (1.65 g, 3.06 mmol, 1 eq), 10% Pd/C (0.20 g), and anhydrous methanol (10 mL) were added sequentially into a 50 mL single-necked flask, and stirred at 30°C. After 3 h, the reaction was completed as monitored by TLC, and the reaction was terminated. The reaction mixture was subjected to filtration suction and concentrated under reduced pressure to obtain 0.18 g of a white solid. $^1$H NMR (400 MHz, DMSO-$d_6$): δ (ppm) 12.01 (s, 1H), 8.73(s, 1H), 7.68 (d, *J* = 2.4 Hz, 1H),7.65-7.40 (m, 1H), 7.38-7.09 (m, 3H), 6.88 (d, *J* = 8.6 Hz, 2H), 6.30 (d, *J* = 7.6 Hz, 1H),4.88-4.84 (m, 1H), 4.32-4.27 (m, 1H), 3.56-3.49 (m, 1H), 3.28 (s, 1H), 3.07-3.02 (m, 1H), 2.50-2.01 (m, 4H), 1.93-1.41 (m, 4H). $^{13}$C NMR (100 MHz, DMSO-$d_6$): δ (ppm) 168.4, 167.9, 159.7, 155.4, 154.6, 152.9, 141.8, 132.6, 125.7, 124.7, 120.6, 116.7, 114.0, 106.0, 74.9, 47.7, 30.3, 30.1. ESI MS: *m/z* 542.1 $[M + H]^+$.

**Example 15 Synthesis of (*Z*)-4-{4-[(2,4-dioxothiazolidin-5-ylidene)methyl]phenoxy}-N-[3-fluoro-4-(trifluoromethoxy)phenyl]piperidin-1-carboxamide (SP-B06)**

3-Fluoro-4-trifluoromethoxyaniline (1.21 g, 4.72 mmol, 1.5 eq), $Et_3N$ (0.95 g, 18.70 mmol, 3 eq), and dry DCM (10 mL) were added sequentially into a 100 mL three-necked flask, cooled to -15°C in a cold trap, and a solution (15 mL) of BTC (0.48 g, 1.60 mmol, 0.51 eq) in DCM was added dropwise within 3 min, and a mixture was naturally heated to room temperature. After 2 h, the reaction was completed as monitored by TLC, and the reaction was stopped.

5-{(*Z*)-4-[(1r,4r)-4-aminocyclohexyl]oxybenzylidene}thiazolidin-2,4-dione (1.17 g, 3.68 mmol, 1 eq), $Et_3N$ (0.95 g, 18.7 mmol, 3 eq), DCM (6 mL), and a solution of 3-fluoro-4-trifluoromethoxyaniline isocyanate (20 mL) in DCM were added sequentially into a 100 mL three-necked flask, and a resulting mixture was reacted at room temperature. After 6 h, as monitored by TLC, and the reaction was stopped. The reaction mixture was concentrated under reduced pressure to remove DCM, 30 mL of 1N hydrochloric acid was added thereto and a resulting mixture was stirred for 5 min, followed by filtration suction. The resulting filter cake was rinsed with 20 mL of water to obtain 1.46 g of a crude product as a white solid. The crude product was purified by silica gel column chromatography, with silica gel 1.5 times the amount of the sample for mixing with the sample and 10 times the amount of the sample for silica gel column loading, using an eluent of EA: PE=1 : 7, to obtain 0.34 g of white solid with a yield of 39.48%. $^1$H NMR (400 MHz, DMSO-$d_6$): δ (ppm) 12.49 (s, 1H), 8.71 (s, 1H),7.75

(s, 1H), 7.69 (d, *J* = 2.4 Hz, 1H), 7.54 (d, *J* = 8.8 Hz, 2H),7.55-7.36 (m, 1H), 7.13-7.08 (m, 3H), 6.31 (d, *J* = 7.6 Hz, 1H), 4.50-4.44 (m, 1H), 3.58-3.51 (m, 1H), 2.08-2.05 (m, 2H), 1.96-1.91 (m, 2H), 1.54-1.34 (m, 4H). $^{13}C$ NMR (100 MHz, DMSO-$d_6$): δ (ppm) 168.4, 167.9, 159.7, 155.4, 154.6, 152.9, 141.8, 132.6, 125.7, 124.7, 120.6, 116.7, 114.0, 106.2, 106.0, 74.9, 47.7, 30.3, 30.1. ESI MS: *m*/*z* 540.0 [M + H]$^+$.

**Example 16 Synthesis of 4-{4-[(2,4-dioxothiazolidin-5-ylidene)methyl]phenoxy}-N-[3-fluoro-4-(trifluoromethoxy)phenyl]piperidin-1-carboxamide (SP-A06)**

(*Z*)-4-{4-[(2,4-dioxothiazolidin-5-ylidene)methyl]phenoxy}-N-[3-fluoro-4-(trifluoromethoxy)phenyl]piperidin-1-carboxamide (1.65 g, 3.06 mmol, 1 eq), 10% Pd/C (0.20 g), and anhydrous methanol (10 mL) were added sequentially into a 50 mL single-necked flask, and stirred at 30°C. After 3 h, the reaction was completed as monitored by TLC, and the reaction was terminated. The reaction mixture was subjected to filtration suction and concentrated under reduced pressure to obtain 1.04 g of a white solid. $^1H$ NMR (400 MHz, DMSO-$d_6$): δ (ppm) 12.01 (s, 1H), 8.73(s, 1H), 7.68 (d, *J* = 2.4 Hz, 1H),7.65-7.40 (m, 1H), 7.38-7.09 (m, 3H), 6.88 (d, *J* = 8.6 Hz, 2H), 6.30 (d, *J* = 7.6 Hz, 1H),4.88-4.84 (m, 1H), 4.32-4.27 (m, 1H), 3.56-3.49 (m, 1H), 3.28 (s, 1H), 3.07-3.02 (m, 1H), 2.50-2.01 (m, 4H), 1.93-1.41 (m, 4H). $^{13}C$ NMR (100 MHz, DMSO-$d_6$): δ (ppm) 168.4, 167.9, 159.7, 155.4, 154.6, 152.9, 141.8, 132.6, 125.7, 124.7, 120.6, 116.7, 114.0, 106.0, 74.9, 47.7, 30.3, 30.1. ESI MS: *m*/*z* 542.1 [M + H]$^+$.

**Example 17 Synthesis of (Z)-5-[4-(piperidin-4-oxy)benzylidene]imidazolin-2,4-dione**

2,4-Imidazolidinedione (1.95 g, 19.50 mmol) and water (5 mL) were added to a 100 mL round-bottom flask in sequence, and heated to 70°C, and a resulting mixture was adjusted to pH=7 with saturated aqueous sodium bicarbonate solution. Ethanolamine (2.38 g, 19.50 mmol) was added thereto, and a resulting system was heated to 100°C. A solution of 4-(piperidin-4-oxy)benzaldehyde (4 g, 19.50 mmol) in EtOH (15 mL) was added dropwise. As the reaction proceeded, solids precipitated. After 16 h, the reaction was completed as monitored by TLC, and the reaction was stopped. The reaction mixture was cooled to room temperature and subjected to filtration suction, and the resulting filter cake was rinsed with a small amount of water to obtain 1.8 g of a white solid. ESI MS: *m*/*z* 287.13 [M + H]$^+$.

**Example 18 Synthesis of *N*-[(1r,3R,5S,7r)-3,5-dimethyladamantan-1-yl]-4-{4-[(Z)-(2,5-dioxyimidazolin-4-ylidene)methyl]phenoxy}piperidin-1-carboxamide (SP-B03)**

Memantine (1.10 g, 3.46 mmol), $Et_3N$ (0.70 g, 6.92 mmol), and dry DCM (5 mL) were added sequentially into a 100 mL three-necked flask, and a resulting mixture was cooled to - 10°C in an ice-salt bath, and a solution (4 mL) of BTC (0.51 g, 1.73 mmol) in DCM was added dropwise thereto within 3 min. A resulting mixture was naturally heated to room temperature, and the reaction was continued for 4 h and stopped, and the solvent therein was evaporated under reduced pressure.

(*Z*)-5-[4-(piperidin-4-oxy)benzylidene]imidazolin-2,4-dione (0.89 g, 3.08 mmol), $Et_3N$ (0.70 g, 6.92 mmol), and DCM (10 mL) were added sequentially to a 100 mL three-necked flask, and the memantine isocyanate solution (10 mL) in DCM was added dropwise at room temperature within 20 min. The reaction was completed as monitored by TLC. The reaction solution was concentrated under reduced pressure to obtain 3.14 g of a white solid. The crude product was purified by silica gel column chromatography, with silica gel 1.5 times the amount of the sample for mixing with the sample and 5 times the amount of the sample for silica gel column loading, using an eluent of EA: PE=1 : 3, to obtain 1.02 g of a white solid. $^1H$ NMR (400 MHz, DMSO-$d_6$): δ (ppm) 11.11 (s, 1H), 10.40 (s, 1H),7.56 (d, *J* = 8.8 Hz, 2H), 6.98 (d, *J* = 8.8 Hz, 2H),6.38 (s, 1H), 5.70 (s, 1H), 4.62-4.58 (m, 1H),3.64-3.61 (m, 2H), 3.09-3.02 (m, 2H), 2.06-2.05 (m, 1H), 1.91-1.80 (m, 2H),1.76 (s, 1H), 1.76 (s, 1H), 1.59-1.43 (m, 6H), 1.31-1.16 (m, 4H), 1.06 (s, 2H), 0.81 (s, 6H).

**Example 19 Synthesis of (*Z*)-4-[4-(2,5-dioxoimidazolin-4-ylidene)methyl]phenoxy)-N-[4-(trifluoromethoxy)phenyl]piperidin-1-carboxamide (SP-B04)**

*p*-Trifluoromethoxyaniline (0.41 g, 3.50 mmol), $Et_3N$ (0.70 g, 7.00 mmol), and dry DCM (10 mL) were added sequentially to a 100 mL three-necked flask, cooled to -10°C in an ice-salt bath, and a solution (10 mL) of BTC (0.33 g, 1.13 mmol) in DCM was added dropwise within 3 min. A resulting mixture was naturally heated to room temperature. After 2 h, the reaction was completed as monitored by TLC (EA: PE=1 : 1, 2 drops of glacial acetic acid), and the reaction was stopped.

(*Z*)-5-[4-(piperidin-4-oxy)benzylidene]imidazolin-2,4-dione (0.50 g, 1.75 mmol), $Et_3N$ (0.71 g, 7.00 mmol), DCM (5 mL), *p*-trifluoromethoxyaniline isocyanate solution (20 mL) in DCM were added sequentially into a 100 mL three-necked flask, a resulting mixture was reacted at room temperature for half an hour, DMSO (6 mL) was added thereto, and the reaction temperature was raised to reflux. After 12 h, as monitored by TLC, the reaction was stopped. The reaction mixture

was concentrated under reduced pressure to remove DCM, 30 mL of 1N hydrochloric acid was added thereto and a resulting mixture was stirred for 5 min, followed by filtration suction. The resulting filter cake was rinsed with 20 mL of water to obtain 0.65 g of a crude product as a white solid.

The crude product was purified by silica gel column chromatography, with silica gel 1.5 times the amount of the sample for mixing with the sample and 5 times the amount of the sample for silica gel column loading, using an eluent of EA: PE=1 : 5, to obtain 0.42 g of a white solid. $^1$H NMR (400 MHz, DMSO-$d_6$): δ (ppm) 11.25 (s, 1H), 10.42 (s, 1H),7.59 (d, *J* = 8.8 Hz, 2H), 7.41-7.39 (m, 2H), 7.37-7.13 (m, 1H), 7.13-7.12 (m, 2H), 7.03 (d, *J* = 8.8 Hz, 2H),6.39 (s, 1H), 5.75 (s, 1H), 3.81 (d, *J* = 49 Hz, 2H),3.44 (d, *J* = 49 Hz, 2H), 2.01 (s, 2H), 1.69 (s, 2H). $^{13}$C NMR (100 MHz, DMSO-$d_6$): δ (ppm) 166.1, 157.6, 156.1, 153.4, 151.7, 131.7, 129.7, 126.7, 126.2, 125.6, 122.4, 116.6, 109.1, 72.0, 55.4, 30.6.

**Example 20 Synthesis of 4-{(1r,4r)-4-[(tert-butyloxycarbonyl)amino]cyclohexyl}oxy)phenylacetate**

Trans-4-Boc-aminocyclohexanol (9.27 g, 43.08 mmol, 1 eq), 4-hydroxyphenylacetate (6.55 g, 43.08 mmol, 1 eq), TPP (16.94 g, 64.62 mmol, 1.5 eq), and THF (25 mL) were added sequentially into a 500 mL three-necked flask, cooled to below -10°C in an ice-salt bath, and a solution (12.5 mL) of DIAD (13.07 g, 64.62 mmol, 1.5 eq) in THF was added dropwise at one drop/two seconds. After 72 h, the reaction was completed as monitored by TLC, and the reaction was stopped. The THF was removed by concentration under reduced pressure to obtain 35.57g of a crude product as a claybank oil, which was purified by silica gel column chromatography, with silica gel 1.5 times the amount of the sample for mixing with the sample and 10 times the amount of the sample for silica gel column loading, using an eluent of EA: PE=1 : 30, to obtain 2.33 g of a white solid.

**Example 21 Synthesis of tert-butyl [(1r,4r)-4-(4-hydroxyphenoxy)cyclohexyl] carbamate**

4-{(1r,4r)-4-[(tert-butyloxycarbonyl)amino]cyclohexyl}oxy)phenylacetate (0.16 g, 0.46 mmol, 1 eq), THF (5 mL), $H_2O$ (0.5 mL), and LiOH (0.03 g, 1.37 mmol, 3 eq) were added sequentially into a 25 mL single-necked flask and stirred at room temperature. After 50 min, the reaction was stopped as monitored by TLC, and the reaction mixture was concentrated under reduced pressure to remove THF. The product was extracted with EA (30 mL), and the resulting extraction liquid was adjusted to pH=4 with 1N HCl, followed by extraction with DCM (30 mL×3). The resulting organic layer was dried over anhydrous magnesium sulfate, and subjected to filtration suction, followed by concentrating to dryness under reduced pressure to obtain 0.10 g of a crude product as a light yellow solid. ESI MS: *m*/*z* 330.2 $[M + H]^+$.

**Example 22 Synthesis of methyl 2-(4-{(1r,4r)-4-[(tert-butyloxycarbonyl)amino]cyclohexyl)oxy}phenoxy)acetate**

Tert-butyl[(1r,4r)-4-(4-hydroxyphenoxy)cyclohexyl]carbamate (0.43 g, 1.40 mmol, 1 eq) and MeCN (15 mL) were added to a 25 mL single-necked flask in sequence, followed by adding $K_2CO_3$ (0.58 g, 4.20 mmol, 3 eq) and KI (0.023 g, 0.14 mmol, 0.1 eq). Methyl bromoacetate (0.32 g, 2.10 mmol, 1.5 eq) was added dropwise, the atmosphere therein was replaced with Ar 3 times, and the resulting system was heated to reflux. After 16 h, the reaction was stopped as monitored by TLC. The reaction mixture was concentrated under reduced pressure to dryness, and water (30 mL) was then added thereto. The resulting mixture was extracted with DCM (30 mL×3),the organic layers were combined, washed with water (30 mL), washed with saturated NaCl solution (30 mL), and dried over anhydrous magnesium sulfate, followed by filtration suction. The resulting filtrate was concentrated under reduced pressure to obtain 0.78 g of a brownish black oily liquid. The crude product was purified by silica gel column chromatography, with silica gel 1.5 times the amount of the sample for mixing with the sample and 3 times the amount of the sample for silica gel column loading, using an eluent of EA: PE=1 : 5, to obtain 0.40 g of a white solid with a yield of 75.35%. ESI MS: m/z 402.1 $[M + H]^+$.

**Example 23 Synthesis of methyl 2-{4-[(1r,4r)-4-aminocyclohexyl]oxy}phenoxy)acetate**

Methyl 2-(4-{(1r,4r)-4-[(tert-butyloxycarbonyl)amino]cyclohexyl)oxy}phenoxy)acetate (0.30 g, 0.79 mmol) and DCM (10 mL) were added sequentially to a 25 mL single-necked flask, cooled to 0°C in an ice bath, and TFA (3 mL) was added dropwise thereto. After 2 h, the reaction was completed as monitored by TLC, and the reaction was stopped. TFA in the reaction solution was removed by vacuum distillation to obtain 0.21 g of a brown oily substance.

**Example 24 Synthesis of methyl 2-[4-((1r,4r)-4-{3-[3-fluoro-4-(trifluoromethoxy)phenyl]ureidolcyclohexyl)oxy] phenoxy)acetate (SP-C03)**

BTC (0.08 g, 0.27 mmol, 0.34 eq), $Et_3N$ (0.16 g, 1.58 mmol, 3 eq), and dry DCM (5 mL) were added sequentially into a 25 mL single-necked flask, cooled to -80°C in a cold trap, a solution (5 mL) of 3-fluoro-4-trifluoromethoxyaniline (0.21 g, 0.79 mmol, 1 eq) in DCM was added dropwise within 3 min, and a resulting mixture was naturally heated to room

temperature. After 10 min, the reaction was completed as monitored by TLC, and the reaction was stopped.

3-fluoro-4-trifluoromethoxyaniline isocyanate solution (10 mL) in DCM and $Et_3N$ (0.16 g, 1.58 mmol, 3 eq) were added into a 25 mL three-necked flask, cooled to 0°C in an ice bath, and a solution (10 mL) of methyl 2-{4-[(1r,4r)-4-aminocyclohexyl]oxy}phenoxy)acetate (0.22 g, 0.79 mmol, 1 eq) in DCM was added dropwise. The mixture was reacted at room temperature. After 30 min, the reaction was completed as monitored by TLC, and stopped. The DCM was removed by concentration under reduced pressure to obtain 0.44 g of a crude product as a white solid. The crude product was purified by silica gel column chromatography, with silica gel 1.5 times the amount of the sample for mixing with the sample and 10 times the amount of the sample for silica gel column loading, using an eluent of EA: PE=1 : 5, to obtain 0.10 g of a white solid with a yield of 25.6%. ESI MS: m/z 523.0 $[M + Na]^+$.

**Example 25 Synthesis of 2-[4-((1r,4r)-4-{3-[3-fluoro-4-(trifluoromethoxy)phenyl]ureido}cyclohexyl)oxy]|phenoxy)acetic acid (SP-C02)**

Methyl 2-[4-((1r,4r)-4-{3-[3-fluoro-4-(trifluoromethoxy)phenyl]ureido}cyclohexyl)oxy] phenoxy)acetate (70 mg, 0.14 mmol, 1 eq), THF (2 mL), $H_2O$ (0.5 mL), and LiOH (10 mg, 0.42 mmol, 3 eq) were added sequentially into a 25 mL single-necked flask, and stirred at room temperature. After 50 min, the reaction was monitored by TLC and stopped, the THF therein was removed by concentration under reduced pressure. EA (30 mL) was added thereto and a resulting mixture was extracted with water (20 mL×2), the aqueous layer was adjusted to pH=2 with 1N HCl, followed by filtration suction, to obtain 20.02 mg of a white solid with a yield of 29.06%. $^1H$ NMR (400 MHz, DMSO-$d_6$): δ (ppm) 13.04 (s, 1H), 9.02 (s, 1H), 7.68 (dd, *J* = 13.4Hz, 2.2 Hz, 1H), 7.38 (t, *J* = 8.8Hz, 1H), 7.11 (d, *J* = 8.8 Hz, 1H), 6.86 (d, *J* = 8.0 Hz, 2H), 6.80 (d, *J* = 8.0 Hz, 2H), 6.55 (d, *J* = 7.6 Hz, 1H), 4.49 (s, 2H), 3.51-3.49 (m, 2H), 1.99 (d, *J* = 9.6 Hz, 2H), 1.89 (d, *J* = 9.6 Hz, 2H), 1.46-1.23 (m, 4H).$^{13}C$ NMR (101 MHz, DMSO-$d_6$) δ 171.46, 155.36, 154.74, 152.91, 152.60, 151.84, 142.12, 142.02, 128.75, 128.62, 124.65, 124.52, 121.97, 119.42, 117.47, 115.78, 113.93, 106.06, 105.83, 75.32, 66.10, 47.72, 30.34, 30.26. ESI MS: *m/z* 484.8 $[M - H]^-$.

**Example 26 Synthesis of ethyl 2-(4-{(1r,4r)-4-[(tert-butyloxycarbonyl)amino]cyclohexyl}oxy)phenoxy)acetate**

Methyl 2-(4-{(1r,4r)-4-[(tert-butyloxycarbonyl)amino]cyclohexyl)oxy}phenoxy)acetate (4.00 g, 13.02 mmol, 1 eq) and MeCN (20 mL) were added into a 25 mL single-necked flask in sequence, followed by adding $K_2CO_3$ (5.40 g, 39.06 mmol, 3 eq) and KI (0.22 g, 1.30 mmol, 0.1 eq). Ethyl chloroacetate (2.39 g, 19.53 mmol, 1.5 eq) was added dropwise thereto, the atmosphere was replaced with Ar 3 times, and a resulting mixture was heated to reflux. After 16 h, the reaction was monitored by TLC and stopped. The reaction solution was concentrated under reduced pressure to dryness, water (30 mL) was added thereto. The resulting mixture was extracted with DCM (30 mL×3),the organic layers were combined, washed with water (30 mL), washed with saturated NaCl solution (30 mL), and dried over anhydrous magnesium sulfate, followed by filtration suction. The resulting filtrate was concentrated under reduced pressure to obtain 0.78 g of a brownish black oily liquid. The crude product was purified by silica gel column chromatography, with silica gel 1.5 times the amount of the sample for mixing with the sample and 3 times the amount of the sample for silica gel column loading, using an eluent of EA: PE=1 : 5, to obtain 4.06 g of a white solid.

**Example 27 Synthesis of ethyl 2-{4-[(1r,4r)-4-aminocyclohexyl]oxy}phenoxy)acetate**

Ethyl 2-(4-{(1r,4r)-4-[(tert-butyloxycarbonyl)amino]cyclohexyl}oxy)phenoxy)acetate (4.06 g, 10.33 mmol) and DCM (20 mL) were added sequentially to a 100 mL single-necked flask, cooled to 0°C in an ice bath, and TFA (6 mL) was then added dropwise thereto. After 6 h, the reaction was completed as monitored by TLC, and the reaction was stopped. DCM and TFA in the reaction solution were removed by vacuum distillation to obtain 4.40 g of a brown oily substance, and the crude product was directly used for the next step without purification.

**Example 28 Synthesis of ethyl 2-[4-((1r,4r)-4-{3-[3-fluoro-4-(trifluoromethoxy)phenyl]ureidolcyclohexyl)oxy] phenoxy)acetate (SP-C04)**

BTC (0.08 g, 0.27 mmol, 0.34 eq) and dry DCM (5 mL) were added sequentially into a 25 mL single-necked flask, cooled to -80°C in a cold trap, a solution (15 mL) of 3-fluoro-4-trifluoromethoxyaniline (0.21 g, 0.79 mmol, 1 eq) and $Et_3N$ (0.24 g, 2.37 mmol, 3 eq) in DCM was added dropwise within 30 min, and a resulting mixture was naturally heated to room temperature. After 10 min, the reaction was completed as monitored by TLC, and the reaction was stopped.

3-fluoro-4-trifluoromethoxyaniline isocyanate solution (25 mL) in DCM and $Et_3N$ (0.24 g, 2.37 mmol, 3 eq) were added into a 100 mL three-necked flask, cooled to 0°C in an ice bath, and a solution (10 mL) of ethyl 2-{4-[(1r,4r)-4-aminocyclohexyl]oxy}phenoxy)acetate (0.23 g, 0.79 mmol, 1 eq) in DCM was added dropwise thereto. The resulting mixture was reacted at room temperature. After 30 min, the reaction was monitored by TLC and stopped. The DCM therein

was removed by concentration under reduced pressure to obtain 0.62 g of a crude product as a white solid. The crude product was purified by silica gel column chromatography, with silica gel 1.5 times the amount of the sample for mixing with the sample and 12 times the amount of the sample for silica gel column loading, using an eluent of EA: PE=1 : 10, to obtain 0.27 g of a white solid with a yield of 69.23%. $^{1}$H NMR (400 MHz, DMSO-$d_6$) δ 8.73 (s, 1H), 7.68 (dd, *J* = 13.4, 2.5 Hz, 1H), 7.44 - 7.34 (m, 1H), 7.09 (ddd, *J* = 9.0, 2.6, 1.3 Hz, 1H), 6.92 - 6.79 (m, 4H), 6.31 (d, *J* = 7.6 Hz, 1H), 4.69 (s, 2H), 4.16 (q, *J* = 7.1 Hz, 3H), 3.55 - 3.45 (m, 1H), 2.05 - 1.97 (m, 2H), 1.95 - 1.87 (m, 2H), 1.50 - 1.25 (m, 4H), 1.21 (t, *J* = 7.1 Hz, 3H). $^{13}$C NMR (101 MHz, DMSO-$d_6$) δ 169.44, 154.63, 152.93, 152.18, 141.95, 141.84, 128.85, 124.73, 121.97, 117.48, 115.98, 114.01, 106.15, 105.92, 75.29, 65.72, 61.00, 47.78, 30.38, 30.27, 14.52. ESI MS: m/z 537.1 [M + Na]$^+$.

**Example 29 Synthesis of methyl 2-[4-((1r,4r)-4-{3-[3-fluoro-4-(trifluoromethoxy)phenyl]ureidolcyclohexyl)oxy] phenoxy)-2-methylpropanoate (SP-C06)**

3-fluoro-4-trifluoromethoxyaniline isocyanate solution (10 mL) in DCM and $Et_3N$ (0.24 g, 2.37 mmol, 3 eq) were added into a 25 mL three-necked flask, cooled to 0°C in an ice bath, and a solution (10 mL) of methyl 2-{4-[(1r,4r)-4-aminocyclohexyl]oxy}phenoxy)-2-methylpropanoate (0.24 g, 0.79 mmol, 1 eq) in DCM was added dropwise thereto. The resulting mixture was reacted at room temperature. After 30 min, the reaction was monitored by TLC and stopped. The DCM therein was removed by concentration under reduced pressure to obtain 0.47 g of a crude product as a white solid. The crude product was purified by silica gel column chromatography, with silica gel 1.5 times the amount of the sample for mixing with the sample and 10 times the amount of the sample for silica gel column loading, using an eluent of EA: PE=1 : 5, to obtain 0.32 g of a white solid with a yield of 76.19%. $^{1}$H NMR (400 MHz, DMSO-$d_6$) δ 8.73 (s, 1H), 7.68 (dd, *J* = 13.5, 2.5 Hz, 1H), 7.39 (t, *J* = 8.9 Hz, 1H), 7.09 (dt, *J* = 9.3, 1.9 Hz, 1H), 6.89 - 6.81 (m, 2H), 6.79 - 6.71 (m, 2H), 6.30 (d, *J* = 7.6 Hz, 1H), 4.21 (dt, *J* = 9.9, 5.6 Hz, 1H), 3.70 (s, 3H), 3.50 (s, 1H), 3.43 - 3.36 (m, 2H), 2.01 (d, *J* = 15.5 Hz, 2H), 1.95 - 1.87 (m, 2H), 1.45 (s, 10H). $^{13}$C NMR (101 MHz, DMSO-$d_6$) δ 174.26, 154.63, 153.28, 148.83, 141.94, 141.84, 128.85, 124.72, 121.97, 121.76, 119.42, 116.85, 114.01, 106.16, 105.92, 79.60, 75.05, 52.69, 47.79, 30.42, 30.31, 25.37, 15.62. ESI MS: m/z 551.2 [M + Na]$^+$.

**Example 30 Synthesis of 2-[4-((1r,4r)-4-{3-[3-fluoro-4-(trifluoromethoxy)phenyl]ureido}cyclohexyl)oxy]phenoxy)-2-methylpropanoic acid (SP-C05)**

Methyl 2-[4-((1r,4r)-4-{3-[3-fluoro-4-(trifluoromethoxy)phenyl]ureido}cyclohexyl)oxy] phenoxy)-2-methylpropanoate (0.20 g, 0.38 mmol, 1 eq), THF (5 mL), H2O (0.5 mL), and LiOH (30 mg, 1.20 mmol, 3 eq) were added sequentially into a 25 mL single-necked flask, and stirred at room temperature. After 3 h, the reaction was monitored by TLC and stopped, and the THF therein was removed by concentration under reduced pressure. DCM (30 mL) was added thereto and a resulting mixture was extracted with water (10 mL×2), and the aqueous layer was adjusted to pH=2 with 1N HCl, followed by filtration suction to obtain 0.15 g of a white solid with a yield of 76.92%. $^{1}$H NMR (400 MHz, DMSO-$d_6$) δ 12.93 (s, 1H), 8.78 (s, 1H), 7.68 (dd, *J* = 13.5, 2.5 Hz, 1H), 7.44 - 7.35 (m, 1H), 7.09 (ddd, *J* = 9.1, 2.6, 1.3 Hz, 1H), 6.90 - 6.82 (m, 2H), 6.82 - 6.75 (m, 2H), 6.34 (d, *J* = 7.6 Hz, 1H), 4.21 (dq, *J* = 9.6, 5.6, 4.7 Hz, 1H), 3.57 - 3.46 (m, 1H), 2.05 - 1.97 (m, 2H), 1.91 (dd, *J* = 12.8, 4.2 Hz, 2H), 1.50 - 1.26 (m, 10H). $^{13}$C NMR (101 MHz, DMSO-$d_6$) δ 175.60, 155.37, 154.64, 152.93, 149.26, 141.96, 141.86, 124.71, 121.97, 121.32, 116.88, 114.00, 106.14, 105.90, 79.27, 75.08, 47.77, 30.40, 30.29, 25.46. ESI MS: m/z 512.8 [M - H]$^-$.

**Example 31 Synthesis of methyl 2-[4-((1r,4r)-4-{3-[4-(trifluoromethoxy)phenyl]ureidolcyclohexyl)oxy]phenoxy) acetate (SP-C09)**

BTC (0.08 g, 0.27 mmol, 0.34 eq) and dry DCM (5 mL) were added sequentially into a 25 mL single-necked flask, cooled to -80°C in a cold trap, and a solution (20 mL) of *p*-trifluoromethoxyaniline (0.14 g, 0.79 mmol, 1 eq) and $Et_3N$ (0.24 g, 2.37 mmol, 3 eq) in DCM was added dropwise within 30 min, and a resulting mixture was then naturally heated to room temperature. After 10 min, the reaction was completed as monitored by TLC, and the reaction was stopped.

*p*-Trifluoromethoxyaniline isocyanate solution (10 mL) in DCM and $Et_3N$ (0.24 g, 2.37 mmol, 3 eq) were added into a 25 mL three-necked flask, cooled to 0°C in an ice bath, and a solution (10 mL) of methyl 2-{4-[(1r,4r)-4-aminocyclohexyl]oxy}phenoxy)acetate (0.22 g, 0.79 mmol, 1 eq) in DCM was added dropwise. The resulting mixture was then reacted at room temperature. After 30 min, the reaction was completed as monitored by TLC and stopped. The DCM was removed by concentration under reduced pressure to obtain 0.50 g of a crude product as a white solid. The crude product was purified by silica gel column chromatography, with silica gel 1.5 times the amount of the sample for mixing with the sample and 10 times the amount of the sample for silica gel column loading using an eluent of EA: PE= 1 : 10, to obtain 0.31 g of a white solid.

$^{1}$H NMR (400 MHz, DMSO-$d_6$) δ 8.51 (s, 1H), 7.51 - 7.43 (m, 2H), 7.21 (d, *J* = 8.2 Hz, 2H), 6.91 - 6.80 (m, 4H), 6.18 (d, *J* = 7.6 Hz, 1H), 4.71 (s, 2H), 4.19 (dt, *J* = 9.8, 5.7 Hz, 1H), 3.69 (s, 3H), 3.57 - 3.46 (m, 1H), 2.00 (dd, *J* = 11.5, 4.1 Hz, 2H),

1.96 - 1.87 (m, 2H), 1.49 - 1.26 (m, 4H).$^{13}$C NMR (101 MHz, DMSO-$d_6$) δ 169.93, 154.87, 152.21, 152.17, 142.43, 140.29, 122.09, 119.01, 117.50, 115.95, 75.34, 65.62, 52.19, 47.71, 30.48, 30.29. ESI MS: m/z 505.0 $[M + Na]^+$.

**Example 32 Synthesis of 2-[4-((1r,4r)-4-{3-[4-(trifluoromethoxy)phenyl]ureido} cyclohexyl)oxy]phenoxy)acetic acid (SP-C08)**

Methyl 2-[4-((1r,4r)-4-{3-[4-(trifluoromethoxy)phenyl]ureido}cyclohexyl)oxy]phenoxy) acetate (0.20 g, 0.41 mmol, 1 eq), THF (5 mL), $H_2O$ (0.5 mL), and LiOH (30 mg, 1.20 mmol, 3 eq) were added sequentially into a 25 mL single-necked flask, and stirred at room temperature. After 3 h, the reaction was monitored by TLC and stopped, and the THF therein was removed by concentration under reduced pressure. DCM (30 mL) was added thereto and a resulting mixture was extracted with water (10 mL×2), and the aqueous layer was adjusted to pH=2 with 1N HCl, followed by filtration suction to obtain 0.15 g of a white solid with a yield of 78.94%. $^1$H NMR (400 MHz, DMSO-$d_6$) δ 8.76 (s, 1H), 7.51 - 7.44 (m, 2H), 7.21 (d, *J* = 8.6 Hz, 2H), 6.91 - 6.76 (m, 4H), 6.40 (d, *J* = 7.6 Hz, 1H), 4.52 (s, 2H), 4.17 (tt, *J* = 9.7, 4.0 Hz, 1H), 3.55 - 3.45 (m, 1H), 2.04 - 1.95 (m, 2H), 1.94 - 1.86 (m, 2H), 1.36 (dddd, *J* = 40.3, 20.2, 16.5, 10.0 Hz, 5H).$^{13}$C NMR (101 MHz, DMSO-$d_6$) δ 171.14, 154.96, 152.56, 151.89, 142.35, 140.44, 122.04, 118.97, 117.49, 115.79, 75.39, 66.00, 47.67, 30.45, 30.29. ESI MS: *m/z* 466.8 $[M - H]^-$.

**Example 33 Synthesis of ethyl 2-[4-((1r,4r)-4-{3-[4-(trifluoromethoxy)phenyl]ureidolcyclohexyl)oxy]phenoxy) acetate (SP-C10)**

BTC (0.08 g, 0.27 mmol, 0.34 eq) and dry DCM (5 mL) were added sequentially into a 25 mL single-necked flask, cooled to -80°C in a cold trap, a solution (15 mL) of *p*-trifluoromethoxyaniline (0.14 g, 0.79 mmol, 1 eq) and $Et_3N$ (0.24 g, 2.37 mmol, 3 eq) in DCM was added dropwise within 30 min, and a resulting mixture was naturally heated to room temperature. After 10 min, the reaction was completed as monitored by TLC (EA: PE=1 : 3), and the reaction was stopped.

p-Trifluoromethoxyaniline isocyanate solution (10 mL) in DCM and $Et_3N$ (0.24 g, 2.37 mmol, 3 eq) were added into a 25 mL three-necked flask, cooled to 0°C in an ice bath, and a solution (10 mL) of ethyl 2-{4-[(1r,4r)-4-aminocyclohexyl]oxy}phenoxy)acetate (0.23 g, 0.79 mmol, 1 eq) was added dropwise. The resulting mixture was reacted at room temperature. After 30 min, the reaction was completed as monitored by TLC and stopped. The DCM therein was removed by concentration under reduced pressure to obtain 0.93 g of a crude product as a white solid. The crude product was purified by silica gel column chromatography, with silica gel 1.5 times the amount of the sample for mixing with the sample and 12 times the amount of the sample for silica gel column loading, using an eluent of EA: PE=1 : 10, to obtain 0.24 g of a white solid.

$^1$H NMR (400 MHz, DMSO-$d_6$) δ 8.52 (s, 1H), 7.50 - 7.43 (m, 2H), 7.22 (d, *J* = 8.6 Hz, 2H), 6.92 - 6.80 (m, 4H), 6.18 (d, *J* = 7.6 Hz, 1H), 4.69 (s, 2H), 4.19 - 4.09 (m, 2H), 3.60 - 3.42 (m, 1H), 2.04 - 1.96 (m, 2H), 1.92 (d, *J* = 13.7 Hz, 2H), 1.48 - 1.24 (m, 5H), 1.21 (t, *J* = 7.1 Hz, 4H).$^{13}$C NMR (101 MHz, DMSO-$d_6$) δ 169.43, 154.87, 152.20, 142.43, 140.28, 122.08, 119.02, 117.49, 115.98, 75.34, 65.74, 61.00, 47.70, 30.48, 30.29, 14.51. ESI MS: m/z 519.1 $[M + Na]^+$.

**Example 34 Synthesis of methyl 2-methyl-2-[4-((1,4r)-4-{3-[4-(trifluoromethoxy) phenyl]ureido}cyclohexyl)oxy] phenoxy)-2-methylpropanoate (SP-C12)**

p-Trifluoromethoxyaniline isocyanate solution (10 mL) in DCM and $Et_3N$ (0.24 g, 2.37 mmol, 3 eq) were added into a 25 mL three-necked flask, cooled to 0°C in an ice bath, and a solution (10 mL) of methyl 2-{4-[(1r,4r)-4-aminocyclohexyl]oxy}phenoxy)-2-methylpropanoate (0.24 g, 0.79 mmol, 1 eq) in DCM was added dropwise. The resulting mixture was reacted at room temperature, and after 30 min, the reaction was completed as monitored by TLC and stopped. The DCM therein was removed by concentration under reduced pressure to obtain 0.47 g of a crude product as a white solid. The crude product was purified by silica gel column chromatography, with silica gel 1.5 times the amount of the sample for mixing with the sample and 10 times the amount of the sample for silica gel column loading, using an eluent of EA: PE=1 : 5, to obtain 0.33 g of a white solid with a yield of 76.19%. $^1$H NMR (400 MHz, DMSO-$d_6$) δ 8.51 (s, 1H), 7.50 - 7.42 (m, 2H), 7.21 (d, *J* = 8.5 Hz, 2H), 6.88 - 6.81 (m, 2H), 6.79 - 6.72 (m, 2H), 6.18 (d, *J* = 7.6 Hz, 1H), 4.21 (tt, *J* = 9.7, 3.7 Hz, 1H), 3.70 (s, 3H), 3.51 (dtd, *J* = 10.9, 7.3, 4.2 Hz, 1H), 2.01 (dd, *J* = 12.6, 4.6 Hz, 2H), 1.92 (dd, *J* = 13.0, 4.3 Hz, 2H), 1.51 - 1.25 (m, 11H). $^{13}$C NMR (101 MHz, DMSO-$d_6$) δ 174.25, 154.88, 153.32, 148.84, 142.45, 140.29, 122.07, 121.78, 119.03, 116.88, 79.62, 75.12, 52.68, 47.72, 30.52, 30.33, 25.38. ESI MS: m/z 533.0 $[M + Na]^+$.

**Example 35 Synthesis of 2-methyl-2-[4-((1,4r)-4-{3-[4-(trifluoromethoxy)phenyl] ureido}cyclohexyl)oxy]phenoxy)-2-methylpropanoic acid (SP-C11)**

2-Methyl-2-[4-((1,4r)-4-{3-[4-(trifluoromethoxy)phenyl]ureido}cyclohexyl)oxy] phenoxy)-2-methylpropanoic acid (0.20 g, 0.39 mmol, 1 eq), THF (5 mL), $H_2O$ (0.5 mL), and LiOH (30 mg, 1.20 mmol, 3 eq) were added sequentially

into a 25 mL single-necked flask, and stirred at room temperature. After 3 h, the reaction was monitored by TLC and stopped, and the THF therein was removed by concentration under reduced pressure. DCM (30 mL) was added thereto and a resulting mixture was extracted with water (10 mL×2), and the aqueous layer was adjusted to pH=2 with 1N HCl, followed by filtration suction to obtain 0.14 g of a white solid with a yield of 73.68%.

$^1$H NMR (400 MHz, DMSO-$d_6$) δ 8.54 (s, 1H), 7.51 - 7.43 (m, 2H), 7.22 (d, *J* = 8.6 Hz, 2H), 6.90 - 6.72 (m, 4H), 6.20 (d, *J* = 7.6 Hz, 1H), 4.21 (td, *J* = 9.6, 4.8 Hz, 1H), 3.57 - 3.45 (m, 1H), 2.08 - 1.97 (m, 2H), 1.91 (dd, *J* = 12.0, 4.6 Hz, 2H), 1.50 - 1.23 (m, 11H). $^{13}$C NMR (101 MHz, DMSO-$d_6$) δ 175.60, 154.88, 152.94, 149.26, 142.42, 140.30, 122.08, 121.31, 119.01, 116.88, 79.27, 75.13, 47.70, 30.51, 30.32, 25.46. ESI MS: *m/z* 494.9 [M - H]$^-$.

**Example 36 Synthesis of methyl 2-[4-((1R,4r)-4-{3-[(1R,3R,5S,7R)-3,5-dimethyladamantan-1-yl]ureido}cyclohexyl)oxy]phenoxy}acetate (SP-C15)**

BTC (0.08 g, 0.27 mmol, 0.34 eq) and dry DCM (5 mL) were added sequentially into a 25 mL single-necked flask, and cooled to -80°C in a cold trap, and a solution (20 mL) of memantine (0.14 g, 0.79 mmol, 1 eq) and $Et_3N$ (0.24 g, 2.37 mmol, 3 eq) in DCM was added dropwise within 30 min, and a resulting mixture was naturally heated to room temperature. After 10 min, the reaction was completed as monitored by TLC, and stopped.

Memantine isocyanate solution (10 mL) in DCM and $Et_3N$ (0.24 g, 2.37 mmol, 3 eq) were added into a 25 mL three-necked flask, cooled to 0°C in an ice bath, and a solution (10 mL) of methyl 2-{4-[(1r,4r)-4-aminocyclohexyl]oxy}phenoxy)acetate (0.22 g, 0.79 mmol, 1 eq) in DCM was added dropwise. The resulting mixture was reacted at room temperature, and after 30 min, the reaction was completed as monitored by TLC and stopped. The DCM therein was removed by concentration under reduced pressure to obtain 0.50 g of a crude product as a white solid. The crude product was purified by silica gel column chromatography, with silica gel 1.5 times the amount of the sample for mixing with the sample and 10 times the amount of the sample for silica gel column loading, using an eluent of EA: PE= 1 : 10, to obtain 0.33 g of a white solid.

$^1$H NMR (400 MHz, DMSO-$d_6$) δ 6.89 - 6.79 (m, 4H), 5.58 (d, *J* = 7.6 Hz, 1H), 5.41 (s, 1H), 4.70 (s, 2H), 4.15 (tt, *J* = 10.0, 4.0 Hz, 1H), 3.69 (s, 3H), 3.35 (dt, *J* = 7.3, 3.6 Hz, 1H), 2.04 (p, *J* = 3.2 Hz, 1H), 1.96 (dt, *J* = 13.4, 3.9 Hz, 2H), 1.86 - 1.77 (m, 2H), 1.67 (d, *J* = 3.1 Hz, 2H), 1.49 (s, 4H), 1.40 - 1.11 (m, 9H), 1.07 (s, 2H), 0.79 (s, 6H). $^{13}$C NMR (101 MHz, DMSO-$d_6$) δ 169.92, 157.00, 152.25, 152.17, 117.56, 115.96, 75.50, 65.66, 52.18, 51.49, 50.87, 48.61, 47.37, 42.91, 41.00, 32.36, 30.83, 30.61, 30.40, 30.10. ESI MS: m/z 507.1 [M + Na]$^+$.

**Example 37 Synthesis of 2-[4-((1R,4r)-4-{3-[(1R,3R,5S,7R)-3,5-dimethyladamantan-1-yl]ureido}cyclohexyl)oxy]phenoxy}acetic acid (SP-C14)**

Methyl 2-[4-((1R,4r)-4-{3-[(1R,3R,5S,7R)-3,5-dimethyladamantan-1-yl]ureido} cyclohexyl)oxy]phenoxy}acetate (0.20 g, 0.41 mmol, 1 eq), THF (5 mL), $H_2O$ (0.5 mL), and LiOH (30 mg, 1.24 mmol, 3 eq) were added sequentially into a 25 mL single-necked flask, and stirred at room temperature. After 3 h, the reaction was monitored by TLC and stopped, and the THF therein was removed by concentration under reduced pressure. DCM (30 mL) was added thereto and a resulting mixture was extracted with water (10 mL×2), the aqueous layer was adjusted to pH=2 with 1N HCl, followed by filtration suction to obtain 0.15 g of a white solid.

$^1$H NMR (400 MHz, DMSO-$d_6$) δ 6.85 - 6.78 (m, 2H), 6.78 - 6.70 (m, 2H), 5.77 (d, *J*= 7.6 Hz, 1H), 5.59 (s, 1H), 4.24 (s, 2H), 4.11 (tt, *J* = 9.5, 4.0 Hz, 1H), 3.33 (ddd, *J* = 11.1, 7.1, 3.7 Hz, 1H), 2.04 (p, *J* = 3.2 Hz, 1H), 2.00 - 1.90 (m, 2H), 1.81 (dq, *J* = 11.8, 3.8 Hz, 2H), 1.67 (d, *J* = 3.1 Hz, 2H), 1.55 - 1.43 (m, 4H), 1.42 - 1.25 (m, 4H), 1.25 - 1.09 (m, 5H), 1.07 (s, 2H), 0.79 (s, 6H). $^{13}$C NMR (101 MHz, DMSO-$d_6$) δ 171.28, 157.12, 153.18, 151.45, 117.53, 115.68, 75.61, 67.74, 51.46, 50.89, 48.61, 47.33, 42.93, 40.99, 32.36, 30.79, 30.62, 30.40, 30.10.

**Example 38 Synthesis of ethyl 2-[4-((1R,4r)-4-{3-[(1R,3R,5S,7R)-3,5-dimethyladamantan-1-yl]ureido}cyclohexyl)oxy]phenoxy}acetate (SP-C16)**

BTC (0.08 g, 0.27 mmol, 0.34 eq) and dry DCM (5 mL) were added sequentially into a 25 mL single-necked flask, and cooled to -80°C in a cold trap, a solution (15 mL) of memantine (0.14 g, 0.79 mmol, 1 eq) and $Et_3N$ (0.24 g, 2.37 mmol, 3 eq) in DCM was added dropwise within 30 min, and a resulting mixture was naturally heated to room temperature. After 10 min, the reaction was completed as monitored by TLC, and stopped.

Memantine isocyanate solution (10 mL) in DCM and $Et_3N$ (0.24 g, 2.37 mmol, 3 eq) were added into a 25 mL three-necked flask, and cooled to 0°C in an ice bath, and a solution (10 mL) of ethyl 2-{4-[(1r,4r)-4-aminocyclohexyl]oxy}phenoxy)acetate (0.23 g, 0.79 mmol, 1 eq) in DCM was added dropwise. The resulting mixture was reacted at room temperature for 30 min, and the reaction was completed as monitored by TLC and stopped. The DCM therein was removed by concentration under reduced pressure to obtain 0.93 g of a crude product as a white solid. The crude product was purified by silica gel column chromatography, with silica gel 1.5 times the amount of the sample for mixing with the sample

and 15 times the amount of the sample for silica gel column loading (except for non-fluorescent urea of memantine not detected byiodine), using an eluent of EA: PE=1 : 10, to obtain 0.20 g of a white solid.

$^1$H NMR (400 MHz, DMSO-$d_6$) δ 6.89 - 6.79 (m, 4H), 5.58 (d, $J$ = 7.6 Hz, 1H), 5.41 (s, 1H), 4.68 (s, 2H), 4.16 (q, $J$ = 7.1 Hz, 3H), 3.35 (dd, $J$ = 7.1, 3.6 Hz, 1H), 2.04 (p, $J$ = 3.2 Hz, 1H), 2.01 - 1.90 (m, 2H), 1.86 - 1.76 (m, 2H), 1.67 (d, $J$ = 3.2 Hz, 2H), 1.49 (s, 4H), 1.40 - 1.11 (m, 11H), 1.07 (s, 2H), 0.79 (s, 6H). $^{13}$C NMR (101 MHz, DMSO-$d_6$) δ 169.43, 156.99, 152.21, 152.17, 117.51, 115.97, 75.46, 65.73, 61.00, 51.47, 50.85, 48.60, 47.36, 42.90, 40.99, 32.36, 30.84, 30.61, 30.40, 30.09, 14.52.

### Example 39 Synthesis of methyl 2-[4-((1R,4r)-4-{3-[(1R,3R,5S,7R)-3,5-dimethyladamantan-1-yl]ureido}cyclohexyl)oxy]phenoxy}-2-methylpropanoate (SP-C18)

BTC (0.08 g, 0.27 mmol, 0.34 eq) and dry DCM (5 mL) were added sequentially into a 25 mL single-necked flask, cooled to -80°C in a cold trap, a solution (15 mL) of memantine (0.14 g, 0.79 mmol, 1 eq) and $Et_3N$ (0.24 g, 2.37 mmol, 3 eq) in DCM was added dropwise within 30 min, and a resulting mixture was naturally heated to room temperature. After 10 min, the reaction was completed as monitored by TLC, and stopped.

Memantine isocyanate solution (10 mL) in DCM and $Et_3N$ (0.24 g, 2.37 mmol, 3 eq) were added into a 25 mL three-necked flask, cooled to 0°C in an ice bath, and a solution (10 mL) of methyl 2-{4-[(1r,4r)-4-aminocyclohexyl]oxy} phenoxy)-2-methylpropanoate (0.24 g, 0.79 mmol, 1 eq) in DCM was added dropwise. The resulting mixture was reacted at room temperature. After 30 min, the reaction was completed as monitored by TLC, and stopped. The DCM therein was removed by concentration under reduced pressure to obtain 0.93 g of a crude product as a white solid. The crude product was purified by silica gel column chromatography, with silica gel 1.5 times the amount of the sample for mixing with the sample and 15 times the amount of the sample for silica gel column loading, using an eluent of EA: PE=1 : 10, to obtain 0.32 g of a white solid.

$^1$H NMR (400 MHz, DMSO-$d_6$) δ 6.87 - 6.78 (m, 2H), 6.78 - 6.69 (m, 2H), 5.58 (d, $J$ = 7.6 Hz, 1H), 5.41 (s, 1H), 4.22 - 4.11 (m, 1H), 3.69 (s, 3H), 2.04 (p, $J$ = 3.1 Hz, 1H), 2.01 - 1.92 (m, 2H), 1.86 - 1.77 (m, 2H), 1.67 (d, $J$ = 3.2 Hz, 2H), 1.50 - 1.13 (m, 18H), 1.07 (s, 2H), 0.79 (s, 6H). $^{13}$C NMR (101 MHz, DMSO-$d_6$) δ 174.25, 156.99, 153.32, 148.80, 121.77, 116.88, 79.61, 75.21, 52.68, 51.48, 50.86, 48.60, 47.37, 42.91, 40.99, 32.36, 30.88, 30.61, 30.44, 30.09, 25.38.

### Example 39 Synthesis of 2-[4-((1R,4r)-4-{3-[(1R,3R,5S,7R)-3,5-dimethyladamantan -1-yl]ureido}cyclohexyl) oxy]phenoxy}-2-methylpropanoic acid (SP-C17)

Methyl 2-[4-((1R,4r)-4-{3-[(1R,3R,5S,7R)-3,5-dimethyladamantan-1-yl]ureido} cyclohexyl)oxy]phenoxy}-2-methylpropanoate (0.20 g, 0.39 mmol, 1 eq), THF (5 mL), $H_2O$ (0.5 mL), and LiOH (30 mg, 1.24 mmol, 3 eq) were added sequentially into a 25 mL single-necked flask, and stirred at room temperature. After 3 h, the reaction was monitored by TLC and stopped, and the THF therein was removed by concentration under reduced pressure. DCM (30 mL) was added thereto and a resulting mixture was extracted with water (10 mL×2), and the aqueous layer was adjusted to pH=2 with 1N HCl, followed by filtration suction to obtain 0.10 g of a white solid.

$^1$H NMR (400 MHz, DMSO-$d_6$) δ 6.79 (s, 4H), 5.73 (d, $J$ = 7.7 Hz, 1H), 5.54 (s, 1H), 4.14 (td, $J$ = 9.6, 4.8 Hz, 1H), 3.33 (ddd, $J$ = 10.8, 7.0, 3.6 Hz, 1H), 2.04 (p, $J$ = 3.2 Hz, 1H), 2.01 - 1.91 (m, 2H), 1.85 - 1.76 (m, 2H), 1.67 (d, $J$ = 3.2 Hz, 2H), 1.55 - 1.43 (m, 4H), 1.43 - 1.10 (m, 14H), 1.07 (s, 2H), 0.79 (s, 6H). $^{13}$C NMR (101 MHz, DMSO-$d_6$) δ 175.96, 157.08, 152.49, 149.72, 121.01, 116.83, 79.75, 75.30, 51.46, 50.88, 48.60, 47.34, 42.92, 40.98, 32.36, 30.84, 30.62, 30.43, 30.09, 25.78.

### Example 40 Synthesis of phenyl 4-(((1r,4r)-4-aminocyclohexyl)oxy)acetate

Phenyl 4-(((1r,4r)-4-((tert-butoxycarbonyl)amino)cyclohexyl)oxy)acetate (1.69 g, 4.84 mmol) and DCM (12 mL) were added sequentially to a 100 mL single-necked flask, and TFA (6 mL) was added dropwise at room temperature. After 3 h, the reaction was completed as monitored by TLC (EA: PE=1 : 1), and stopped. TFA in the reaction solution was removed by vacuum distillation to obtain 2.06 g of a brown oily substance. The product was dried in an oven at 60°C for 12 h and the crude product was used directly in the next step without purification.

### Example 41 Synthesis of phenyl 4-(((1r,4r)-4-(3-(3-fluoro-4-(trifluoromethoxy) phenyl)ureido)cyclohexyl)oxy) acetate (SP-C01)

BTC (0.51 g, 1.17 mmol, 0.34 eq) and dry DCM (10 mL) were added sequentially into a 100 mL single-necked flask, cooled to -78°C in a cold trap, a solution (10 mL) of 3-fluoro-4-trifluoromethoxyaniline (1 g, 5.13 mmol, 1 eq) and $Et_3N$ (1.65 g, 15.39 mmol, 3 eq) in DCM was added dropwise within 30 min, and a resulting mixture was naturally heated to room temperature and stirred for another 4 h. The reaction after 4 h was monitored by TLC: a drop of the reaction solution was

taken and then placed in an EP tube, and memantine was added thereto, iodine fumigation was conducted (EA: PE=1 : 3), and the reaction was completed.

A mixed solution of phenyl 4-(((1r,4r)-4-aminocyclohexyl)oxy)acetate·TFA (1.86 g, 5.13 mmol, 1eq), $Et_3N$ (1.65 g, 15.39 mmol, 3 eq), and DCM (5 mL) was added to a 100 mL single-necked flask, and the above reaction solution was added dropwise thereto and the resulting mixture was reacted at room temperature. After 2 h, the reaction was completed as monitored by TLC (EA: PE=1 : 3, AcOH 2d), and the reaction was stopped. 15 mL of water was added thereto and the resulting mixture was extracted 2 times. The extraction liquid was washed once with 15 mL of saturated NaCl solution, and dried over anhydrous magnesium sulfate, followed by filtration suction, and concentrating to dryness under reduced pressure to obtain 0.23 g of a crude product as a yellow oily substance. The crude product was loaded into a column with 5 times silica gel after being mixed with 1.2 times silica gel, with an eluent (EA: PE=1 : 10) to obtain 0.42 g of a white solid with a yield of 17.3%. $^1H$ NMR (400 MHz, DMSO-$d_6$): δ (ppm) 8.727.69 (d, *J* = 2.5 Hz, 1H), 7.65-7.38 (m, 1H), 7.36-7.35 (m, 1H), 7.11-6.94 (m, 4H), 6.30 (d, *J* = 8.0 Hz, 1H), 4.29 (d, *J*= 4.0 Hz, 1H), 3.51 (s, 1H), 2.23 (s, 3H), 2.05 (d, *J* = 3.0 Hz, 2H), 2.02-1.91 (m, 2H), 1.48-1.34 (m, 4H). $^{13}C$ NMR (100 MHz, DMSO-$d_6$): δ (ppm) 169.9, 155.3, 154.6, 152.9, 144.3, 141.9, 124.7, 123.1, 116.8, 114.0, 114.0, 106.2, 105.9, 75.0, 47.8, 30.4, 30.2, 21.1. ESI MS: *m*/*z* 471.1 $[M + H]^+$.

### Example 42 Synthesis of 4-(((1r,4r)-4-(3-(3-fluoro-4-(trifluoromethoxy)phenyl) ureido)cyclohexyl)oxy)phenol (SP-C01b)

Phenyl 4-(((1r,4r)-4-(3-(3-fluoro-4-(trifluoromethoxy)phenyl)ureido)cyclohexyl) oxy)acetate (0.24 g, 0.51 mmol,1 eq), LiOH (2.1 g, 17.20 mmol,34 eq), and $H_2O$ (7 mL) were added sequentially into a 100 mL single-necked flask, and reacted at room temperature. After 1.5 h, the reaction was completed as monitored by TLC, and the reaction was stopped. The product was extracted with DCM (30 mL×3),and organic layers were combined, and dried over anhydrous sodium sulfate, followed by concentrating to dryness under reduced pressure to obtain 0.21 g of a crude product as a brown solid, with a yield of 96.33%.

### Example 43 Synthesis of methyl 4-(((1r,4r)-4-((tert-butoxycarbonyl)amino) cyclohexyl)oxy)benzoate

Cis-4-Boc-aminocyclohexanol (1.00 g, 4.64 mmol), methyl p-hydroxybenzoate (0.7 g, 4.64 mmol), TPP (1.8 g, 6.96 mmol), A4 molecular sieve (dried in advance in an oven at 120°C for 4 h), and THF (10 mL) were added sequentially into a 100 mL single-necked flask. The atmosphere was replaced with argon 3 times and the system was cooled to below -10°C in an ice-salt bath. A solution (5 mL) of DIAD (1.4 g, 6.96 mmol) in THF was added dropwise at one drop/two seconds. After 12 h, the reaction was monitored by TLC, EA: PE=1 : 1, and the reaction mixture was moved to 30°C and the 4A molecular sieve was supplemented. After 6 h, the reaction was mostly completed as monitored by TLC (EA: PE=1 : 1), and the reaction was stopped. The THF therein was removed by concentration under reduced pressure to obtain 5.2 g of a crude product of claybank oil, which was slurried without solid precipitation, and loaded into a column with 4 times silica gel after being mixed with 1.2 times silica gel, using an eluent of EA: PE=1 : 10, to obtain 0.73 g of a white solid with a yield of 45.1%.

### Example 44 Synthesis of methyl 4-(((1r,4r)-4-aminocyclohexyl)oxy)benzoate

Methyl (((1r,4r)-4-((tert-butoxycarbonyl)amino)cyclohexyl)oxy)benzoate (0.60 g, 2.09 mmol) and DCM (5 mL) were added sequentially to a 100 mL single-necked flask, and TFA (4 mL) was added dropwise at room temperature. After 3 h, the reaction was completed as monitored by TLC (EA: PE=1 : 1), and the reaction was stopped. TFA in the reaction solution was removed by vacuum distillation to obtain 0.62 g of a brown oily substance. The brown oily substance was dried in an oven at 60°C for 12 h and was used directly in the next step without purification.

### Example 45 Synthesis of methyl 4-(((1r,4r)-4-(3-(3-fluoro-4-(trifluoromethoxy) phenyl)ureido)cyclohexyl)oxy) benzoate

BTC (0.23 g, 0.79 mmol) and dry DCM (15 mL) were added sequentially into a 100 mL single-necked flask, and cooled to -78°C in a cold trap. A solution (10 mL) of 3-fluoro-4-trifluoromethoxyaniline (0.41 g, 2.32 mmol) and $Et_3N$ (1.41 g, 13.92 mmol) in DCM was added dropwise within 30 min, and a resulting mixture was naturally heated to room temperature and stirred for another 4 h. The reaction after 4 h was monitored by TLC: a drop of the reaction solution was taken and then placed into an EP tube, and methyl 4-(((1r,4r)-4-aminocyclohexyl)oxy)benzoate was added, EA: PE=1 : 3.

A mixed solution of methyl 4-(((1r,4r)-4-aminocyclohexyl)oxy)benzoate·TFA (0.58 g, 1.59 mmol), $Et_3N$ (1.41 g, 13.92 mmol), and DCM (10 mL) was added to a 100 mL single-necked flask, and reacted at room temperature. After 2 h, the reaction was completed as monitored by TLC (EA: PE=1 : 3), and the reaction was stopped. 15 mL of water was added thereto, and the resulting mixture was extracted 2 times. The extraction liquid was washed once with 15 mL of saturated

NaCl solution, and dried over anhydrous magnesium sulfate, followed by filtration suction, and concentrating to dryness under reduced pressure to obtain 0.93 g of a crude product as a yellow oily substance. The crude product was loaded into a column with 7 times silica gel after being mixed with 1.2 times silica gel, with an eluent (EA: PE=1 : 5) to obtain 0.40 g of a white solid with a yield of 40.0%. ESI-MS: m/z 453.2 $[M+H]^+$

**Example 46 Synthesis of 4-(((1r,4r)-4-(3-(3-fluoro-4-(trifluoromethoxy)phenyl) ureido)cyclohexyl)oxy)benzoic acid (SP-C01c)**

Methyl 4-(((1r,4r)-4-(3-(3-fluoro-4-(trifluoromethoxy)phenyl)ureido)cyclohexyl)oxy) benzoate (2.59 g, 5.50 mmol, 1 eq), THF (20 mL), $H_2O$ (2 mL), and LiOH (0.40 g, 16.50 mmol, 3 eq) were added sequentially into a 100 mL single-necked flask, and stirred at room temperature. After 3 h, the reaction was monitored by TLC (EA: PE=1 : 1) and stopped, and the THF therein was removed by concentration under reduced pressure. DCM (50 mL) was added thereto and a resulting mixture was extracted with water (15 mL$\times$2), and the aqueous layer was adjusted to pH=2 with 1N HCl, and concentrated under reduced pressure until a small amount of solvent remained, followed by filtration suction and rinsing with 5 mL of water to obtain 0.53 g of a light yellow solid. The yield of the 3 combination steps was 21.13%.

$^1H$ NMR (600 MHz, DMSO-$d_6$) $\delta$ 10.19 (s, 1H), 8.88 (s, 1H), 8.35 (s, 3H), 7.86 (d, *J*= 8.6 Hz, 2H), 7.78 (d, *J* = 8.5 Hz, 1H), 7.04 (d, *J* = 8.6 Hz, 5H), 6.85 (d, *J* = 8.3 Hz, 4H), 4.39 (tt, *J* = 10.0, 4.3 Hz, 1H), 3.04 (dt, *J* = 10.8, 5.5 Hz, 1H), 2.15 - 2.09 (m, 2H), 2.06 - 2.01 (m, 2H), 1.61 - 1.50 (m, 2H), 1.50 - 1.38 (m, 2H).

**Example 47 Synthesis of phenyl 4-(((1R,4r)-4-(3-((1R,3R,5S,7R)-3,5-dimethyladamantan-1-yl)ureido)cyclohexyl)oxy)acetate (SP-C01d)**

BTC (0.02 g, 0.07 mmol, 0.34 eq) and dry DCM (5 mL) were added sequentially into a 100 mL single-necked flask, cooled to -78°C in a cold trap, a solution (10 mL) of memantine (0.04 g, 0.22 mmol, 1 eq) and $Et_3N$ (0.07 g, 0.66 mmol, 3 eq) in DCM was added dropwise within 30 min, and a resulting mixture was naturally heated to room temperature and stirred for another 4 h. The reaction after 4 h was monitored by TLC: a drop of the reaction solution was taken and then placed in an EP tube, and 4-trifluoromethoxyaniline was added thereto, iodine fumigation was conducted (EA: PE=1 : 3), and the reaction was completed.

A mixed solution of phenyl 4-(((1r,4r)-4-aminocyclohexyl)oxy)acetate·TFA (0.08 g, 0.22 mmol, 1eq), $Et_3N$ (0.07 g, 0.66 mmol, 3 eq), and DCM (5 mL) was added to a 25 mL single-necked flask, and the reaction solution was added dropwise thereto, and a resulting mixture was reacted at room temperature. The reaction after 2 h was completed as monitored by TLC, and the reaction was stopped. 15 mL of water was added thereto, and the resulting mixture was extracted 2 times. The extraction liquid was washed once with 15 mL of saturated NaCl solution, and dried over anhydrous magnesium sulfate, followed by filtration suction, and concentrating to dryness under reduced pressure to obtain 0.10 g of a crude product as a yellow oily substance. The crude product was loaded into a column with 5 times silica gel after being mixed with 1.2 times silica gel, with an eluent (EA: PE=1 : 10) to obtain 32 mg of a white solid with a yield of 32.0%. $^1H$ NMR (400 MHz, DMSO-$d_6$): $\delta$ (ppm) 7.04 - 6.97 (d, 2H), 6.97 - 6.89 (d, 2H), 5.58 (d, *J* = 7.9 Hz, 1H), 5.41 (d, *J* = 3.2 Hz, 1H), 4.25 (tt, *J* = 9.9, 3.9 Hz, 1H), 3.35 (dd, *J* = 7.3, 3.7 Hz, 1H), 2.23 (s, 3H), 2.02 (ddt, *J* = 24.4, 8.4, 3.5 Hz, 3H), 1.87 - 1.80 (m, 2H), 1.67 (d, *J* = 3.1 Hz, 2H), 1.50 (s, 4H), 1.37 (ddd, *J* = 15.2, 9.1, 4.3 Hz, 2H), 1.31 - 1.22 (m, 4H), 1.22 - 1.16 (m, 2H), 1.16 - 1.10 (m, 1H), 1.07 (s, 2H), 0.80 (s, 6H). $^{13}C$ NMR (100 MHz, DMSO-$d_6$): $\delta$ (ppm) 169.94, 157.00, 155.37, 144.29, 123.06, 116.77, 75.17, 51.49, 50.87, 48.61, 47.33, 42.91, 41.00, 32.36, 30.89, 30.82, 30.61, 30.31, 30.10, 21.26. ESI MS: m/z 455.2 $[M + H]^+$.

**Example 48 Synthesis of tert-butyl ((1r,4r)-4-(4-acetylphenoxy)cyclohexyl) carbamate**

Cis-4-Boc-aminocyclohexanol (1 g, 4.64 mmol, 1eq), acetaminophen (0.70 g, 4.64 mmol, 1 eq), $PPh_3$ (1.83 g, 6.96 mmol, 1.5 eq), 4A molecular sieve (dried in advance in an oven at 120°C for 4 h), and THF (20 mL) were added sequentially into a 250 mL single-necked flask. The atmosphere was replaced with argon 3 times, and the system was cooled to -10°C in an ice-salt bath, and a solution (10 mL) of DIAD (1.21 g, 6.96 mmol, 1.5 eq) in THF was added dropwise at one drop/two seconds. After 12 h, the reaction was mostly completed as monitored by TLC (EA: PE=1 : 2), and the reaction was stopped. The THF therein was removed by concentration under reduced pressure to obtain 5.62 g of a claybank oily substance. The claybank oily substance was stirred with 10 mL of ethanol and 5 mL of petroleum ether for 2 h, and slurried, followed by filtration suction to obtain 0.90 g of a light yellow solid with a yield of 55.9%.

**Example 49 Synthesis of N-(4-(((1r,4r)-4-aminocyclohexyl)oxy)phenyl)acetamide**

Tert-butyl ((1r,4r)-4-(4-acetylphenoxy)cyclohexyl)carbamate (0.90 g, 2.58 mmol) and DCM (6 mL) were added sequentially to a 100 mL single-necked flask, and TFA (4 mL) was added dropwise at room temperature. After 3 h, the

reaction was completed as monitored by TLC (EA: PE=1 : 1), and the reaction was stopped. TFA in the reaction solution was removed by vacuum distillation to obtain 1.06 g of a crude product as a brown oily substance. The crude product was dried in an oven at 60°C for 12 h and then used directly in the next step without purification.

**Example 50 Synthesis of *N*-(4-(((1r,4r)-4-(3-(3-fluoro-4-(trifluoromethoxy) phenyl)ureido)cyclohexyl)oxy)phenyl)acetamide (SP-C01e)**

BTC (0.08 g, 0.26 mmol, 0.34 eq) and dry DCM (10 mL) were added sequentially into a 50 mL single-necked flask, and the resulting mixture was cooled to -78°C in a cold trap. A solution (5 mL) of 3-fluoro-4-trifluoromethoxyaniline (0.15 g, 0.77 mmol, 1 eq) and $Et_3N$ (0.47 g, 4.61 mmol, 3 eq) in DCM was added dropwise thereto within 15 min, and a resulting mixture was naturally heated to room temperature and stirred for another 0.5 h. After 0.5 h, the reaction mixture was monitored by TLC: a drop of the reaction solution was taken and then placed into an EP tube, and N-(4-(((1r,4r)-4-aminocyclohexyl)oxy)phenyl)acetamide·TFA was added thereto, EA: PE=1 : 1.

A mixed solution of *N*-(4-(((1r,4r)-4-aminocyclohexyl)oxy)phenyl)acetamide·TFA (0.30 g, 0.77 mmol), $Et_3N$ (0.47 g, 4.61 mmol), and DCM (5 mL) was added to a 100 mL single-necked flask, and the resulting mixture was reacted at room temperature. After 0.5 h, the reaction was completed as monitored by TLC (EA: PE=1 : 1), and the reaction was stopped. 15 mL of water was added thereto, and the resulting mixture was extracted 2 times. The extraction liquid was washed once with 15 mL of saturated NaCl solution, dried over anhydrous magnesium sulfate, followed by filtration suction, and concentrating to dryness under reduced pressure to obtain 0.52 g of a crude product as a yellow oily substance. The crude product was loaded into a column with 4 times silica gel after being mixed with 1.2 times silica gel, with an eluent (EA: PE=1 : 5, EA: PE=1 : 3) to obtain 0.11 g of a white solid with a yield of 33.4%. ESI-MS: m/z 470.2 $[M+H]^+$, 492.1 $[M+Na]^+$

$^1$H-NMR (400 MHz, DMSO-$d_6$): δ (ppm) 9.75 (s, 1H), 8.74 (s, 1H),7.67 (dd, *J*= 13.4, 2.4 Hz, 1H), 7.44 (d, *J*= 8.9 Hz, 2H), 7.38 (t, *J*= 8.7 Hz, 1H), 7.09 (d, *J*= 8.8 Hz, 1H), 6.86 (d, *J*= 8.9 Hz, 2H), 6.29 (d, *J*= 7.6 Hz, 1H), 4.26-4.21 (m, 1H), 3.53-3.48 (m, 1H), 2.03-2.20 (m, 2H), 2.20 (s, 3H),1.93-1.90 (m, 2H), 1.46-1.38 (m, 2H), 1.38-1.32 (m, 2H).

**Example 51 Synthesis of N-(4-hydroxyphenyl)-2-methylbutanamide**

2-Methylbutyric acid (3.0 g, 27.51 mmol, 1 eq) and dry THF (15 mL) were added into a 100 mL flask, and the resulting mixture was cooled to 0°C in an ice bath. $SOCl_2$ (3.93 g, 33.01 mmol, 1.2 eq) was added dropwise, and the resulting mixture was stirred for 30 min. 1.5 mL of the mixture was taken and then added into an EP tube, anhydrous methanol was then added thereto. The reaction was completed as monitored by TLC, and the reaction mixture was concentrated to dryness under reduced pressure.

*p*-Aminophenol (3.0 g, 27.51 mmol, 1 eq) and THF (5 mL) were added into a 100 mL round-bottom flask, and then the above solution (10 mL) of the 2-methylbutyryl chloride in THF was dropwise added thereto. The reaction was completed as monitored by TLC until the oxidation of raw materials, then the reaction was stopped. The reaction mixture was concentrated under reduced pressure to remove THF. The remaining was extracted 2 times by adding DCM (20 mL) and water (20 mL) thereto, and the extraction liquid was washed once with saturated NaCl solution (25 mL), and dried over anhydrous magnesium sulfate, followed by filtration suction. The resulting organic phase was concentrated under reduced pressure to obtain 16.5 g of a yellow oily substance, which was loaded into a column with 4 times silica gel after being mixed with 1.2 times silica gel, and subjected to column chromatography with an eluent (EA: PE=1 : 5) to obtain 4.85 g of a white solid with a yield of 91.3%. ESI-MS: m/z 194.1 $[M+H]^+$, 216.1 $[M+Na]^+$

**Example 52 Synthesis of tert-butyl ((1r,4r)-4-(4-(2-methylbutanamido) phenoxy)cyclohexyl)carbamate**

Cis-4-Boc-aminocyclohexanol (2.00 g, 9.29 mmol, 1 eq), N-(4-hydroxyphenyl)-2-methylbutanamide (1.79 g, 9.29 mmol, 1 eq), TPP (3.65 g, 13.9 mmol, 1.5 eq), A4 molecular sieve (dried in advance in an oven at 120°C for 4 h), and THF (15 mL) were added into a 100 mL single-necked flask in sequence. The atmosphere was replaced with argon 3 times and the system was cooled to below -10°C in an ice-salt bath. A solution (5 mL) of DIAD (2.43 g, 13.9 mmol, 1.5 eq) in THF was added dropwise at one drop/two seconds, and the resulting system was then heated to room temperature. After 12 h, the reaction was mostly completed as monitored by TLC (EA: PE=1 : 1), and the reaction was stopped. The reaction mixture was concentrated under reduced pressure to remove part of the THF, and 15 mL of anhydrous ethanol and 15 mL of ether were added thereto to slurry. 2.24 g of a light pink solid was obtained by filtration suction. The obtained solid was monitored by TLC, dissolved in 20 mL of DCM, and washed with saturated $Na_2CO_3$ solution (16 mL). The organic layer was dried over anhydrous magnesium sulfate, followed by filtration suction, and concentrating to dryness under reduced pressure to obtain 1.94 g of a pure product as a white solid, with a yield of 53.6%.

### Example 53 Synthesis of *N*-(4-(((1r,4r)-4-aminocyclohexyl)oxy)phenyl)-2-methylbutanamide

Tert-butyl ((1r,4r)-4-(4-(2-methylbutanamido)phenoxy)cyclohexyl)carbamate (1.94 g, 4.97 mmol) and DCM (5 mL) were added sequentially into a 100 mL single-necked flask, and TFA (6 mL) was added dropwise at room temperature. After 3 h, the reaction was completed as monitored by TLC (EA: PE=1 : 1), and the reaction was stopped. TFA in the reaction solution was removed by concentration under reduced pressure to obtain 1.92 g of a brown oily substance, i.e., N-(4-(((1r,4r)-4-aminocyclohexyl)oxy)phenyl)-2-methylbutanamide·TFA. The product was dried in an oven at 60°C for 12 h and used directly in the next step without purification.

### Example 54 Synthesis of *N*-(4-(((1R,4r)-4-(3-((1R,3R,5S,7R)-3,5-dimethyladamantan-1-yl)ureido)cyclohexyl) oxy)phenyl)acetamide (SP-C01f)

BTC (0.14 g, 0.47 mmol) and dry DCM (10 mL) were added sequentially into a 100 mL single-necked flask, and a resulting mixture was cooled to -78°C in a cold trap. A solution (10 mL) of memantine (0.25 g, 1.41 mmol) and $Et_3N$ (0.85 g, 8.46 mmol) in DCM was added dropwise within 30 min, and a resulting system was naturally heated to room temperature and stirred for another 4 h.

A mixed solution of A4 (0.35 g, 1.41 mmol), $Et_3N$ (0.28 g, 2.82 mmol), and DCM (5 mL) was added into a 100 mL single-necked flask, and reacted at room temperature. After 2 h, the reaction was monitored by TLC, and stopped. 15 mL of water was added thereto, and the resulting mixture was extracted 2 times. The extraction liquid was washed once with 15 mL of saturated NaCl solution, and dried over anhydrous magnesium sulfate, followed by filtration suction, and concentrating to dryness under reduced pressure to obtain 0.52 g of a crude product as a yellow oily substance. The crude product was loaded into a column with 4 times silica gel after being mixed with 1.2 times silica gel, with an eluent (EA: PE=1 : 5) to obtain 0.21 g of a product with a yield of 36.7%.

$^1$H-NMR (400 MHz, DMSO-$d_6$): δ (ppm) 9.73 (s, 1H), 7.43 (d, *J*= 8.96 Hz, 2H), 6.84 (d, *J*= 8.96 Hz, 2H), 5.54 (d, *J*= 7.60 Hz, 1H), 5.41 (s, 1H), 4.22-4.17 (m, 1H), 3.37-3.34 (m, 1H), 2.05-2.03 (m, 1H), 1.99 (s, 3H), 1.97-1.94 (m, 1H), 1.84-1.81 (m, 2H), 1.67-1.66 (m, 2H), 1.52-1.46 (m, 4H), 1.40-1.34 (m, 2H), 1.30 (s, 1H), 1.27-1.24 (m, 4H), 1.19-1.14 (m, 2H), 1.07 (s, 2H), 0.79 (s, 6H).

### Example 55 Synthesis of *N*-(4-(((1r,4r)-4-(3-(3-fluoro-4-(trifluoromethoxy)phenyl) ureido)cyclohexyl)oxy)benzene)-2-methylbutanamide (SP-C01g)

BTC (0.10 g, 0.35 mmol, 1 eq) and dry DCM (10 mL) were added sequentially into a 50 mL single-necked flask, and cooled to -78°C in a cold trap, a solution (10 mL) of 3-fluoro-4-trifluoromethoxyaniline (0.2 g, 1.02 mmol, 1 eq) and $Et_3N$ (0.93 g, 9.22 mmol, 3 eq) in DCM was added dropwise within 30 min, and a resulting mixture was naturally heated to room temperature and stirred for another 0.5 h. The reaction was monitored by TLC: a drop of the reaction solution was taken and then placed in an EP tube, and memantine was added thereto.

A mixed solution of N-(4-(((1r,4r)-4-aminocyclohexyl)oxy)phenyl)-2-methylbutanamide·TFA (0.41 g, 1.02 mmol, 1 eq), $Et_3N$ (0.93 g, 9.22 mmol, 3 eq), and DCM (10 mL) was added to a 100 mL single-necked flask, and a resulting mixture was reacted at room temperature. After 0.5 h, the reaction was completed as monitored by TLC (EA: PE=1 : 2, AcOH 1d), and the reaction was stopped. 15 mL of water was added thereto, and the reaction mixture was extracted 2 times. The extraction liquid was washed once with 15 mL of saturated NaCl solution, and dried over anhydrous magnesium sulfate, followed by filtration suction, and concentrating to dryness under reduced pressure to obtain 0.74 g of a crude product as a yellow oily substance. The crude product was loaded into a column with 7 times silica gel after being mixed with 1.2 times silica gel, with an eluent (EA: PE=1 : 5, EA: PE=1 : 2) to obtain 0.13 g of a yellow solid. The product did not have enough purity, and was then placed in a 25 mL single-necked flask and dissolved in 0.5 mL of DCM. Then 4 mL of petroleum ether was added thereto to precipitate a white solid, followed by filtration suction to obtain 62 mg of a product with a yield of 11.9%, while recovering a filtrate. ESI-MS: m/z 512.3 $[M+H]^+$

$^1$H-NMR (400 MHz, DMSO-$d_6$): δ (ppm) 9.64 (s, 1H), 8.17 (s, 1H),7.67 (dd, *J*= 13.4, 2.1 Hz, 1H), 7.48 (d, *J*= 8.8 Hz, 2H), 7.38 (t, *J*= 8.9 Hz, 1H), 7.10-7.08 (m, 1H), 6.87 (d, *J*= 8.8 Hz, 2H), 6.27 (d, *J*= 7.4 Hz, 1H), 4.26-4.22 (m, 1H), 3.53-3.52 (m, 1H), 2.36-2.31 (m, 1H), 2.03-2.00 (m, 2H), 1.93-1.90 (m, 2H), 1.63-1.56 (m, 1H), 1.48-1.30 (m, 5H), 1.06 (d, *J*= 6.7 Hz, 3H), 0.85 (t, *J*= 7.4 Hz, 3H).

### Example 56 Synthesis of tert-butyl ((1r,4r)-4-(4-nitrophenoxy)cyclohexyl)carbamate

Cis-4-Boc-aminocyclohexanol (10.00 g, 0.046 mol, 1 eq), 4-nitrophenol (6.47 g, 0.046 mol, 1 eq), $PPh_3$ (13.95 g, 0.069 mmol, 1.5 eq), and THF (10 mL) were added sequentially into a 100 mL three-necked flask, and cooled to below -10°C in an ice-salt bath. A solution (20 mL) of DIAD (18.10 g, 0.069 mmol, 1.5 eq) in THF was added dropwise at one drop/two seconds. After 8 h, the reaction was completed as monitored by TLC (EA: PE=1 : 2), and the reaction was

stopped. The THF therein was removed by concentration under reduced pressure to obtain 12.21 g of claybank oily substance, which was stirred with 50 mL of ethanol for 2 h, and slurried, followed by filtration suction to obtain 12.46 g of a yellow solid, with a yield of 80.58%.

**Example 57 Synthesis of (1r,4r)-4-(4-nitrophenoxy)cyclohexane-1-amine**

Tert-butyl ((1r,4r)-4-(4-nitrophenoxy)cyclohexyl)carbamate (0.60 g, 2.09 mmol) and DCM (5 mL) were added sequentially to a 100 mL single-necked flask, and TFA (4 mL) was added dropwise at room temperature. After 3 h, the reaction was completed as monitored by TLC (EA: PE=1 : 1), and the reaction was stopped. TFA in the reaction solution was removed by vacuum distillation to obtain 0.62 g of a brown oily substance. The brown oily substance was dried in an oven at 60°C for 12 h and used directly in the next step without purification.

**Example 58 Synthesis of 1-((1r,3R,5S,7R)-3,5-dimethyladamantan-1-yl)-3-((1r,4R)-4-(4-nitrophenoxy)cyclohexyl)urea**

BTC (5.50 g, 18.53 mmol, 0.5 eq) and dry DCM (50 mL) were added sequentially into a 250 mL single-necked flask, and cooled to -80°C in a cold trap, a solution (50 mL) of memantine (6.56 g, 37.06 mmol, 1 eq) and $Et_3N$ (30.00 g, 296.48 mmol, 8 eq) in DCM was added dropwise within 4 h, and a resulting mixture was naturally heated to room temperature. After 30 min, the reaction was completed as monitored by TLC, and the reaction was stopped. The reaction solution was evaporated to dryness, DCM (50 mL) and $Et_3N$ (30.00 g, 296.48 mmol, 8 eq) were added thereto, and a resulting mixture was cooled to 0°C in an ice bath, and a solution (50 mL) of I9 (8.89 g, 37.06 mmol, 1 eq) in DCM was added dropwise. The resulting mixture was reacted at room temperature. After 30 min, the reaction was completed as monitored by TLC and stopped. The DCM therein was removed by concentration under reduced pressure to obtain 17.82 g of a reddish brown oil, which was directly used for the next step without purification.

**Example 59 Synthesis of 1-((1r,4R)-4-(4-aminophenoxy)cyclohexyl)-3-((1r,3R,5S,7R)-3,5-dimethyladamantan-1-yl)urea (SP-C01h)**

2-Methylbutyric acid (3.0 g, 27.51 mmol, 1 eq), dry THF (10 mL), HATU (0.034 g, 0.29 mmol, 1.2 eq), and DIEA (0.18 g, 1.74 mmol, 6 eq) were added into a 25 mL flask, and stirred for 30 min. A solution (5 mL) of 1-((1r,4R)-4-(4-aminophenoxy)cyclohexyl)-3-((1r,3R,5S,7R)-3,5-dimethyladamantan-1-yl)urea (0.1 g, 0.24 mmol, 1 eq) in THF was added thereto. The reaction was completed as monitored by TLC, and the reaction was stopped, and a product was subjected to filtration suction to obtain 72 mg of a white solid, with a yield of 60.56%.

$^1$H NMR (400 MHz, Chloroform-d) δ 7.37(d, $J$ = 8.8 Hz, 2H), 7.21 (s, 1H), 6.82 (d, $J$ = 8.8 Hz, 2H), 4.14 - 4.06 (m, 1H), 3.56 - 3.51 (m, 1H), 2.38 (q, $J$ = 7.6 Hz, 2H), 2.17 - 1.99 (m, 6H), 1.78 (d, $J$ = 3.1 Hz, 2H), 1.56 - 1.41 (m, 3H), 1.39 - 1.08 (m, 12H), 0.84 (s, 6H).$^{13}$C NMR (101 MHz, Chloroform-d) δ 156.72, 154.42, 131.17, 121.86, 116.60, 75.57, 50.64, 48.46, 42.72, 41.02, 32.48, 31.04, 30.59, 30.23, 30.21, 30.10, 9.81.

**Example 60 Synthesis of methyl 4-(((1r,4r)-4-((tert-butoxycarbonyl)amino) cyclohexyl)oxy)benzoate**

Cis-4-Boc-aminocyclohexanol (1.00 g, 4.64 mmol), methyl p-hydroxybenzoate (0.7 g, 4.64 mmol), TPP (1.8 g, 6.96 mmol), A4 molecular sieve (dried in advance in an oven at 120°C for 4 h), and THF (10 mL) were added sequentially into a 100 mL single-necked flask. The atmosphere was replaced with argon 3 times and the system was cooled to below -10°C in an ice-salt bath. A solution (5 mL) of DIAD (1.4 g, 6.96 mmol) in THF was added dropwise at one drop/two seconds. After 12 h, the reaction was monitored by TLC, and the reaction mixture was moved to 30°C and the 4A molecular sieve was supplemented. After 6 h, the reaction was mostly completed as monitored by TLC, and the reaction was stopped. The THF therein was removed by concentration under reduced pressure to obtain 5.2 g of a crude product as a claybank oil, which was slurried without solid precipitation. The crude product was loaded into a column with 4 times silica gel after being mixed with 1.2 times silica gel, using an eluent of EA: PE=1 : 10, to obtain 0.73 g of a white solid, with a yield of 45.1%.

**Example 61 Synthesis of methyl 4-(((1r,4r)-4-aminocyclohexyl)oxy)benzoate**

Methyl 4-(((1r,4r)-4-((tert-butoxycarbonyl)amino)cyclohexyl)oxy)benzoate (0.60 g, 2.09 mmol) and DCM (5 mL) were added sequentially to a 100 mL single-necked flask, and TFA (4 mL) was added dropwise at room temperature. After 3 h, the reaction was completed as monitored by TLC, and the reaction was stopped. TFA in the reaction solution was removed by vacuum distillation to obtain 0.62 g of a crude product as a brown oily substance. The crude product was dried in an oven at 60°C for 12 h and used directly in the next step without purification.

**Example 62 Synthesis of methyl 4-(((1r,4r)-4-(3-(4-(trifluoromethoxy)phenyl) ureido)cyclohexyl)oxy)benzoate**

BTC (0.23 g, 0.79 mmol) and dry DCM (15 mL) were added sequentially into a 100 mL single-necked flask, and cooled to -78°C in a cold trap, a solution (10 mL) of free *p*-trifluoromethoxyaniline (0.41 g, 2.32 mmol) and $Et_3N$ (1.41 g, 13.92 mmol) in DCM was added dropwise within 30 min, and a resulting mixture was naturally heated to room temperature and stirred for another 4 h.

A mixed solution of I15·TFA (0.58 g, 1.59 mmol), $Et_3N$ (1.41 g, 13.92 mmol), and DCM (10 mL) was added into a 100 mL single-necked flask, and a resulting mixture was reacted at room temperature. After 2 h, the reaction was completed as monitored by TLC, and the reaction was stopped. 15 mL of water was added thereto and a resulting mixture was extracted 2 times. The extraction liquid was washed once with 15 mL of saturated NaCl solution, and dried over anhydrous magnesium sulfate, followed by filtration suction, and concentrating to dryness under reduced pressure to obtain 0.93 g of a crude product as a yellow oily substance. The crude product was loaded into a column with 7 times silica gel after being mixed with 1.2 times silica gel, with an eluent (EA: PE=1 : 5) to obtain 0.40 g of a white solid, with a yield of 40.0%. ESI-MS: m/z 453.2 $[M+H]^+$

**Example 63 Synthesis of 4-(((1r,4r)-4-(3-(4-(trifluoromethoxy)phenyl) ureido)cyclohexyl)oxy)benzoic acid**

Methyl 4-(((1r,4r)-4-(3-(4-(trifluoromethoxy)phenyl)ureido)cyclohexyl)oxy)benzoate (0.15 g, 0.33 mmol), THF (3 mL), $H_2O$ (2 mL), and LiOH (39.8 mg, 0.99 mmol) were added into a 25 mL single-necked flask in sequence, and stirred at room temperature. After 3 h, a small portion of reactants were reacted as monitored by TLC. The reaction mixture was heated to 30°C and 0.1 g of LiOH was supplemented. After 2 h, a part of reactants were reacted as monitored by TLC. The reaction mixture was heated to 50°C and 0.2 g of LiOH was supplemented. After 2 h, the reaction was monitored by TLC and stopped. The reaction mixture was adjusted to a pH value of 2 with 3N HCl, and then concentrated under reduced pressure until a small amount of solvent was present, followed by filtration suction and washing with 5 mL of water to obtain 70 mg of a light yellow solid with a yield of 48.6%.

**Example 64 Synthesis of 4-(((1r,4r)-4-(3-(3-fluoro-4-(trifluoromethoxy)phenyl) ureido)cyclohexyl)oxy)benzamide (SP-C01i)**

4-(((1r,4r)-4-(3-(4-(trifluoromethoxy)phenyl)ureido)cyclohexyl)oxy)benzoic acid (0.17 g, 0.39 mmol) and dry THF (10 mL) were added into a 25 mL flask at room temperature. HATU (0.18 g, 0.46 mmol) was added thereto and a resulting mixture was stirred for 15 min. DIEA (0.12 g, 0.97 mmol) was added dropwise, followed by stirring for 0.5 h, and $NH_3$ $H_2O$ (0.3 mL) was added thereto. After 0.5 h, the reaction was completed as monitored by TLC. The reaction mixture was concentrated under reduced pressure to remove THF, and then extracted 2 times by adding DCM (12 mL) and water (10 mL). The extraction liquid was washed once with saturated NaCl solution (25 mL), and dried over anhydrous magnesium sulfate, followed by filtration suction. The organic phase was concentrated under reduced pressure to obtain 0.20 g of a yellow oily substance, which was loaded into a column with 4 times silica gel after being mixed with 1.2 times silica gel, and then subjected to column chromatography with an eluent (EA: PE=1 : 3) to obtain 0.12 g of a pale yellow solid with a yield of 70.5%.

$^1H$ NMR (400 MHz, DMSO-$d_6$) δ 8.81 (s, 1H), 7.82 (dd, *J* = 9.4, 2.7 Hz, 3H), 7.54 - 7.45 (m, 2H), 7.21 (d, *J* = 8.5 Hz, 2H), 7.14 (s, 1H), 7.02 - 6.94 (m, 2H), 6.48 (d, *J* = 7.6 Hz, 1H), 4.43 (tt, *J* = 9.8, 4.0 Hz, 1H), 3.54 (ddt, *J* = 14.2, 10.6, 5.3 Hz, 1H), 2.06 (dd, *J* = 12.6, 4.3 Hz, 2H), 2.01 - 1.89 (m, 2H), 1.55 - 1.30 (m, 4H). $^{13}C$ NMR (101 MHz, DMSO-$d_6$) δ 167.89, 160.26, 155.02, 142.36, 140.48, 129.85, 126.78, 122.03, 121.97, 119.43, 118.99, 115.28, 74.64, 47.64, 30.44, 30.15.

**Example 65 Synthesis of *N*-(4-(((1R,4r)-4-(3-((1R,3R,5S,7R)-3,5-dimethyladamantan-1-yl)ureido)cyclohexyl) oxy)phenyl)propanamide (SP-C01j)**

Propionic acid (0.02 g, 0.24 mmol, 1 eq), dry THF (10 mL), HATU (0.034 g, 0.29 mmol, 1.2 eq), and DIEA (0.18 g, 1.74 mmol, 6 eq) were added into a 25 mL flask, and stirred for 30 min. A solution (5 mL) of 1-((1r,4R)-4-(4-aminophenoxy) cyclohexyl)-3-((1r,3R,5S,7R)-3,5-dimethyladamantan-1-yl)urea (0.1 g, 0.24 mmol, 1 eq) in THF was added thereto. The reaction was completed as monitored by TLC (EA: PE=1 : 2), and the reaction was stopped, and the reaction mixture was subjected to filtration suction to obtain 80 mg of a white solid with a yield of 71.32%.

$^1H$ NMR (400 MHz, DMSO-*d6*) δ 9.66 (s, 1H), 7.48(d, *J* = 8.8 Hz, 2H), 6.85 (d, *J* = 8.8 Hz, 2H), 5.58 (d, *J* = 7.5 Hz, 1H), 5.42 (s, 1H), 4.22 - 4.18 (m, 1H), 2.38 - 2.31 (m, 1H), 2.05 (s, 1H), 1.97(d, *J* = 10.5 Hz, 2H), 1.82(d, *J* = 10.5 Hz, 2H), 1.68 (s, 2H), 1.62 - 1.55 (m, 2H), 1.07 - 1.05 (m, 2H), 0.83 (t, *J* = 7.4 Hz, 3H) 0.80 (s, 6H).

**Example 66 Synthesis of *N*-(4-(((1r,4r)-4-(3-(3-fluoro-4-(trifluoromethoxy)phenyl)ureido)cyclohexyl)oxy)phenyl)acetamide (SP-C01k)**

BTC (0.08 g, 0.26 mmol) and dry DCM (10 mL) were added sequentially into a 50 mL single-necked flask, and cooled to -78°C in a cold trap, a solution (5 mL) of 3-fluoro-4-trifluoromethoxyaniline (0.15 g, 0.77 mmol) and $Et_3N$ (0.47 g, 4.61 mmol) in DCM was added dropwise within 15 min, and a resulting mixture was naturally heated to room temperature and stirred for another 0.5 h. After 0.5 h, the reaction was monitored by TLC, EA: PE=1 : 1.

A mixed solution of N-(4-(((1r,4r)-4-aminocyclohexyl)oxy)phenyl)acetamide·TFA (0.30 g, 0.77 mmol), $Et_3N$ (0.47 g, 4.61 mmol), and DCM (5 mL) was added to a 100 mL single-necked flask, and the resulting mixture was reacted at room temperature. After 0.5 h, the reaction was completed as monitored by TLC, and the reaction was stopped. The reaction mixture was extracted 2 times after adding 15 mL of water. The extraction liquid was washed once with 15 mL of saturated NaCl solution, and dried over anhydrous magnesium sulfate, followed by filtration suction, and concentrating to dryness under reduced pressure to obtain 0.52 g of a crude product as a yellow oily substance. The crude product was loaded into a column with 4 times silica gel after being mixed with 1.2 times silica gel, with an eluent (EA: PE=1 : 5, EA: PE=1 : 3) to obtain 0.21 g of a white solid with a yield of 36.7%.

**Example 67 Synthesis of ethyl 3-((4-((((1r,4r)-4-(3-(3-fluoro-4-(trifluoromethoxy)phenyl)ureido)cyclohexyl)oxy)phenyl)amino)-3-oxopropanoate (SP-C01s)**

Monoethyl malonate (0.16 g, 0.12 mmol, 1 eq), dry THF (5 mL), HATU (0.017 g, 0.29 mmol, 1.2 eq), and DIEA (0.09 g, 0.87 mmol, 6 eq) were added into a 25 mL flask, and stirred for 30 min. A solution (5 mL) of 1-((1r,4r)-4-(4-aminophenoxy)cyclohexyl)-3-(3-fluoro-4-(trifluoromethoxy)phenyl)urea (0.05 g, 0.12 mmol, 1 eq) in THF was added thereto. The reaction was completed as monitored by TLC (EA: PE=1 : 1, AcOH 1d), and the reaction was stopped, and a resulting reaction mixture was subjected to filtration suction to obtain 42 mg of a white solid with a yield of 64.67%. $^1H$ NMR (400 MHz, DMSO-$d_6$) δ 9.68 (s, 1H), 8.73 (s, 1H), 7.68 (dd, *J* = 13.4, 2.5 Hz, 1H), 7.50 - 7.43 (m, 2H), 7.39 (t, *J* = 8.9 Hz, 1H), 7.10 (dt, *J* = 9.1, 1.8 Hz, 1H), 6.92 - 6.83 (m, 2H), 6.30 (d, *J* = 7.6 Hz, 1H), 4.24 (tt, *J* = 9.6, 4.0 Hz, 1H), 3.63 - 3.46 (m, 1H), 2.27 (q, *J* = 7.5 Hz, 2H), 2.02 (dd, *J* = 12.7, 4.4 Hz, 2H), 1.92 (dd, *J* = 12.9, 4.0 Hz, 2H), 1.51 - 1.27 (m, 4H), 1.07 (t, *J* = 7.5 Hz, 3H).

**Example 68 Synthesis of methyl 4-(4-(((1r,4r)-4-((tert-butoxycarbonyl)amino)cyclohexyl)oxy)phenoxy)butanoate**

Tert-butyl ((1r,4r)-4-(4-hydroxyphenoxy)cyclohexyl)carbamate (0.30 g, 0.98 mmol, 1 eq), $K_2CO_3$ (0.40 g, 2.92 mmol, 3 eq), KI (0.02 g, 0.10 mmol, 0.1 eq), and TBAB (0.03 g, 0.10 mmol, 0.1 eq) were added sequentially into a 25 mL single-necked flask, and MeCN (15 mL) was then added thereto. Methyl 4-bromobutyrate (0.26 g, 1.46 mmol, 1.5 eq) was added dropwise, the atmosphere was replaced with Ar 3 times, and a resulting system was heated to reflux. After 3 h, the reaction was monitored by TLC (EA: PE=1 : 3) and stopped. The reaction mixture was concentrated to dryness under reduced pressure, and the remianing was extracted by adding water (20 mL) and DCM (20 mL×2). The organic layers were combined, washed with water (10 mL), washed with saturated NaCl solution (10 mL), and dried over anhydrous magnesium sulfate, followed by filtration, and concentrating under reduced pressure to obtain 0.46 g of a brownlish black oily liquid. The crude product was purified by silica gel column chromatography, with silica gel 1.5 times the amount of the sample for mixing with the sample and 7 times the amount of the sample for silica gel column loading, using an eluent of EA: PE=1 : 15, to obtain 0.12 g of a white solid with a yield of 30.07%.

**Example 69 Synthesis of methyl 4-(4-(((1r,4r)-4-aminocyclohexyl)oxy) phenoxy)butyrate**

Methyl 4-(4-(((1r,4r)-4-((tert-butoxycarbonyl)amino)cyclohexyl)oxy)phenoxy)butanoate (0.12 g, 0.29 mmol, 1 eq) and DCM (8 mL) were added sequentially to a 100 mL single-necked flask, and cooled to 0°C in an ice bath, and TFA (0.5 mL) was then added dropwise. After 2 h, the reaction was completed as monitored by TLC (EA: PE=1 : 3), and the reaction was stopped. DCM and TFA in the reaction solution were removed by vacuum distillation to obtain 0.11 g of a crude product of a pale yellow solid, and the crude product was directly used for the next step without purification. ESI MS: m/z 308.1 $[M + H]^+$.

**Example 70 Synthesis of methyl 4-(4-(((1R,4r)-4-(3-((1R,3R,5S,7R)-3,5-dimethyladamantan-1-yl)ureido)cyclohexyl)oxy)phenoxy)butanoate (SP-C20)**

BTC (0.97 g, 3.26 mmol, 0.5 eq) and dry DCM (10 mL) were added sequentially into a 250 mL single-necked flask, and cooled to -80°C in a cold trap, and a solution (15 mL) of memantine (1.15 g, 6.51 mmol, 1 eq) and $Et_3N$ (5.27 g, 52.08

mmol, 8 eq) in DCM was added dropwise within 30 min, and a resulting mixture was naturally heated to room temperature. After 30 min, the reaction was completed as monitored by TLC (EA: PE=1 : 3), and the reaction was stopped. The reaction solution was evaporated to dryness, and DCM (15 mL) and $Et_3N$ (5.27 g, 52.08 mmol, 8 eq) were added thereto. A resulting mixture was cooled to 0°C in an ice bath, and a solution (10 mL) of methyl 4-(4-(((1r,4r)-4-aminocyclohexyl)oxy)phenoxy) butanoate (2.00 g, 6.51 mmol, 1 eq) in DCM was added dropwise. A resulting mixture was reacted at room temperature. After 30 min, the reaction was monitored by TLC (EA: PE=1 : 2), and stopped. The DCM therein was removed by concentration under reduced pressure to obtain 4.05 g of a yellow oily substance, which was mixed with 1.5 times silica gel and then loaded into a column with 4 times silica gel (EA: PE=1 : 7) to obtain 1.05 g of a white solid with a yield of 31.48%. $^1H$ NMR (400 MHz, DMSO-$d_6$) δ 6.94 - 6.82 (m, 4H), 5.65 (d, *J* = 7.6 Hz, 1H), 5.49 (s, 1H), 4.19 (tt, *J* = 10.0, 3.9 Hz, 1H), 3.96 (t, *J* = 6.3 Hz, 2H), 3.66 (s, 3H), 2.51 (t, *J* = 7.3 Hz, 3H), 2.10 (p, *J* = 3.2 Hz, 1H), 2.03 - 1.93 (m, 4H), 1.92 - 1.83 (m, 2H), 1.73 (d, *J*= 3.1 Hz, 2H), 1.55 (s, 4H), 1.49 - 1.15 (m, 9H), 1.13 (s, 2H), 0.85 (s, 6H). $^{13}C$ NMR (101 MHz, DMSO-$d_6$) δ 173.54, 157.00, 153.02, 151.71, 117.66, 115.77, 75.52, 67.31, 51.79, 51.47, 50.86, 48.60, 47.37, 42.90, 40.99, 32.36, 30.85, 30.61, 30.43, 30.09, 24.77.

**Example 71 Synthesis of 4-(4-(((1R,4r)-4-(3-((1R,3R,5S,7R)-3,5-dimethyladamantan-1-yl)ureido)cyclohexyl)oxy) phenoxy)butanoic acid (SP-C21)**

SP-C20 (0.50 g, 0.98 mmol, 1 eq), THF (15 mL), $H_2O$ (5 mL), and LiOH (0.10 g, 2.93 mmol, 3 eq) were added into a 25 mL single-necked flask in sequence, and stirred at room temperature. After 1.5 h, the reaction was monitored by TLC (EA: PE=1 : 3), and stopped. The THF therein was removed by concentration under reduced pressure, the remaining was extracted with EA (20 mL), and the resulting aqueous layer was adjusted to pH=2 with 1N HCl, followed by filtration suction under reduced pressure to obtain 0.42 g of a pale yellow solid with a yield of 86.01%. $^1H$ NMR (400 MHz, DMSO-$d_6$) δ 6.94 - 6.74 (m, 4H), 4.16 - 4.10 (m, 1H), 3.90 (t, *J* = 6.4 Hz, 4H), 3.90 (t, *J* = 6.4 Hz, 2H),3.33 (tt, *J* = 10.4, 3.8 Hz, 1H), 2.37 (t, *J* = 7.3 Hz, 2H), 2.04 (dt, *J* = 6.8, 3.3 Hz, 1H), 1.97 (d, *J* = 4.1 Hz, 1H), 1.92 (dt, *J* = 14.1, 6.1 Hz, 3H), 1.82 (dd, *J* = 13.5, 3.6 Hz, 2H), 1.68 - 1.67 (m, 2H), 1.52 - 1.44 (m, 4H), 1.41 - 1.11 (m, 9H), 1.09 - 1.04 (m, 2H), 0.80 (s, 6H). $^{13}C$ NMR (101 MHz, DMSO-$d_6$) δ 174.60, 157.05, 153.08, 151.68, 117.66, 115.79, 75.50, 67.42, 51.47, 51.39, 50.86, 48.59, 48.55, 47.35, 42.94, 42.90, 40.98, 32.46, 32.36, 30.82, 30.62, 30.39, 30.09, 24.80.

**Example 72 Synthesis of methyl 4-(4-(((1r,4r)-4-(3-(3-fluoro-4-(trifluoromethoxy)phenyl)ureido)cyclohexyl)oxy) phenoxy)butanoate (SP-C23)**

BTC (0.03 g, 0.10 mmol, 0.34 eq) and dry DCM (5 mL) were added sequentially into a 25 mL single-necked flask, and cooled to -80°C in a cold trap, a solution (10 mL) of 4-trifluoromethoxyaniline (0.05 g, 0.29 mmol, 1 eq) and $Et_3N$ (0.09 g, 0.87 mmol, 3 eq) in DCM was added dropwise within 5 min, and a resulting mixture was naturally heated to room temperature. After 10 min, the reaction was completed as monitored by TLC (EA: PE=1 : 3), and the reaction was stopped. The reaction solution was evaporated to dryness, and DCM (10 mL) and $Et_3N$ (0.09 g, 0.87 mmol, 3 eq) were added. A resulting mixture was cooled to 0°C in an ice bath, and a solution (5 mL) of methyl 4-(4-(((1r,4r)-4-aminocyclohexyl)oxy) phenoxy)butanoate (0.09 g, 0.29 mmol, 1 eq) in DCM was added dropwise. A resulting mixture was reacted at room temperature. After 30 min, the reaction was completed as monitored by TLC (EA: PE=1 : 2), and stopped. The DCM therein was removed by concentration under reduced pressure to obtain 0.15 g of a crude product as a white solid. The crude product was mixed with 1.5 times silica gel and then loaded into a column with 4 times silica gel (EA: PE=1 : 7) to obtain 0.10 g of a white solid with a yield of 65.28%. $^1H$ NMR (400 MHz, DMSO-$d_6$) δ 9.26 (s, 1H), 7.69 (dd, *J* = 13.5, 2.5 Hz, 1H), 7.37 (td, *J* = 9.0, 1.2 Hz, 1H), 7.12 (ddd, *J* = 9.0, 2.6, 1.4 Hz, 1H), 6.91 - 6.79 (m, 4H), 6.77 (d, *J* = 7.6 Hz, 1H), 4.17 (tt, *J* = 9.3, 3.8 Hz, 1H), 3.91 (t, *J* = 6.3 Hz, 2H), 3.61 (s, 4H), 3.57 - 3.45 (m, 1H), 2.46 (t, *J* = 7.3 Hz, 2H),2.01 - 1.99 (m, 2H), 1.97 - 1.89 (m, 4H), 1.44 - 1.31 (m, 4H). $^{13}C$ NMR (101 MHz, DMSO-$d_6$) δ 173.53, 154.85, 153.01, 152.89, 151.67, 142.26, 142.15, 124.63, 117.61, 115.77, 113.94, 106.02, 105.78, 75.36, 51.78, 47.73, 30.43, 30.36, 30.29, 24.77.

**Example 73 Synthesis of 4-(4-(((1r,4r)-4-(3-(3-fluoro-4-(trifluoromethoxy) phenyl)ureido)cyclohexyl)oxy)phenoxy)butanoic acid (SP-C24)**

SP-C23 (0.50 g, 0.95 mmol, 1 eq), THF (15 mL), $H_2O$ (5 mL), and LiOH (0.10 g, 4.18 mmol, 4.4 eq) were added into a 25 mL single-necked flask in sequence, and stirred at room temperature. After 1.5 h, the reaction was monitored by TLC (EA: PE=1 : 3), and stopped. The THF therein was removed by concentration under reduced pressure, and the remaining was extracted with EA (20 mL), the resulting aqueous layer was adjusted to pH=2 with 1N HCl, followed by filtration suction under reduced pressure to obtain 0.47 g of a pale yellow solid with a yield of 96.21%. $^1H$ NMR (400 MHz, DMSO-$d_6$) δ 12.11 (s, 1H), 9.19 (s, 1H), 7.68 (dd, *J* = 13.5, 2.6 Hz, 1H), 7.38 (td, *J* = 8.9, 1.2 Hz, 1H), 7.10 (ddd, *J* = 9.0, 2.6, 1.3 Hz, 1H), 6.91 - 6.79 (m, 4H), 6.57 (s, 1H), 4.19 (tt, *J* = 9.3, 3.8 Hz, 1H), 3.90 (t, *J* = 6.4 Hz, 2H), 3.54 (ddt, *J* = 11.1, 7.4, 3.9 Hz, 1H), 2.37 (t, *J* = 7.3 Hz, 2H), 2.05 - 1.97 (m, 2H), 1.96 - 1.85 (m, 4H), 1.50 - 1.21 (m, 5H). $^{13}C$ NMR (101 MHz, DMSO-$d_6$)

δ 174.62, 155.37, 154.80, 153.10, 152.92, 151.65, 142.11, 142.01, 124.70, 117.63, 115.81, 113.86, 105.95, 105.71, 75.25, 67.44, 47.62, 30.64, 30.27, 30.13, 24.82.

**Example 74 Synthesis of methyl 2-(5-((Z)-4-(((1r,4r)-4-(tert-butoxycarbonyl)amino)cyclohexyl)oxy)benzylidene)-2,4-dioxythiazolidin-3-yl)acetate**

Tert-butyl ((1r,4r)-4-(4-formylphenoxy)cyclohexyl)carbamate (4.00 g, 12.53 mmol, 1 eq), methyl 2-(2,4-dioxythiazolin-3-yl)acetate (2.37 g, 12.53 mmol, 1 eq), piperidine (0.50 g, 6.27 mmol, 0.5 eq), acetic acid (0.38 g, 6.27 mmol, 0.5 eq), and toluene (20 mL) were added sequentially into a 100 mL round-bottom flask, and heated to reflux. As the reaction proceeded, solids precipitated. After 8 h, the reaction was completed as monitored by TLC (EA: PE=1 : 1), and the reaction was stopped. The reaction solution was cooled to room temperature and subjected to filtration suction, and the resulting filter cake was rinsed with a small amount of *n*-hexane to obtain 3.89 g of a white solid with a yield of 63.36%, without product in the filtrate detected by TLC.

**Example 75 Synthesis of methyl 2-(5-((Z)-4-(((1r,4r)-4-aminocyclohexyl)oxy)benzylidene)-2,4-dioxythiazolidin-3-yl)acetate**

Methyl 2-(5-((Z)-4-(((1r,4r)-4-(tert-butoxycarbonyl)amino)cyclohexyl)oxy) benzylidene)-2,4-dioxythiazolidin-3-yl)acetate (3.89 g, 7.94 mmol, 1 eq) and DCM (8 mL) were added sequentially to a 100 mL single-necked flask, and cooled to 0°C in an ice bath, and TFA (2 mL) was then added dropwise. After 4 h, the reaction was completed as monitored by TLC (EA: PE=1 : 3), and the reaction was stopped. DCM and TFA in the reaction solution were removed by vacuum distillation to obtain 3.87 g of a pale yellow solid, and the crude product was directly used for the next step without purification. ESI MS: m/z 390.1 $[M + H]^+$.

**Example 76 Synthesis of methyl 2-(2,4-dioxo-5-((Z)-4-(((1r,4r)-4-(3-(4-(trifluoromethoxy)phenyl)ureido)cyclohexyl)oxy)benzylidene)thiazolidin-3-yl)acetate (SP-D01)**

BTC (0.03 g, 0.10 mmol, 0.34 eq) and dry DCM (5 mL) were added sequentially into a 25 mL single-necked flask, and cooled to -80°C in a cold trap, a solution (10 mL) of 4-trifluoromethoxyaniline (0.05 g, 0.29 mmol, 1 eq) and $Et_3N$ (0.13 g, 1.30 mmol, 5 eq) in DCM was added dropwise within 5 min, and a resulting mixture was naturally heated to room temperature. After 10 min, the reaction was completed as monitored by TLC (EA: PE=1 : 3), and the reaction was stopped. The reaction solution was evaporated to dryness, and DCM (10 mL) and $Et_3N$ (0.13 g, 1.30 mmol, 5 eq) were added thereto. A resulting mixture was cooled to 0°C in an ice bath, and a solution (5 mL) of methyl 2-(5-((Z)-4-(((1r,4r)-4-aminocyclohexyl)oxy)benzylidene)-2,4-dioxythiazolidin-3-yl)acetate (0.10 g, 0.26 mmol, 1 eq) in DCM was added dropwise. A resulting mixture was reacted at room temperature. After 30 min, the reaction was completed as monitored by TLC (EA: PE=1 : 1, AcOH 2d), and stopped. The DCM therein was removed by concentration under reduced pressure to obtain 0.23 g of a crude product as a white solid. The crude product was purified by silica gel column chromatography, with silica gel 1.5 times the amount of the sample for mixing with the sample and 5 times the amount of the sample for silica gel column loading, using an eluent of EA: PE=1 : 10, to obtain 0.10 g of a white solid with a yield of 66.67%. ESI MS: *m/z* 616.0 $[M + Na]^+$. $^1H$ NMR (400 MHz, DMSO-$d_6$) δ 9.74 (s, 1H), 7.96 (s, 1H), 7.65 - 7.57 (m, 2H), 7.54 - 7.45 (m, 2H), 7.22 (d, J = 8.6 Hz, 2H), 7.18 - 7.12 (m, 2H), 6.44 (d, *J* = 7.6 Hz, 1H), 4.51 (s, 2H), 3.72 (s, 3H), 3.57 - 3.53 (m, 1H), 3.28 - 3.25 (m, 1H), 2.12 - 2.03 (m, 2H), 1.98 - 1.90 (m, 2H), 1.58 - 1.32 (m, 6H).

**Example 77 Synthesis of 2-(2,4-dioxo-5-((Z)-4-(((1r,4r)-4-(3-(4-(trifluoromethoxy)phenyl)ureido)cyclohexyl)oxy) benzylidene)thiazolidin-3-yl)acetic acid**

SP-D01 (0.08 g, 0.13 mmol, 1 eq), THF (5 mL), $H_2O$ (1 mL), and LiOH (0.01 g, 0.40 mmol, 3 eq) were added into a 25 mL single-necked flask in sequence, and stirred at room temperature. After 1.5 h, the reaction was monitored by TLC (EA: PE=1 : 3), and stopped. The reaction mixture was concentrated under reduced pressure to remove THF, and adjusted to pH=4 with 1N HCl, and a resulting mixture was extracted by adding EA (10 mL) and $H_2O$ (10 mL) thereto. The organic layer was dried over anhydrous magnesium sulfate, followed by filtration suction, and concentrating to dryness under reduced pressure to obtain 70 mg of a white solid with a yield of 92.98%. ESI MS: *m/z* 578.3 $[M - H]^-$.

**Example 78 Synthesis of methyl 2-(5-((Z)-4-(((1R,4r)-4-(3-((1R,3R,5S,7R)-3,5-dimethyladamantan-1-yl)ureido) cyclohexyl)oxy)benzylidene)-2,4-dioxythiazolin-3-yl)acetate**

BTC (0.03 g, 0.10 mmol, 0.34 eq) and dry DCM (5 mL) were added sequentially into a 25 mL single-necked flask, and cooled to -80°C in a cold trap, a solution (10 mL) of memantine (0.05 g, 0.28 mmol, 1 eq) and $Et_3N$ (0.28 g, 2.80 mmol,

10 eq) in DCM was added dropwise within 5 min, and a resulting mixture was naturally heated to room temperature. After 10 min, the reaction was completed as monitored by TLC (EA: PE=1 : 3), and the reaction was stopped. The reaction solution was evaporated to dryness, DCM (10 mL) and $Et_3N$ (0.28 g, 2.80 mmol, 10 eq) were added thereto, and a resulting mixture was cooled to 0°C in an ice bath, and a solution (5 mL) of N4 (0.10 g, 0.28 mmol, 1 eq) in DCM was added dropwise. A resulting mixture was reacted at room temperature. After 30 min, the reaction was monitored by TLC (EA: PE=1 : 1, AcOH 2d) and stopped. The DCM therein was removed by concentration under reduced pressure to obtain 0.31 g of a crude product as a white solid. The crude product was purified by silica gel column chromatography, with silica gel 1.5 times the amount of the sample for mixing with the sample and 5 times the amount of the sample for silica gel column loading, using an eluent of EA: PE=1 : 10, to obtain 0.08 g of a white solid with a yield of 48.20 %. ESI MS: *m/z* 594.66 $[M - H]^-$. $^1H$ NMR (400 MHz, DMSO-$d_6$) δ 7.96 (s, 1H), 7.65 - 7.56 (m, 2H), 7.18 - 7.09 (m, 2H), 5.62 (d, *J* = 7.6 Hz, 1H), 5.43 (s, 1H), 4.51 (s, 2H), 4.45 (dd, *J* = 8.9, 4.7 Hz, 1H), 3.72 (s, 3H), 2.09 - 1.99 (m, 3H), 1.89 - 1.81 (m, 2H), 1.68 (d, *J* = 3.1 Hz, 2H), 1.50 (s, 4H), 1.48 - 1.37 (m, 2H), 1.33 - 1.14 (m, 7H), 1.08 (s, 2H), 0.80 (s, 6H).

**Example 79 Synthesis of (Z) 5-(4-nitrobenzylidene)thiazolidin-2,4-dione**

p-Nitrobenzaldehyde (5.00 g, 33.09 mmol, 1 eq), 2,4-thiazolidinedione (3.88 g, 33.09 mmol, 1 eq), piperidine (1.41 g, 16.55 mmol, 0.5 eq), acetic acid (1.00 g, 16.55 mmol, 0.5 eq), and toluene (40 mL) were added sequentially into a 100 mL round-bottom flask, and heated to reflux. As the reaction proceeded, solids precipitated. After 8 h, the reaction was completed as monitored by TLC (EA: PE=1 : 1, AcOH 1d), and the reaction was stopped. The reaction solution was cooled to room temperature and subjected to filtration suction. The resulting filter cake was rinsed with a small amount of n-hexane to obtain 5.06 g of a reddish-brown solid. The filtrate was evaporated to dryness and concentrated, and DCM (10 mL) and MeOH (1 mL) were added for slurrying to obtain 3.06 g of a yellow solid with a yield of 98.19%. $^1H$ NMR (400 MHz, DMSO-$d_6$): δ (ppm) $^1H$ NMR (400 MHz, DMSO-$d_6$) δ 12.69 (s, 1H), 8.31 (d, *J* = 8.5 Hz, 2H), 7.83 (d, *J* = 8.5 Hz, 2H), 7.81 (s, 1H). ESI MS: *m/z* 249.1 $[M - H]^-$.

**Example 80 Synthesis of (*Z*)-5-(4-aminobenzylidene)thiazolidin-2,4-dione**

(*Z*) 5-(4-Nitrobenzylidene)thiazolidin-2,4-dione (8.27 g, 33.09 mmol, 1 eq), Fe (11.12 g, 198.54 mmol, 6 eq), EtOH (250 mL), $H_2O$ (70 mL), and $NH_4Cl$ (17.70 g, 330.90 mmol, 10 eq) were added sequentially into a 500 mL three-necked flask, and heated to reflux. After 1.5 h, the reaction was completed as monitored by TLC (EA: PE=1 : 1, AcOH 1d), and the reaction was stopped. The reaction solution was cooled to room temperature and subjected to filtration suction. The resulting filter cake was rinsed with a small amount of DCM. The filtrate was evaporated and concentrated to remove EtOH and part of $H_2O$, and the remaining was extracted with DCM: MeOH=10 : 1 (60 mL×4). The extraction liquid was dried over anhydrous magnesium sulfate, followed by filtration suction. The filtrate was concentrated under reduced pressure to obtain 2.06 g of a red-brown solid with a yield of 72.38%. ESI MS: *m/z* 199.3 $[M - H]^-$.

**Example 81 Synthesis of 1-((1r,3R,5S,7r)-3,5-dimethyladamantan-1-yl)-3-(4-((E)-(2,4-dioxythiazolin-5-ylidene) methyl)phenyl)urea (SP-E01)**

BTC (0.05 g, 0.15 mmol, 0.34 eq) and dry DCM (5 mL) were added sequentially into a 25 mL single-necked flask, and cooled to -80°C in a cold trap, and a solution (10 mL) of memantine (0.08 g, 0.45 mmol, 1 eq) and $Et_3N$ (0.46 g, 4.50 mmol, 10 eq) in DCM was added dropwise within 5 min, and a resulting mixture was naturally heated to room temperature. After 30 min, the reaction was completed as monitored by TLC (EA: PE=1 : 3), and the reaction was stopped. The reaction solution was evaporated to dryness, DCM (10 mL) and $Et_3N$ (0.46 g, 4.50 mmol, 10 eq) were added thereto, and a resulting mixture was cooled to 0°C in an ice bath, and a solution (5 mL) of (Z)-5-(4-aminobenzylidene)thiazolidin-2,4-dione (0.10 g, 0.45 mmol, 1 eq) in DCM was added dropwise. A resulting mixture was reacted at room temperature. After 30 min, the reaction was monitored by TLC (EA: PE=1 : 1, AcOH 2d) and stopped. 1N HCl (30 mL) was added thereto, and a resulting mixture was extracted with DCM (30 mL×3). The resulting organic layers were combined, washed with water (30 mL), washed with saturated NaCl solution (30 mL), and dried over anhydrous magnesium sulfate, followed by filtration suction, and concentrating under reduced pressure to obtain 0.37 g of a crude product as a yellow oily substance. The crude product was purified by silica gel column chromatography, with silica gel 1.5 times the amount of the sample for mixing with the sample, and 7 times the amount of the sample for silica gel column loading, with an eluent of EA: PE=1 : 10, to obtain 0.11 g of a white solid with a yield of 57.49%. ESI MS: *m/z* 423.9 $[M - H]^-$.

**Example 82 Synthesis of (*E*)-1-(4-((2,4-dioxythiazolinyl-5-ylidene)methyl)phenyl)-3-(3-fluoro-4-(trifluoromethoxy)phenyl)urea (SP-E03)**

BTC (0.05 g, 0.15 mmol, 0.34 eq) and dry DCM (5 mL) were added sequentially into a 25 mL single-necked flask,

and cooled to -80°C in a cold trap, a solution (10 mL) of 4-trifluoromethoxyaniline (0.08 g, 0.45 mmol, 1 eq) and $Et_3N$ (0.46 g, 4.50 mmol, 10 eq) in DCM was added dropwise within 5 min, and a resulting mixture was naturally heated to room temperature. After 30 min, the reaction was completed as monitored by TLC (EA: PE=1 : 3), and the reaction was stopped. The reaction solution was evaporated to dryness, excess phosgene was removed, DCM (10 mL) and $Et_3N$ (0.46 g, 4.50 mmol, 10 eq) were added thereto, and a resulting mixture was cooled to 0°C in an ice bath, and a solution (5 mL) of (Z)-5-(4-aminobenzylidene)thiazolidin-2,4-dione (0.10 g, 0.45 mmol, 1 eq) in DCM was added dropwise. The mixture was reacted at room temperature for 15 min, and the reaction was stopped as monitored by TLC (EA: PE=1 : 1, AcOH 2d). The product was added with 1N HCl (30 mL), and a resulting mixture was extracted with DCM (30 mL×3), the organic layers were combined, washed with water (30 mL), washed with saturated NaCl solution (30 mL), dried with anhydrous magnesium sulfate, subjected to filtration suction, and concentrated under reduced pressure to obtain 0.31 g of crude yellow oily substance. The crude product was purified by silica gel column chromatography, with silica gel 1.5 times the amount of the sample for mixing with the sample, and 7 times the amount of the sample for silica gel column loading, with an eluent of EA: PE=1 : 10, to obtain 0.09 g of a white solid with a yield of 47.37 %. ESI MS: *m/z* 421.8 $[M - H]^-$.

**Test Example 1**

1. sEH inhibitory activity test

Detection principle: a specific substrate, (3-phenyl-oxy)-acetic acid cyano-(6-methoxy-naphthalen-2-yl) methyl ester, namely PHOME, is non-fluorescent but can be hydrolyzed under the action of sEH enzyme to generate a product 6-methoxy-2-naphthaldehyde. The 6-methoxy-2-naphthaldehyde can emit fluorescence with a wavelength of 465 nm when excited by 330 nm light, and an intensity of the detected fluorescence signal is inversely proportional to an inhibitory effect on the sEH enzyme. Based on the above principle, the inhibition rates of samples with different concentrations were calculated compared with the positive control group. The $IC_{50}$ value of the compound was calculated based on the inhibition rate and concentration using SPSS 20 software.

2. Preparation of reagents and drugs

25 mM Tris-HCl buffer (pH=7.4, containing 0.1 mg/mL BSA): 5 mg BAS was added to 12.5 mL of 1 M Tris-HCl buffer, and a resulting mixture was diluted with purified water, adjusted to pH value of 7.4 with hydrochloric acid, and diluted to 500 mL.
PHOME solution: 0.79 mg PHOME was dissolved in 106 μL DMSO to obtain a 20 mM PHOME solution, which was diluted to 1/3 mM with Tris-HCl buffer when used.
sEH solution: sEH (5 mg/mL) stock solution was stored in a -80°C refrigerator and diluted to 4 μg/mL with 25 mM Tris-HCl buffer when used.
A sample powder to be tested was dissolved in DMSO to a 20 mM solution, stored in a - 20°C refrigerator for later use, and diluted with Tris-HCl buffer to the corresponding concentration when used.

3. Experimental grouping

Experimental design: solvent group, 100% activity group (A), inhibitor group (B), positive control group (C), as shown in Table 1.

Table 1 Experimental Grouping

| Well | Buffer | DMSO | Inhibitor | sEH | Substrates |
|---|---|---|---|---|---|
| Solvent group | 168 μL | 2 μL | - | - | 30 μL |
| 100% activity group (A) | 148 μL | 2 μL | - | 20 μL | 30 μL |
| Inhibitor group (B) | 148 μL | - | 2 μL | 20 μL | 30 μL |
| Positive control group (C) | 148 μL | - | 2 μL | 20 μL | 30 μL |

4 Experimental steps

(a) 148 μL/well Tris-HCl buffer was added into a black-bottom 96-well microplate;

(b) 2 μL of the sample solution to be tested was added, the solvent group and 100% activity group were replaced with an equal volume of DMSO, and the lead compound GL-B401 was added in the positive control group, the structural formula of which was

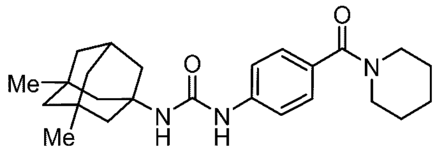

;

(c) a total of 5 concentrations were set in the inhibitor group, with final concentrations of 10 nM, 5 nM, 2.5 nM, 1.25 nM, and 0.625 nM, respectively;
(d) 20 μL of s-EH solution (final concentration 400 ng/mL) was added, except that an equal volume of Tris-HCl buffer was added instead in the solvent group;
(e) 30 μL of PHOME substrate was added to start the reaction (final concentration 50 μM) and incubated in a 37°C incubator for 10 min;
(f) fluorescence signal data were detected by microplate reader, with excitation wavelength of 330 nm and emission wavelength of 465 nm.

5. Data analysis

Three replicate wells were set for each sample, and the average of the three replicate wells was the fluorescence value (F) of the compound to be tested. The inhibition rate % = [(AF-BF)/AF]×100, where AF represents the fluorescence value of the 100% activity group and BF represents the fluorescence value of the inhibitor group. The $IC_{50}$ value of the compound was calculated based on the inhibition rate and concentration using SPSS 20 software.
The activity of the compounds was evaluated using a biochemical method with [cyano-(6-methoxynaphthalen-2-yl)methyl] 2-(3-phenyloxiran-2-yl)acetate (PHOME) as a substrate using recombinant human sEH (HsEH) and murine sEH (MsEH). The results are shown in Table 2.

Table 2 sEH $IC_{50}$ of HsEH and MsEH of compounds

| Number | HsEH $IC_{50}$ (nM) | MsEH $IC_{50}$(nM) | Number | HsEH $IC_{50}$ (nM) | MsEH $IC_{50}$(nM) |
|---|---|---|---|---|---|
| SP-A01 | 0.55 | 0.54 | SP-C01s | 0.3 | 0.6 |
| SP-A02 | 83.5 | 8.3 | SP-C01t | 0.6 | 1.4 |
| SP-B02 | 19.9 | 23.4 | SP-D01 | 1.1 | 2.4 |
| SP-B03 | 120.7 | 134.9 | SP-D04 | 1.2 | 10.1 |
| SP-B04 | 156.4 | 107.9 | SP-C02 | 0.6 | 2.2 |
| SP-A05 | 0.53 | 0.43 | SP-C03 | 0.4 | 1.4 |
| SP-A06 | 1.3 | 0.73 | SP-C04 | 1.3 | 2.2 |
| SP-B06 | 0.69 | 1.1 | SP-C05 | 1.9 | 12.6 |
| SP-A07 | 0.07 | 0.67 | SP-C06 | 1.3 | 2.4 |
| SP-B07 | 0.06 | 0.42 | SP-C08 | 0.3 | 1.6 |
| SP-C01 | 0.03 | 1.1 | SP-C09 | 0.4 | 0.9 |
| SP-C01b | 0.2 | 0.9 | SP-C10 | 1.0 | 1.3 |
| SP-C01c | 63.4 | 43.6 | SP-C 11 | 2.4 | 7.4 |
| SP-C01d | 0.5 | 3.1 | SP-C12 | 1.2 | 1.8 |
| SP-C01e | 0.3 | 0.7 | SP-C 14 | 0.4 | 0.6 |
| SP-C01f | 0.3 | 1.2 | SP-C15 | 0.7 | 6.3 |
| SP-C01g | 0.9 | 1.3 | SP-C 16 | 0.6 | 11.7 |
| SP-C01h | 0.5 | 2.1 | SP-C 17 | 1.9 | 1.6 |

(continued)

| Number | HsEH $IC_{50}$ (nM) | MsEH $IC_{50}$(nM) | Number | HsEH $IC_{50}$ (nM) | MsEH $IC_{50}$(nM) |
|---|---|---|---|---|---|
| SP-C01i | 0.3 | 0.7 | SP-C18 | 0.6 | 3.0 |
| SP-C01j | 2.1 | 1.6 | SP-C20 | 1.0 | 5.0 |
| SP-C01k | 0.3 | 1.2 | SP-C21 | 0.4 | 1.9 |
| SP-C01m | 0.9 | 6.7 | SP-C23 | 0.9 | 2.4 |
| SP-C01n | 0.7 | 1.3 | SP-C24 | 0.5 | 0.9 |

**Test Example 2**

1. PPARγ agonist activity test
Detection principle: when pM-hPPAR binds to the appropriate ligand, and is activated and binds to the GAL4DNA binding site in the plasmid pB4-RES-tk-luc, thereby initiating the expression of the downstream luciferase reporter gene. The amount of luciferase is detected to determine whether the compound to be tested is a PPAR agonist.
2. Test results were: SP-C01 for PPAR $\gamma EC_{50}$=4.2 μM, SP-A01 for PPAR $\gamma EC_{50}$=6.77 μM, SP-A07 for PPAR $\gamma EC_{50}$=7.75 μM, SP-B07 for PPAR $\gamma EC_{50}$=1.43 μM.

Table 3 PPAR γ of compounds in examples

| Number | PPARγ (compared with Rosiglitazone, %) | Number | PPARγ (compared with Rosiglitazone, %) |
|---|---|---|---|
| SP-A01 | 34.5 | SP-C01s | 0.3 |
| SP-A02 | 25.2 | SP-C01t | 0.6 |
| SP-B02 | 14.6 | SP-D01 | 1.1 |
| SP-B03 | 10.2 | SP-D04 | 1.2 |
| SP-B04 | 9.6 | SP-C02 | 11.9 |
| SP-B05 | 17.3 | SP-C03 | 10.2 |
| SP-A05 | 14.3 | SP-C04 | 10.0 |
| SP-A06 | 28.4 | SP-C05 | 8.9 |
| SP-B06 | 19.3 | SP-C06 | 9.9 |
| SP-A07 | 21.1 | SP-C08 | 12.3 |
| SP-B07 | 19.3 | SP-C09 | 8.1 |
| SP-C01 | 38.55 | SP-C 10 | 6.1 |
| SP-C01b | 32.3 | SP-C 11 | 7.2 |
| SP-C01c | 5.01 | SP-C 12 | 8.8 |
| SP-C01d | 10.2 | SP-C14 | 5.0 |
| SP-C01e | 19.16 | SP-C15 | 6.2 |
| SP-C01f | 5.88 | SP-C16 | 7.1 |
| SP-C01g | 7.20 | SP-C 17 | 8.0 |
| SP-C01h | 5.69 | SP-C18 | 12.9 |
| SP-C01i | 12.97 | SP-C20 | 14.6 |
| SP-C01j | 7.93 | SP-C21 | 10.2 |
| SP-C01k | 0.3 | SP-C23 | 9.6 |
| SP-C01m | 0.9 | SP-C24 | 17.3 |

(continued)

| Number | PPARγ (compared with Rosiglitazone, %) | Number | PPARγ (compared with Rosiglitazone, %) |
|---|---|---|---|
| SP-C01n | 0.7 | | |

**Test Example 3 Acute toxicity test**

3.1 Experimental animals

Healthy KM mice, half male and half female, SPF-grade, in a weight of 18-22 g, were raised in an environment at 24°C±2°C, a relative humidity of 60%±10%, and alternating 12 h light and 12 h dark, with free access to food and water.

3.2 Experimental reagents and drugs

Table 4 Experimental supplies

| Name | Manufacturer | Batch No. |
|---|---|---|
| **SP-B07** | - | / |
| Physiological saline | Nanjing Senbeijia Biotech Co., Ltd., China | PM20080903 |
| DMSO | Sigma | 20180923 |
| Tween 80 | Sinopharm Chemical Reagent Co., Ltd., China | 20121225 |

3.3 Experimental method

(1) Drug preparation

SP-B07 and SP-C01 were accurately weighed and dissolved in a mixed solvent of DMSO and Tween 80 (1 : 1). After they were completely dissolved, physiological saline in a volume 9 times the volume of the resulting solution was added thereto for dilution, to finally obtain a drug solution with a concentration of 1 mg/mL.

(2) Grouping, feeding, and drug administration

After KM mice were adaptively fed for one week, the male and female mice were randomly divided into 5 groups (n=8) according to body weight: normal control group (Vehicle), SP-B07 1 g/kg group, SP-C01 1 g/kg group, each group was gavaged with a volume of 10 mL/kg for 14 consecutive days; the other two groups of mice were administrated with SP-C01 1 g/kg and SP-C01 5 g/kg for a single dose and observed for 14 consecutive days.

(3) Physiological conditions and body weight changes of mice

The mice were observed for 30 min immediately after drug administration to monitor whether they had any adverse reactions or died; also, they were observed 1 time every 1 h, 2 h, 4 h, and 8 h to monitor whether they had any adverse reactions or died. Thereafter, the daily activities and mental states of the mice were monitored and recorded daily, and the body weight of each group of mice was weighed and recorded weekly.

(4) Detection of visceral index of mice

2 weeks after drug administration, the mice in each group were grossly dissected, and heart, liver, spleen, lung, and kidney of each mouse were removed, rinsed with physiological saline, and water thereon was removed by using medical gauze, followed by being stored in EP tubes for testing. The visceral index was calculated according to the equation as follows:

$$\text{Visceral index (\%)} = \text{organ weight/body weight} \times 10\%$$

3.4. Data statistics

The experimental data were expressed as Mean ± standard error (Mean ± SEM) and statistically analyzed using GraphPad Prism 8.4 software. Multiple groups were compared using one-factor ANOVA analysis, and variance homogeneity was analyzed using the LSD method, $P<0.05$ was considered a significant difference.

3.5 Experimental results

The influences of the compound on the visceral indexes of male and female mice are shown in FIG. 6 to FIG. 8. There was no significant difference in the visceral index of the mice in the drug-administrated group compared with the blank group. FIG. 6 shows the influence of compound SP-B07 on the body weight of KM mice. SP-B07 prepared in 0.5% CMC Na solution was intragastrically administered at a dose of 1.0 g/kg. The control group was administrated with 0.5% CMC Na. Panel (A) in FIG. 6 shows that SP-B07 and 0.5% CMC Na were continuously administered (o.p.) for 14 d, and body weight was monitored. Panel (B) in FIG. 6 shows the calculation of the visceral index 2 weeks after administration. FIG. 7 shows an influence of the compound SP-C01 on the body weight of KM mice. SP-C01 prepared in 0.5% CMC Na solution was intragastrically administered at doses of 1.0 g/kg and 5.0 g/kg, respectively. The control group was administrated with 0.5% CMC Na. Panel (A) in FIG. 7 shows that single administration (o.p.) of SP-C01 1.0 g/kg and 5.0 g/kg and 0.5% CMC Na, and body weight was monitored for 14 d. Panel (B) in FIG. 7 shows that SP-C01 1.0 g/kg and 0.5% CMC Na were continuously administered (o.p.) for 14 d, and body weight was monitored. FIG. 8 shows an influence of the compound SP-C01 on a visceral index of KM mice. SP-C01 prepared in 0.5% CMC Na solution was intragastrically administered at doses of 1.0 g/kg and 5.0 g/kg, respectively. The control group was administrated with 0.5% CMC Na. Panel (A) in FIG. 8 shows that single administration (o.p.) of SP-C01 1.0 g/kg and 5.0 g/kg and 0.5% CMC Na, and visceral index was calculated on the 14-th day. Panel (B) in FIG. 8 shows that SP-C01 1.0 g/kg and 0.5% CMC Na were continuously administered (o.p.) for 14 d, and visceral index was calculated on the 14-th day.

FIG. 9 shows representative H&E staining sections of the influence of the compound SP-C01 on the organs of KM mice. SP-C01 prepared in 0.5% CMC Na solution was orally administered at a dose of 1.0 g/kg for 14 consecutive days. The control group was administrated with 0.5% CMC Na. No significant abnormalities were found. FIG. 19 shows representative H&E staining sections of the influence of the compound SP-B07 on the organs of KM mice. SP-B07 prepared in 0.5% CMC Na solution was orally administered at a dose of 1.0 g/kg for 14 consecutive days. The control group was administrated with 0.5% CMC Na.

After 14 d, there was no significant difference between the mice in the drug-administrated group and the blank control group. Further pathological section examination of the heart, liver, spleen, lung, kidney, and stomach tissues showed no significant difference compared with the blank control group.

## Test Example 5 Stability of liver microsomes

The microsomal stability of compounds SP-B07 and SP-C01 was evaluated in liver microsomes of SD rats, and the tested compounds showed satisfactory stability in terms of *in vitro* microsomal stability. The half-life ($t_{1/2}$) of compound SP-B07 in rat liver microsomes was 5.50 h. The half-life ($t_{1/2}$) of compound SP-C01 in rat liver microsomes was 5.75 h. Preliminary results showed that the compounds SP-B07 and SP-C01 were metabolically stable and worth further development.

Table 5 Average concentration of SP-B07 in microsomal buffer at different time points (ng/mL)

| Time (min) | 0 | 10 | 60 | 120 | 240 | 360 | 720 | $t_{1/2}$ |
|---|---|---|---|---|---|---|---|---|
| Rat | 100 | 86.26 | 58.82 | 57.01 | 55.61 | 52.33 | 43.73 | 329.88 |

Table 6 Average concentration of SP-C01 in microsomal buffer at different time points (ng/mL)

| Time (min) | 0 | 10 | 30 | 45 | 60 | 100 | 120 | $t_{1/2}$ |
|---|---|---|---|---|---|---|---|---|
| Rat | 100 | 90.14 | 82.09 | 78.06 | 74.76 | 72.06 | 66.87 | 345.12 |

## Test Example 6 Plasma protein binding rate (%PPB)

The plasma protein binding rate (%PPB) of SD rats was measured by classical equilibrium dialysis apparatus. The compound SP-B07 showed a moderate %PPB (95.29%). The compound SP-C01 showed a moderate %PPB (83.9%). Since the therapeutic effect of a drug depends on the concentration of the free drug, a moderate concentration of

%PPB may be beneficial to the drug efficacy *in vivo* and thus produce analgesic and anti-inflammatory effects.

**Test Example 7 Pharmacokinetics (PK)**

To determine the metabolic stability of compounds SP-B07 and SP-C01 *in vivo,* PK characteristics were determined in 6-8 week old Sprague-Dawley (SD) rats, half male and half female, by oral (i.g.) doses of 50 mg/kg and intravenous (i.v.) doses of 10 mg/kg. The PK parameters are shown in Table 7. Blood levels were measured within 8 hours after oral administration. The maximum concentration reached after 0.5 h (1.14 μM); the area under the curve ($AUC_{0-8h}$) was 13.80 (μM·h); the plasma $t_{1/2}$ of SP-B07 was 1.63 h. A maximum concentration (3.29 μM) of compound SP-B07 was reached 2 min after intravenous injection; the area under the curve ($AUC_{0-8h}$) was 14.12 μM·h; and the plasma $t_{1/2}$ of SP-B07 was 1.91 h. SP-B07 had a moderate bioavailability of 19.54%, laying a desirable foundation for the next *in vivo* study. A maximum concentration (20.71 μM) of SP-C01 reached after 0.5 h; the area under the curve ($AUC_{0-8h}$) was 38.88 (μM·h); the plasma $t_{1/2}$ of SP-C01 was 1.63 h. A maximum concentration (5.71 μM) of compound SP-C01 was reached 2 min after intravenous injection; the area under the curve ($AUC_{0-8h}$) was 10.72 μM·h; and the plasma $t_{1/2}$ of SP-C01 was 1.91 h. SP-C01 had a relatively high bioavailability of 72.54% (Table 8), laying a desirable foundation for the next *in vivo* study.

Table 7 PK of SP-B07 after intravenous and oral administration in rats (n=3)

| Parameter | **SP-B07** (n = 3) | **SP-B07** (n = 3) |
|---|---|---|
| | *i.v.* (10 mg/kg) | *p.o.* (50 mg/kg) |
| $T_{max}$(h) | 0.083 | 0.50 |
| $C_{max}$(μM) | 3.29 | 1.14 |
| $t_{1/2}$(h) | 3.26 | 3.48 |
| CL(L/h/kg) | 0.79 | 3.82 |
| $V_Z$(L/kg) | 3.71 | 18.18 |
| $AUC_{(0-8)}$(μM×h) | 14.12 | 13.80 |
| $AUC_{(0-\infty)}$(μM×h) | 16.87 | 17.67 |
| F | | 19.54 |

Table 8 PK of SP-C01 after intravenous and oral administration in rats (n=3)

| Parameter | **SP-C01** (n = 3) | **SP-C01** (n = 3) |
|---|---|---|
| | *i.v.* (10mg/kg) | *p.o.* (50mg/kg) |
| $T_{max}$(h) | 0.033 | 0.25 |
| $C_{max}$(μM) | 5.71 | 20.71 |
| $t_{1/2}$(h) | 1.91 | 1.63 |
| CL(L/h/kg) | 0.35 | 0.94 |
| $V_Z$(L/kg) | 0.99 | 2.26 |
| $AUC_{(0-8)}$(μM×h) | 10.72 | 38.88 |
| $AUC_{(0-\infty)}$(μM×h) | 12.10 | 22.77 |
| F (%) | | 72.54 |

**Test Example 7 Complete Freund's adjuvant (CFA)-induced AIA (adjuvant-induced Arthritis) mouse model**

The CFA-induced AIA mouse model has similar characteristics to human rheumatoid arthritis (RA) in terms of pathogenesis, joint pain, bone destruction, and synovial hyperplasia, and has been widely used in the study of the pathogenesis and treatment mechanisms of human RA. In order to determine whether compound SP-B07 was effective against AIA in female KM mice (20-22 g), five groups were designed for intraperitoneal injection for comparison, namely, model group (CFA), SP-B07 group (SP-B07-10 mg/kg), SP-B07 group (SP-B07-20 mg/kg), and celecoxib group (celecoxib-10 mg/kg). The pain threshold of AIA mice was tested by the hot plate method. The percentage increase in pain threshold was measured 10 min, 45 min, 120 min, and 240 min after drug treatment (panel (A) in FIG. 10). A curve of

the increase in pain threshold over time was plotted (panel (B) in FIG. 10), and the AUC value of the percentage increase in pain threshold was obtained (panel (C) in FIG. 10). Compound SP-C01 began to take effect 10 min after administration and the pain threshold began to raise, which remained at a high level 4 h after administration, while the effect of celecoxib began to decrease 4 h after administration. From the curve of the percentage increase in pain threshold, it can be seen that the analgesic effect of compound SP-B07 was better than that of celecoxib and showed a clear dose-effect relationship. Finally, the thickness of the paws of the AIA mouse model at different time points after compound injection was evaluated and compared with the thickness of the mouse paws before modeling, and the swelling reduction rate of the mouse paws was calculated. As shown in panels (D) to (F) in FIG. 10, compound SP-B07 has a significant detumescence rate on mouse paws and is superior to celecoxib. In conclusion, compound SP-B07 has significant analgesic and anti-inflammatory effects (reducing the swelling of mouse paws) on CFA-induced AIA and is superior to celecoxib.

FIG. 10 shows the evaluation of compound SP-B07 in the treatment of CFA-induced arthritis in the mouse model (AIA); 24 h after AIA induction by CFA (25 μL/25g, i.p.), panel (A) in FIG. 10 shows the percentage increase in pain threshold of the CFA group, CFA+SP-B07 group, and CFA+celecoxib group; panel (B) in FIG. 10 shows a schematic diagram of the change trend of the percentage of pain threshold over time within 240 min in the CFA group, CFA+SP-B07 group, and CFA+celecoxib group; panel (C) in FIG. 10 shows the area under curve (AUC) of the percentage increase in pain threshold of the CFA group, CFA+SP-B07 group, and CFA+celecoxib group; panel (D) in FIG. 10 shows the swelling reduction rate of the CFA group, CFA+SP-B07 group, and CFA+celecoxib group; panel (E) in FIG. 10 shows the schematic diagram of the change trend of the percentage of swelling reduction over time within 12 h in the CFA group, CFA+SP-B07 group, and CFA+celecoxib group; panel (F) in FIG. 10 shows the AUC of the swelling reduction rate within 12 h in the CFA group, CFA+SP-B07 group, and CFA+celecoxib group; significance: compared with the Mod group, ****P*<0.0001, ****P*<0.001, ***P*<0.01, and **P*<0.05.

In order to determine whether compound SP-C01 was effective against AIA in female KM mice (20-22 g), five groups were designed for intraperitoneal injection for comparison, namely, model group (CFA), SP-C01 group (SP-C01-5 mg/kg), SP-C01 group (SP-C01-10 mg/kg), SP-C01 group (SP-C01-20 mg/kg), and celecoxib group (celecoxib-10 mg/kg). The pain threshold of AIA mice was tested by the hot plate method. The percentage increase in pain threshold was measured 10 min, 45 min, 120 min, and 240 min after drug treatment (panel (A) in FIG. 11). A curve of the increase in pain threshold over time was plotted (panel (B) in FIG. 11), and the AUC value of the percentage increase in pain threshold was obtained (panel (C) in FIG. 11). Compound SP-C01 began to take effect 10 min after administration and the pain threshold began to raise, which remained at a high level 4 h after administration, while celecoxib increased the pain threshold within 2 h, and its effect began to decrease 4 h after administration. From the curve of the percentage increase in pain threshold, it can be seen that the analgesic effect of compound SP-C01-5mg/kg is better than that of celecoxib-10 mg/kg after 60 min, and the effect of SP-C01-10mg/kg was better than that of celecoxib-10 mg/kg, showing a clear dose-effect relationship. Finally, the thickness of the paws of the AIA mouse model at different time points after compound injection was evaluated and compared with the thickness of the mouse paws before modeling, and the swelling reduction rate of the mouse paws was calculated. As shown in panels (D) to (F) in FIG. 11, the compound SP-C01 has a significant detumescence rate on mouse paws and was superior to celecoxib. In conclusion, compound SP-C01 has significant analgesic and anti-inflammatory effects (reducing the swelling of mouse paws) on CFA-induced AIA and is significantly superior to celecoxib-10 mg/kg.

FIG. 11 shows the evaluation of compound SP-C01 in the treatment of CFA-induced arthritis in the mouse model (AIA); SP-C01 (5 mg/kg, i.p., i.p.) was used 24 h after CFA induction by AAI (25 μL/25g, i.p.), panel (A) in FIG. 11 shows the percentage increase in pain threshold of the CFA group, CFA+SP-C01 group, and CFA+celecoxib group; panel (B) in FIG. 11 shows a schematic diagram of the change trend of the percentage of pain threshold over time within 240 min in the CFA group, CFA+SP-C01 group, and CFA+celecoxib group; panel (C) in FIG. 11 shows the AUC of the percentage increase in pain threshold of the CFA group, CFA+SP-C01 group, and CFA+celecoxib group; panel (D) in FIG. 11 shows the swelling reduction rate of the CFA group, CFA+SP-C01 group, and CFA+celecoxib group; panel (E) in FIG. 11 shows the schematic diagram of the change trend of the percentage of swelling reduction over time within 12 h in the CFA group, CFA+SP-C01 group, and CFA+celecoxib group; panel (F) in FIG. 11 shows the AUC of the swelling reduction rate within 12 h in the CFA group, CFA+SP-C01 group, and CFA+celecoxib group; significance: compared with the Mod group, ****P<0.0001, ****P*<0.001, ***P*<0.01, and **P*<0.05; compared with the celecoxib group, ##*P*<0.01, #*P*<0.05.

Furthermore, 6 groups were designed for oral gavage comparison, namely model group (CFA), SP-C01 low-dose group (SP-C01-30 mg/kg), SP-C01 high-dose group (SP-C01-60 mg/kg), SP-C01h low-dose group (SP-C01h-30 mg/kg), SP-C01h high-dose group (SP-C01h-60 mg/kg), and celecoxib group (celecoxib-30 mg/kg). Female C57BL/6 (20-22 g) mice were injected with CFA (25 μL/25 g) into plantar area of the right hind paw. The pain threshold of AIA in mice was detected by the hot plate method 24 h after modeling. The percentage increase of pain threshold at 10 min, 30 min, 45 min, 60 min, 120 min and 240 min after drug treatment was shown in panel (A) in FIG. 12. The curve of pain threshold increase over time was plotted (panel (B) in FIG. 12), and the AUC value of pain threshold increase was obtained (panel (C) in FIG. 12). Compound SP-C01 began to take effect 10 min after administration and the pain threshold began to raise, which remained at a high level at 4 h, while celecoxib increased the pain threshold 30 min after administration, which was

slightly later than SP-C01, and the effect began to decrease at 4 h. From the curve of the percentage increase in pain threshold, it can be seen that the analgesic effect of the compound SP-C01-30 mg/kg is better than that of celecoxib-30 mg/kg. Finally, the thickness of the paws of the AIA mouse model at different time points after compound injection was evaluated and compared with the thickness of the mouse paws before modeling, and the swelling reduction rate of the mouse paws was calculated. As shown in panels (D) to (F) in FIG. 12, the compound SP-C01 had a significant detumescence rate on mouse paws and was significantly superior to celecoxib. In conclusion, compound SP-C01 had a significant analgesic and anti-inflammatory effect (reducing the swelling of mouse paws) on the CFA-induced AIA regardless of intraperitoneal injection or oral gavage and was significantly superior to celecoxib.

FIG. 12 shows the evaluation of compound SP-C01 in the treatment of CFA-induced arthritis in the mouse model (AIA). SP-C01 (30 mg/kg, i.g.), SP-C01, SP-C01h (30 mg/kg, i.g.), and celecoxib (30 mg/kg) were administered to C57BL/6 mice 24 h after CFA-induced AAI (25 μL/25 g, i.p.). Panel (A) in FIG. 12 shows the percentage increase of pain threshold in the CFA group, CFA+SP-C01 group, CFA+SP-C01h group, and CFA+celecoxib group; panel (B) in FIG. 12 shows the trend of the percentage increase of pain threshold over time within 240 min in the CFA group, CFA+SP-C01 group, CFA+SP-C01h group, and CFA+celecoxib group; panel (C) in FIG. 12 shows the AUC of the percentage increase in pain threshold of the CFA group, CFA+SP-C01 group, CFA+SP-C01h group, and CFA+celecoxib group; panel (D) in FIG. 12 shows the swelling reduction rate of the CFA group, CFA+SP-C01 group, CFA+SP-C01h group, and CFA+celecoxib group; panel (E) in FIG. 12 shows the trend of the percentage of swelling reduction over time within 12 h in the CFA group, CFA+SP-C01 group, CFA+SP-C01h group, and CFA+celecoxib group; panel (F) in FIG. 12 shows the AUC of swelling reduction rate within 12 h in the CFA group, CFA+SP-C01 group, CFA+SP-C01h group, and CFA+celecoxib group; significance: compared with the Mod group, ***$P$<0.0001, ***$P$<0.001, **$P$<0.01, and *$P$<0.05; compared with the celecoxib group, ##$P$<0.01, #$P$<0.05; compared with Mod, $$P$<0.001, $$P$<0.01, and $P$<0.05.

**Test Example 8 Pharmacodynamic evaluation of LPS-induced sepsis model**

Male C57BL/6 mice (17-20 g) were intraperitoneally injected with LPS (25 mg/kg) to induce endotoxemia (n=7). The models included a modeling group, a dexamethasone administration group 4 h after modeling, an SP-B07 administration group 4 h after modeling, an SP-B07 administration group immediately after modeling, and a lead compound A20 administration group immediately after modeling. The drug was administered twice, with an interval of 8 h between administrations. As shown in FIG. 13 to FIG. 15, the SP-B07 administration group 4 h after modeling significantly prolonged the survival time of septic mice and increased the survival rate by 42.8%. A similar effect was achieved with dexamethasone (57.1%), suggesting that the compound might have a potent anti-inflammatory effect.

FIG. 13 shows an increased survival rate of C57Bl/6 mice in LPS-induced inflammation post treatment with the compound SP-B07. Mice treated with 25 mg/kg LPS by intraperitoneal injection (i.p.) (blue, n=7) show a survival advantage compared with mice in the control group. FIG. 14 shows expression levels of COX-2, sEH, NOS2, and VCAM in mouse plasma detected by Western blot. FIG. 15 shows IL-6, MCP-5, and TNF-α inflammatory factors in mouse plasma detected by Elisa. Significance: **** $P$<0.0001 compared with the LPS group.

**Test Example 9 Sciatic nerve chronic constriction injury (CCI)-induced neuropathic pain**

The effect of SP-C01 was further investigated in the sciatic nerve CCI-induced neuropathic pain model of SD rats. Rats were randomly divided into oral administration of (±)-EC-5026 (30 mg/kg/d) and SP-C01 groups, the SP-C01 group further included 30 mg/kg/d and 100 mg/kg/d groups, and an additional group of SP-C01 30 mg/kg/d intraperitoneal injection was added. Pain relief condition in rats was assessed on days 1, 3, and 7 throughout the study by von Frey test after oral administration of (±)-EC-5026 (30 mg/kg/d) and SP-C01 (30 mg/kg/d) at 12 h dosing intervals (BID), as indicated by an increase in mechanical withdrawal threshold (PWT). The study showed that SP-C01 had no obvious tolerance and had a certain analgesic effect (Panels A to B of FIG. 4). After oral administration of SP-C01 (30 mg/kg/d), SP-C01 (100 mg/kg/d), and intraperitoneal injection of SP-C01 (30 mg/kg/d) at an administration interval of 12 h (BID), the mechanical PWT in the first 7 hours on the first day throughout the study was evaluated by the von Frey test. The anti-neuropathic pain effect increased in a dose-dependent manner, and the oral effect was roughly equivalent to that of intraperitoneal injection (FIG. 16). The experimental results show that the overall analgesic effect of SP-C01 has a cumulative effect over time, and the therapeutic effect is significantly better than that of the positive control drug EC5026 starting from the 7th day.

FIG. 16 shows that SP-C01 had blocked pain measured by the von Frey test (mechanical withdrawal threshold) in a chronic constriction injury model of neuropathy in male SD rats. Data were expressed as Mean ± standard error of the mean. SP-C01 formulated in 10% DMSO and 90% corn oil was orally administered at the indicated doses at an administration interval of 12 h (BID). (n=8 per group); panel (A) in FIG. 16 corresponds to oral administration of (±)-EC-5026 (30 mg/kg/d) and SP-C01 (30 mg/kg/d) to evaluate pain relief in the first 7 h on the first day. Panel (B) in FIG. 16 corresponds to oral administration of (±)-EC-5026 (30 mg/kg/d) and SP-C01 (30 mg/kg/d) throughout the study to evaluate the pain relief on day 1, 3, and 7. Panel (C) in FIG. 16 corresponds to that oral administration of SP-C01 (30

mg/kg/d) and SP-C01 (100 mg/kg/d) as well as intraperitoneal injection of SP-C01 (30 mg/kg/d), to evaluate the SP-C01 dose-dependent pain relief as well as the effects of SP-C01 by different administration methods, characterized by the increase in mechanical paw withdrawal threshold (PWT). Panel (D) in FIG. 16 shows a summary of the AUC values of all experimental results. Significance: ***$P$<0.01, **P<0.01 and *P<0.05, compared with the Mod group; ##$P$<0.01 and #$P$<0.05, compared with the Mod group.

**Test Example 10 L-arginine-induced acute pancreatitis model**

Acute pancreatitis is a potentially life-threatening gastrointestinal disease whose incidence has been increasing over the past few decades. There is no good therapeutic drug at present, and the research on drugs for the treatment of acute pancreatitis is highly urgent. Given that sEH was associated with the pathogenesis of acute pancreatitis, the sEH inhibitor SP-C01 was evaluated in male C57BL/6 mice (20-22 g) at doses of 5 mg/kg and 10 mg/kg for the treatment of L-arginine-induced acute pancreatitis model and compared with celecoxib (5 mg/kg) and ulinastatin (5 mg/kg). Histological analysis of the pancreas was conducted to determine whether SP-C01 treatment reduced the severity of L-arginine-induced pancreatitis. Pathological changes were studied on H&E stained pancreatic sections (FIG. 17).

FIG. 17 shows results of histological analysis on pancreas of mice treated with control, Mod, celecoxib, ulinastatin, and the compound SP-C01. Representative H&E staining sections of the pancreas were shown in the *in vivo* efficacy study. FIG. 18 shows results of histological analysis on pancreas of mice treated with control, Mod, celecoxib, ulinastatin, and the compound SP-C01. Specifically, panel (A) represents edema, panel (B) represents inflammatory cells (mononuclear and polymorphonuclear cells), panel (C) represents parenchymal atrophy, and panel (D) represents the total score (edema, mononuclear cell, polymorphonuclear cell, and parenchymal atrophy) (n=5 for each group) *$p$<0.05, **$p$<0.01 vs Mod.

As expected, the L-arginine model group (5.75±1.64) shows pancreatic damage representative of AP, including edema (panel (A) in FIG. 18), inflammatory cell infiltration (panel (B) in FIG. 18), and parenchymal atrophy (panel (C) in FIG. 18). In contrast, both SP-C01 5 mg/kg (2.40±1.20) and SP-C01 10 mg/kg (1.60±0.49) ameliorate L-arginine-induced AP pancreatic injury. The dose of compound SP-C01 10 mg/kg was superior to that of 5 mg/kg, and compound SP-C01 5 mg/kg (2.40±1.20) was more effective than celecoxib 5 mg/kg (3.50±1.80) in reversing pancreatic injury, edema, and neutrophil infiltration. Compound SP-C01 10 mg/kg (1.60±0.49) was more effective than ulinastatin 5 mg/kg (2.00±0.71) in reversing pancreatic injury and edema.

**Test Example 11 CD60 feed + streptozotocin-induced diabetes model**

Diabetes is a chronic metabolic disease characterized by elevated blood glucose, and its incidence has been increasing over the past few decades. There is no desirable therapeutic drug at present, and the research on drugs for the treatment of diabetes and its related complications such as diabetic pain is highly urgent. Given that sEH and PPARs are associated with the pathogenesis of diabetes and related complications, male C57BL/6 mice (20-22 g) were used to evaluate the treatment of CD60 diet + streptozotocin-induced diabetes and diabetic nephropathy by sEH inhibitors SP-C01 and SP-B07 at doses of 30 mg/kg and 90 mg/kg, and compare with pioglitazone (3 mg/kg). Blood glucose was analyzed to determine whether SP-C01 and SP-B07 treatment reduced the severity of diabetes and related complications (FIG. 20 to FIG. 21).

FIG. 20 shows results of blood glucose analysis of mice treated with control, Mod, pioglitazone, the compound SP-B07, and the compound SP-C01. FIG. 21 shows the treatment results of diabetic neuropathic pain measured by the hot plate method (panels A to B of FIG. 21) and Von-Frey mechanical stimulation (panel C of FIG. 21), respectively. Compared with the Control group, ****$P$<0.0001, ***$P$<0.001, **$P$<0.01 and *$P$<0.05.

As expected, the model group showed diabetic complications such as hair loss, increased periocular secretions, and limited movement, while the drug-administrated group showed no obvious behavioral abnormalities compared with healthy mice. This indicated that both SP-C01 and SP-B07 were effective in lowering blood glucose and improving diabetic neuropathic pain, which was not achieved by the PPARγ single-target agonist pioglitazone.

**Test Example 12 Effect of oral administration of SP-C01 on CFA-induced mouse rheumatoid arthritis model and rat chronic granuloma**

72 female ICR mice that passed the quarantine, weighing 19-26 g, were randomly divided into normal control group, model control group, celecoxib group, SP-C01 low-dose group, SP-C01 medium-dose group, and SP-C01 high-dose group according to their body weight, with 10 animals in each group. Except for the normal group, the animals in the other groups were injected with 25 μL/mouse of CFA at the bottom of right feet. Different drugs were prepared with pure water to the corresponding concentrations before administration. 30 min after modeling, different test substances were administrated to the animals in each group by oral gavage at 20 mL/kg, 1 time on the same day. The normal control group

and the model control group were given the same volume of pure water. The foot pain threshold of each group was tested with a hot plate instrument before the first administration (pain threshold screening before modeling) and 30 min, 60 min, 120 min, and 240 min after the first administration, and the pain threshold increase rate (%) was calculated, pain threshold increase rate (%) = (pain threshold after administration - pain threshold before administration) / pain threshold before administration × 100%, and the right foot thickness was measured 1, 2, 4, 6, 8, and 12 h after administration. On the 20th day after modeling, all model animals were regrouped according to the joint index score and administrated with 20 mL/kg by oral gavage 1 time daily for consecutive 7 days. The joint index score of each group was determined on the 3rd and 7th days after administration, and the pain threshold of the animals was tested with a hot plate instrument after the last administration.

**Table 9 Groups and dose designs**

| Group | Dose (mg/kg) | Volume (mL/kg) | Concentration (mg/mL) | Number of animals (mice) |
|---|---|---|---|---|
| Normal control group | | 20 | | 10 |
| Model control group | | 20 | | 10 |
| Celecoxib group | 30 | 20 | 1.5 | 10 |
| SP-C01 low-dose group | 15 | 20 | 0.75 | 10 |
| SP-C01 medium-dose group | 30 | 20 | 1.5 | 10 |
| SP-C01 high-dose group | 60 | 20 | 3 | 10 |

Influence of SP-C01 on chronic granuloma in rats

50 SD rats that passed quarantine were selected, half male and half female, weighing 210-260 g, were randomly divided into model control group, celecoxib group, SP-C01 low-dose group, SP-C01 medium-dose group, and SP-C01 high-dose group according to body weight, with 10 animals in each group. After the animals were anesthetized by isoflurane inhalation, cotton balls of about 50 mg were buried in the groin of the rats on both sides. The surgical procedure was strictly conducted aseptically. Penicillin was injected intraperitoneally for anti-inflammatory treatment after surgery. Before administration, different drugs were prepared into a concentration of 0.5 mg/mL with pure water in each group, and different test substances were administered by oral gavage at 10 mL/kg, 1 time a day for consecutive 7 days, while the model control group was administrated with an equal volume of pure water. After the last administration, the mice were anesthetized by isoflurane inhalation and then bled, and the cotton ball granuloma was removed and weighed to calculate the degree of swelling (wet weight of cotton ball granuloma - dry weight of cotton ball).

**Table 10 Groups and dose designs**

| Group | Dose (mg/kg) | Volume (mL/kg) | Concentration (mg/mL) | Number of animals (mice) |
|---|---|---|---|---|
| Model control group | | 10 | | 10 |
| Celecoxib group | 20 | 10 | 2 | 10 |
| SP-C01 low-dose group | 10 | 10 | 1 | 10 |
| SP-C01 medium-dose group | 20 | 10 | 2 | 10 |
| SP-C01 high-dose group | 40 | 10 | 4 | 10 |

Data processing and statistical analysis

The significant figures of the test data were rounded off and statistical analysis was conducted in accordance with SOP. The software used for statistics was SPSS22.0. Measurement data were expressed as Mean ± standard deviation ($\bar{x} \pm s$), and Leven's test was used to test normality and homogeneity of variance. If there was no statistical significance (P>0.05), statistical analysis was done using one-factor ANOVA. If ANOVA was statistically significant ($P \leq 0.05$), LSD test (parametric method) was used for comparative analysis. If variance was unequal ($P \leq 0.05$), the Kruskal-Wallis test was used. If the Kruskal-Wallis test was statistically significant ($P \leq 0.05$), Dunnett's Test (nonparametric method) was used for comparative analysis. The statistical results were tested with a boundary of $\alpha$=0.05, where $P \leq 0.05$ indicated statistical

significance, and $P \leq 0.01$ indicated that the difference was highly significant.

Experimental results

Influence of SP-C01 on CFA-induced rheumatoid arthritis model in mice
Influence of SP-C01 on acute paw swelling in mice

As shown in Table 11, the acute swelling of the mouse foot was significantly increased ($P \leq 0.01$) in the model control group at 1 h, 2 h, 4 h, 6 h, 8 h, 12 h, 24 h, and 7 d after administration compared with the normal group. Compared with the model control group, the swelling of the mouse foot in the SP-C01 low-dose group was significantly reduced ($P \leq 0.01$) at 4 h and 6 h after administration. The swelling of the mouse foot in the SP-C01 medium-dose group was significantly reduced ($P \leq 0.01$) at 2 h, 4 h, and 7 d after administration, and at 6 h and 24 h after administration ($P \leq 0.05$). The swelling of the mouse foot in the SP-C01 high-dose group was significantly reduced ($P \leq 0.01$) at 1 h, 2 h, 4 h, 6 h, 8 h, 12 h, 24 h, and 7 d after administration.

Influence of SP-C01 on the acute pain threshold increase rate of mice

As shown in Table 12, the acute pain threshold increase rate of mice was significantly reduced in the model control group at 0.5 h, 1 h, 2 h, and 4 h after administration compared with the normal group ($P \leq 0.01$). Compared with the model control group, the pain threshold increase rate of mice in the SP-C01 low-dose group increased significantly at 2 h and 4 h after administration ($P \leq 0.01$), and the pain threshold increase rate of mice increased significantly at 1 h after administration ($P \leq 0.05$). The pain threshold increase rate of mice in the SP-C01 medium-dose group increased significantly at 1 h, 2 h, and 4 h after administration ($P \leq 0.01$), and the pain threshold increase rate of mice increased significantly at 0.5 h after administration ($P \leq 0.05$). The pain threshold increase rate of mice in the SP-C01 high-dose group increased significantly at 0.5 h, 1 h, 2 h, and 4 h after administration ($P \leq 0.01$).

Influence of SP-C01 on pain threshold of mice

As shown in Table 13, the pain threshold of mice with acute inflammation was significantly reduced in the model control group at 0.5 h, 1 h, 2 h, and 4 h after administration compared with the normal group ($P \leq 0.01$); compared with the model control group, the pain threshold of mice in the SP-C01 low-dose group was significantly increased at 0.5 h, 1 h, 2 h, and 4 h after administration ($P \leq 0.01$); the pain threshold of mice in the SP-C01 medium-dose group was significantly increased at 0.5 h, 1 h, 2 h, and 4 h after administration ($P \leq 0.01$); the pain threshold of mice in the SP-C01 high-dose group was significantly increased at 0.5 h, 1 h, 2 h, and 4 h after administration ($P \leq 0.01$). The pain threshold of mice with chronic inflammation was significantly lower in the model control group compared with the normal group ($P \leq 0.01$), and the pain threshold of mice in the SP-C01 high-dose group was significantly higher than that in the model control group ($P \leq 0.05$).

**Table 11 influence of SP-C01 on acute paw swelling in mice (n=10)**

| Group | Acute paw swelling in mice | | | | | | | |
|---|---|---|---|---|---|---|---|---|
| | 1 h after admini stration | 2 h after admini stration | 4 h after adminis tration | 6 h after administ ration | 8 h after administ ration | 12 h after administ ration | 24 h after administ ration | 7 d after administ ration |
| Normal control group | 0.6±2.0 | 0.5±3.8 | 1.5±3.3 | 1.3±4.2 | -0.7±2.8 | 1.5±5.1 | 1.4±4.5 | 0.6±3.3 |
| Model control group | 29.6±8. 8++ | 32.0±5. 4++ | 37.8±6. 2++ | 62.6±25. 4++ | 66.4±21. 9++ | 71.5±29. 4++ | 63.1±23. 4++ | 61.3±15. 9++ |
| Celecoxi b group | 27.9±8. 7 | 28.4±4. 6 | 27.2±8. 0** | 37.2±26. 8** | 57.3±16. 6 | 61.0±18. 0 | 52.9±14. 1 | 48.3±13. 9 |
| PC-01 | 24.1±8. | 28.7±5. | 22.0±6. | 35.4±18. | 49.5±25. | 67.0±30. | 59.4±20. | 49.9±21. |
| low-dose group | 6 | 6 | 5** | 4** | 4 | 3 | 9 | 5 |
| PC-01 medium-dose group | 26.5±7. 6 | 23.6±3. 9** | 21.9±7. 6** | 41.4±25. 7* | 53.5±26. 3 | 61.4±27. 3 | 47.2±14. 6* | 38.5±17. 9** |
| PC-01 high-dose group | 20.4±6. 9** | 21.6±6. 2** | 19.6±9. 0** | 32.3±16. 1** | 39.5±24. 9** | 41.6±28. 5** | 35.7±12. 8** | 29.7±12. 7** |
| NOTE: compared with the normal control group, ++$P \leq 0.01$; compared with the model control group, **$P \leq 0.01$, *$P \leq 0.05$. | | | | | | | | |

**Table 12 Influence of SP-C01 on the acute pain threshold increase rate of mice (n=10)**

| Group | Acute pain threshold increase rate of mice | | | |
|---|---|---|---|---|
| | 0.5 h after administration | 1 h after administration | 2 h after administration | 4 h after administration |
| Normal control group | 4.3±18.9 | 0.5±13.8 | 5.0±12.6 | 1.3±13.2 |
| Model control group | -41.6+31.4+ | -127.3±186.8++ | -64.9±77.6++ | -92.0±114.0++ |
| Celecoxib group | -35.2±29.8 | -52.3±55.5 | 34.52±54.1** | -11.6±32.6** |
| PC-01 low-dose group | -20.1±32.1 | -25.6±60.3* | -6.9±33.7** | 10.3±36.6** |
| PC-01 medium-dose group | 5.8±63.8* | -1.5±64.4** | 10.6±56.8** | 16.0±33.3** |
| PC-01 high-dose group | 38.0±51.0** | 11.8±15.5** | 19.4±26.3** | 37.4±20.9** |
| NOTE: compared with the normal control group, ++*P*≤0.01; compared with the model control group, ***P*≤0.01, **P*≤0.05. | | | | |

**Table 13 Influence of SP-C01 on pain threshold of mice (n=10)**

| Group | Pain threshold in acute inflammatory state | | | | | Pain threshold in chronic inflammatory state |
|---|---|---|---|---|---|---|
| | Before administration | 0.5 h after administration | 1 h after administration | 2 h after administration | 4 h after administration | End of administration |
| Normal control group | 20.8±6.1 | 21.1±4.9 | 20.7±5.0 | 21.6±5.6 | 21.1±5.8 | 20.0±2.8 |
| Model control group | 20.4±7.2 | 10.1±3.4++ | 12.3±3.0++ | 12.7±4.2++ | 11.0±3.9++ | 10.6±1.3++ |
| Celecox ib group | 20.7±4.2 | 13.2±6.3 | 14.7±5.6 | 23.9±5.3** | 18.7±6.0** | 13.7±4.6* |
| PC-01 low-dose group | 20.8±5.8 | 15.5±4.5** | 18.6±7.6** | 19.9±5.9** | 22.4±6.1** | 10.5±2.4 |
| PC-01 medium-dose group | 20.5±8.3 | 17.4±2.2** | 20.5±3.5** | 23.3±4.9** | 24.6±3.1** | 12.5±4.5 |
| PC-01 high-dose group | 21.0±5.5 | 26.9±2.9** | 24.1±3.1** | 25.4±3.3** | 30.5±3.5** | 14.0±3.4* |
| NOTE: compared with the normal control group, ++*P*≤0.01; compared with the model control group, ***P*≤0.01, **P*≤0.05. | | | | | | |

Influence of SP-C01 on joint index score of mice in chronic inflammatory state

As shown in Table 14, the joint index scores of mice in chronic inflammatory state in the SP-C01 high-dose group are significantly reduced compared with the model control group on the 3rd day after administration (*P*≤0.05), and the joint index scores of mice in chronic inflammatory state in the SP-C01 low-dose group, SP-C01 medium-dose group, and SP-C01 high-dose group are significantly reduced on the 7th day after administration (*P*≤0.01).

**Table 14 Influence of SP-C01 on joint index score of mice (n=10) in chronic inflammatory state**

| Group | Joint index score | | |
|---|---|---|---|
| | Before administration | 3rd day after administration | 7-th day after administration |
| Normal control group | 0.0±0.0 | 0.0±0.0 | 0.0±0.0 |
| Model control group | 2.7±0.7 | 2.7±0.7 | 2.9±0.6 |
| Celecoxib group | 2.7±0.5 | 2.9±0.3 | 2.4±0.5* |
| PC-01 low-dose group | 2.9±0.6 | 2.8±0.4 | 2.3±0.5** |
| PC-01 medium-dose group | 2.8±0.6 | 2.5±0.5 | 2.2±0.6** |
| PC-01 high-dose group | 2.9±0.3 | 2.2±0.4* | 1.6±0.5** |
| NOTE: compared with the model control group ***P*≤0.01, **P*≤0.05. | | | |

Influence of SP-C01 on chronic granulomatous model in rats
Influence of SP-C01 on chronic granuloma in rats

As shown in Table 15, compared with the model control group, the chronic granuloma swelling degree of rats in the SP-C01 low-dose group, SP-C01 medium-dose group, and SP-C01 high-dose group were significantly reduced (*P*≤0.01).

**Table 15 Influence of SP-C01 on chronic** granuloma **in rats (n=10)**

| Group | Dose (mg/kg) | Volume (mL/kg) | Swelling degree |
|---|---|---|---|
| Model control group | | 10 | 1.2570±0.26 |
| Celecoxib group | 20 | 10 | 0.9904±0.12++ |
| PC-01 low-dose group | 10 | 10 | 1.0566±0.10++ |
| PC-01 medium-dose group | 20 | 10 | 0.9877±0.07++ |
| PC-01 high-dose group | 40 | 10 | 0.8825±0.08++ |
| NOTE: compared with the normal control group, ++*P*≤0.01. | | | |

Experimental summary

Influence of SP-C01 on CFA-induced mouse rheumatoid arthritis model: under acute inflammatory conditions, the swelling degree of the mouse paws in the SP-C01 low-dose group was significantly reduced 4 h and 6 h after administration (*P*≤0.01), indicating that low-dose SP-C01 had an antagonistic effect on inflammation in CFA-induced rheumatoid arthritis model mice 4 h and 6 h after administration. The pain threshold of mice was significantly increased at 0.5 h, 1 h, 2 h, and 4 h after administration of low-dose SP-C01 (*P*≤0.01), indicating that low-dose SP-C01 had a significant analgesic effect on CFA-induced rheumatoid arthritis model mice 0.5 h, 1 h, 2 h, and 4 h after administration. The swelling degree of the mouse paws was significantly reduced 2 h, 4 h, and 7 d after the administration of medium-dose SP-C01 (*P*≤0.01), and the swelling degree of the mouse paws was significantly reduced 6 h and 24 h after the administration (*P*≤0.05), indicating that the medium-dose SP-C01 had an antagonistic effect on the inflammation of CFA-induced rheumatoid arthritis model mice 2 h, 4 h, 6 h, 24 h, and 7 d after administration. The pain threshold of the mouse was significantly increased 0.5 h, 1 h, 2 h, and 4 h after the administration of medium-dose SP-C01 (*P*≤0.01), indicating that the medium-dose SP-C01 had a significant analgesic effect on CFA-induced rheumatoid arthritis model mice 0.5 h, 1 h, 2 h, and 4 h after administration. The swelling degree of the mouse paws in the SP-C01 high-dose group was significantly reduced at 1 h, 2 h, 4 h, 6 h, 8 h, 12 h, 24 h, and 7 d after administration (*P*≤0.01), indicating that the high-dose SP-C01 had an antagonistic effect on inflammation in CFA-induced rheumatoid arthritis model mice at 1 h, 2 h, 4 h, 6 h, 8 h, 12 h, 24 h, and 7 d after administration. The pain threshold increase rate of the mouse in the SP-C01 high-dose group was significantly increased at 0.5 h, 1 h, 2 h, and 4 h after administration (*P*≤0.01), indicating that the high-dose SP-C01 had a significant analgesic effect on CFA-induced rheumatoid arthritis model mice at 0.5 h, 1 h, 2 h, and 4 h after administration.

In chronic inflammatory state, the pain threshold of mice administrated with high-dose SP-C01 for consecutive 7 days increased significantly (*P*≤0.05), indicating that high-dose SP-C01 administrated to the mice for consecutive 7 days had a significant analgesic effect on CFA-induced rheumatoid arthritis model mice. On the 3rd day of administration, the

joint index score of mice in the SP-C01 high-dose group was significantly reduced ($P \leq 0.05$), and the joint index scores of mice in the SP-C01 low-dose group, the SP-C01 medium-dose group, and the SP-C01 high-dose group were significantly reduced after 7 d of administration ($P \leq 0.01$), indicating that low-dose SP-C01 administrated for 7 d, medium-dose SP-C01 administrated for 7 d, and high-dose SP-C01 administrated for 3 d and for 7 d had a regulatory effect on the foot joints of CFA-induced rheumatoid arthritis model mice.

The effect of SP-C01 on chronic granuloma in rats: the chronic granuloma swelling degree of rats was significantly reduced by low-dose SP-C01, medium-dose SP-C01 and high-dose SP-C01 ($P \leq 0.01$), indicating that low-dose SP-C01, medium-dose SP-C01 and high-dose SP-C01 had effects on the granuloma swelling of rats of chronic granuloma model.

Test results indicates that:
In an acute inflammatory state, low-dose SP-C01 after administration (4 h, 6 h), medium-dose SP-C01 after administration (2 h, 4 h, 6 h, 24 h, 7 d) and high-dose SP-C01 after administration (1 h, 2 h, 4 h, 6 h, 8 h, 12 h, 24 h, 7 d) have an antagonistic effect on inflammation in CFA-induced rheumatoid arthritis model mice. In the acute inflammatory state, low-dose SP-C01 after administration (0.5 h, 1 h, 2 h, 4 h), medium-dose SP-C01 after administration (0.5 h, 1 h, 2 h, 4 h), high-dose SP-C01 after administration (0.5 h, 1 h, 2 h, 4 h) as well as high-dose SP-C01 in the chronic inflammatory state have a significant analgesic effect on CFA-induced rheumatoid arthritis model mice after continuous administration for 7 d. The positive drug celecoxib in the acute inflammatory state shows analgesic effect only after administration for 2 h. SP-C01 has anti-inflammatory and analgesic effects on CFA-induced rheumatoid arthritis model mice, with an onset time significantly shorter than celecoxib and a longer efficacy duration than celecoxib.

In a chronic inflammatory state, low-dose SP-C01 administrated for 7 d, medium-dose SP-C01 administrated for 7 d, high-dose SP-C01 administrated for 3 d and 7 d have a regulatory effect on the foot joints of the CFA-induced rheumatoid arthritis model mice. Compared with the positive drug celecoxib, the effects of all dose groups of SP-C01 are stronger than celecoxib.

Low-dose SP-C01, medium-dose SP-C01, and high-dose SP-C01 have an inhibitory effect on granuloma swelling in chronic granuloma model rats. Compared with the positive drug celecoxib, the effects of the SP-C01 low-dose and medium-dose groups are comparable to those of celecoxib, and the effect of the SP-C01 high-dose group is greater than that of celecoxib.

Although the present disclosure is described in detail in conjunction with the foregoing examples, they are only a part of, not all of, the examples of the present disclosure. Other examples could be obtained based on these examples without creative efforts, and all of these examples shall fall within the scope of the present disclosure.

## Claims

1. A compound having a structure shown in one selected from the group consisting of Formulas I, II, III, IV, V, and VI:

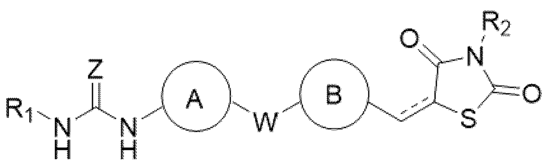

Formula I;

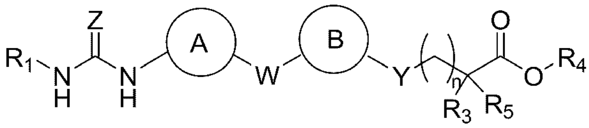

Formula II;

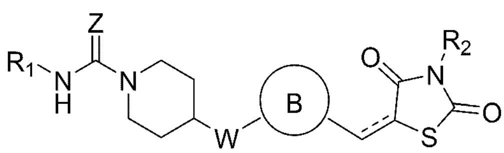

Formula III;

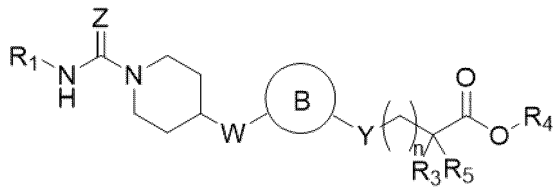

Formula IV;

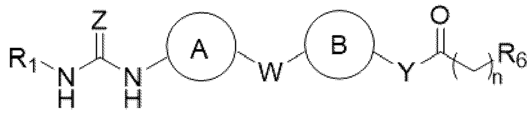

Formula V;

and

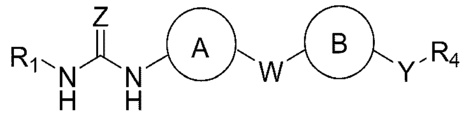

Formula VI;

wherein

$R_1$ is selected from the group consisting of adamantyl, aryl, alkyl-substituted aryl, halogenated aryl, haloalkyl-substituted aryl, haloalkoxy-substituted aryl, and haloaryloxy-substituted aryl; $R_2$ is selected from the group consisting of hydrogen, hydroxyl, alcoholic hydroxyl, amino, carboxyl, acyl, amido, and ester group; $R_3$ is selected from the group consisting of hydrogen, alkyl, alkoxy, hydroxyl, cyano, and carboxyl; $R_4$ is selected from the group consisting of hydrogen and alkyl; $R_5$ is selected from the group consisting of hydrogen, alkyl, alkoxy, hydroxyl, cyano, and carboxyl; $R_6$ is selected from the group consisting of hydrogen, hydroxyl, alkyl, ester group, and amino; A is selected from the group consisting of cycloalkyl, a heterocyclic group, and aryl; and B is selected from the group consisting of a single bond, cycloalkyl, a heterocyclic group, and aryl;
W is selected from the group consisting of a single bond, $-CH_2-$, -O-, -S-, -NH-, and

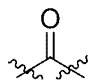

;

Y is selected from the group consisting of a single bond, $-CH_2-$, -O-, -S-, and -NH-;
Z is selected from the group consisting of $=CH_2$, =O, =S, and =NH; and
n is an integer of 0 to 12.

2. The compound as claimed in claim 1, wherein alkyl in the alkyl-substituted aryl and the haloalkyl-substituted aryl is independently selected from the group consisting of methyl, ethyl, propyl, butyl, pentyl, isobutyl, isopropyl, isopentyl, and tert-butyl;

alkoxy in the haloalkoxy-substituted aryl is selected from the group consisting of methoxy, ethoxy, propoxy, isopropoxy, butoxy, cyclopentyloxy, cyclohexyloxy, phenoxy, and benzyloxy; and
halogen in the halogenated aryl, the haloalkyl-substituted aryl, and the haloalkoxy-substituted aryl is independently selected from the group consisting of -F, -Cl, and -Br.

3. The compound as claimed in claim 1, wherein the cycloalkyl is unsubstituted or substituted C3 to C8 cycloalkyl, and a substituent of the substituted C3 to C8 cycloalkyl is independently selected from the group consisting of -F, -Cl, -Br, -OH, $-NH_2$, $-NHCH_3$, $-N(CH_3)_2$, and C1 to C6 alkyl;

the heterocyclic group is independently an unsubstituted or substituted 3- to 10-membered heterocyclic group, and a substituent of the substituted 3- to 10-membered heterocyclic group is independently selected from the group consisting of -F, -Cl, -Br, -OH, $-NH_2$, $-NHCH_3$, $-N(CH_3)_2$, and C1 to C6 alkyl; and
the aryl is independently selected from the group consisting of substituted or unsubstituted phenyl, substituted or

unsubstituted pyridyl, and substituted or unsubstituted naphthyl, and a substituent of the substituted phenyl, the substituted pyridyl, or the substituted naphthyl is independently selected from the group consisting of -F, -Cl, -Br, -OH, $-NH_2$, $-NHCH_3$, $-N(CH_3)_2$, and C1 to C6 alkyl.

**4.** The compound as claimed in claim 1, wherein $R_1$ is selected from the group consisting of adamantyl, halogenated aryl, haloalkyl-substituted aryl, and haloalkoxy-substituted aryl; $R_2$ is selected from the group consisting of hydrogen and ester group; $R_3$ is selected from the group consisting of hydrogen and alkyl; $R_4$ is selected from the group consisting of hydrogen, methyl, and ethyl; $R_5$ is selected from the group consisting of hydrogen and alkyl; $R_6$ is selected from the group consisting of hydroxyl, amino, alkyl, and ester group; A is selected from the group consisting of cyclohexyl and phenyl, and B is selected from the group consisting of a single bond and phenyl;

W is selected from the group consisting of a single bond and -O-;
Y is selected from the group consisting of a single bond, -O-, and -NH-;
Z is =O; and
n is an integer of 0 to 2.

**5.** The compound as claimed in claim 4, wherein $R_1$ is selected from the group consisting of adamantyl,

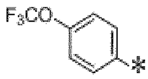,

and

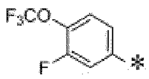;

$R_2$ is selected from the group consisting of hydrogen and $-CH_2-C(O)-CH_3$; $R_3$ is selected from the group consisting of hydrogen and methyl; $R_4$ is hydrogen; $R_5$ is selected from the group consisting of hydrogen and methyl; and $R_6$ is selected from the group consisting of hydroxyl, $-NH_2$, methyl, $-C(O)-O-CH_2-CH_3$, and sec-butyl.

**6.** The compound as claimed in any one of claims 1 to 5, wherein the compound has any one of the following structures:

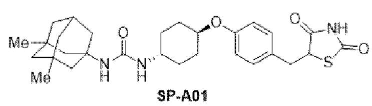
SP-A01

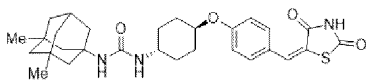
SP-B01

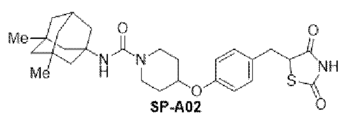
SP-A02

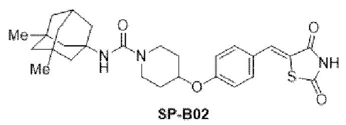
SP-B02

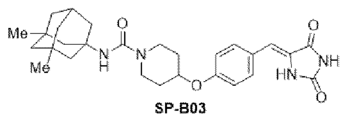
SP-B03

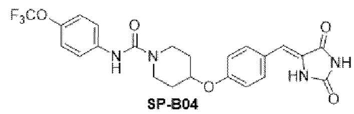
SP-B04

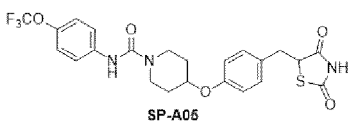
SP-A05

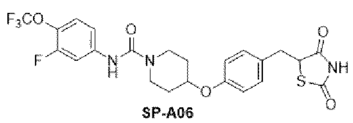
SP-A06

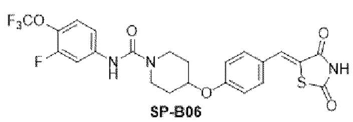
SP-B06

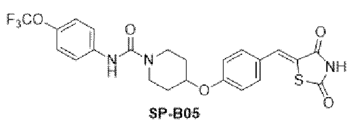
SP-B05

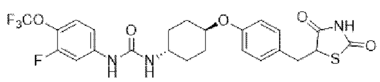
SP-A07

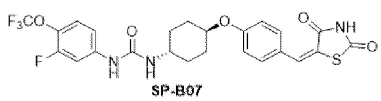
SP-B07

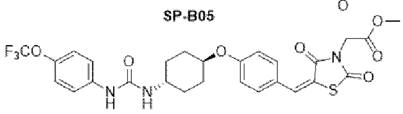
SP-D01

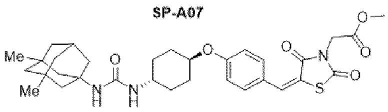
SP-D04

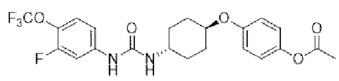
SP-C01

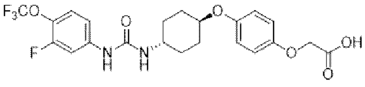
SP-C02

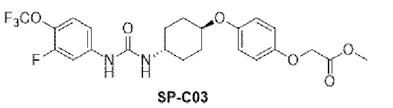
SP-C03

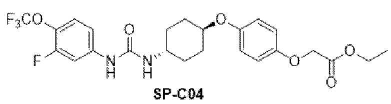
SP-C04

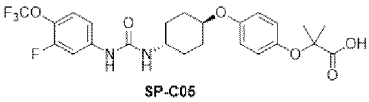
SP-C05

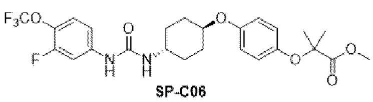
SP-C06

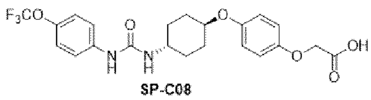
SP-C08

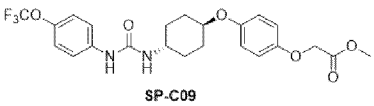
SP-C09

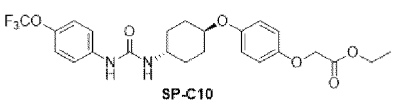
SP-C10

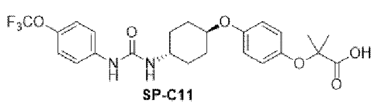
SP-C11

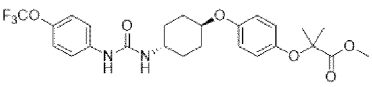
SP-C12

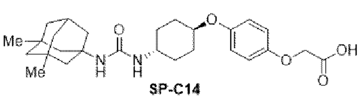
SP-C14

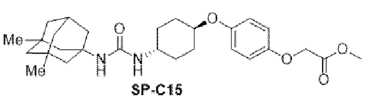
SP-C15

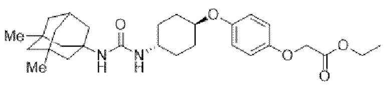
SP-C16

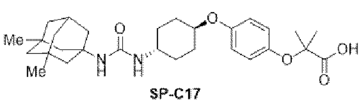
SP-C17

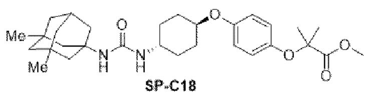
SP-C18

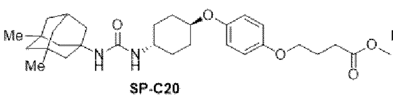
SP-C20

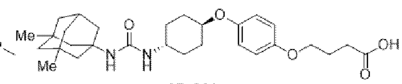
SP-C21

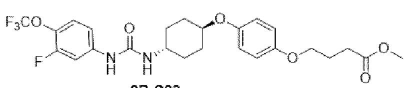
SP-C23

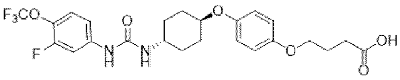
SP-C24

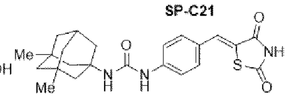
SP-E01

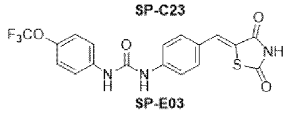
SP-E03

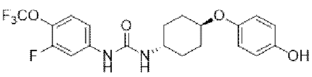
SP-C01b

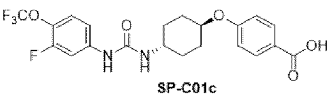
SP-C01c

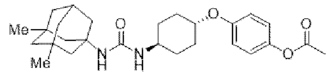
SP-C01d

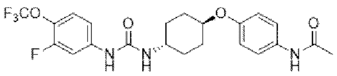
SP-C01e

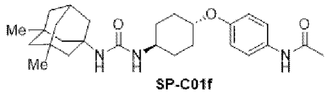
SP-C01f

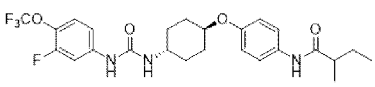
SP-C01g

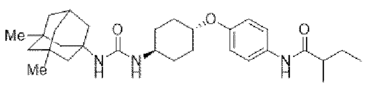
SP-C01h

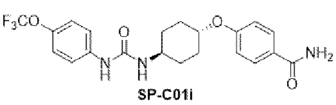
SP-C01i

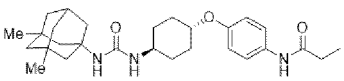
SP-C01j

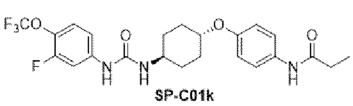
SP-C01k

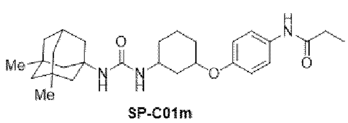
SP-C01m

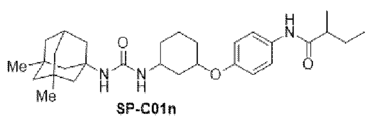
SP-C01n

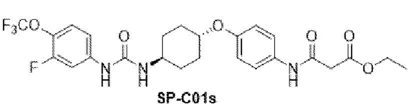
SP-C01s

**7.** A method for preparing the compound as claimed in any one of claims 1 to 6, wherein

(1) under the condition that the compound has a structure shown in Formula I, the method comprises:

subjecting a compound a and a compound b to a first substitution to obtain a compound c;
subjecting the compound c and a compound d to a first condensation to obtain a compound e;
subjecting the compound e to a first hydrolysis to obtain a compound f;
subjecting the compound f, a compound w, and a compound af to a first nucleophilic substitution to obtain a compound g; wherein the compound g is the compound having the structure shown in Formula I where a thiazolidinedione group is linked to a double bond; and
subjecting the compound g to a first reduction to obtain the compound having the structure shown in Formula I where the thiazolidinedione group is linked to a single bond; wherein
structural formulas of the compound a, the compound b, the compound c, the compound d, the compound e, the compound f, the compound g, the compound w, and the compound af are as follows:

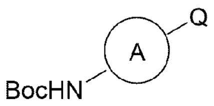
Compound a

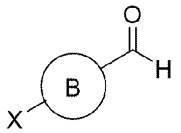
Compound b

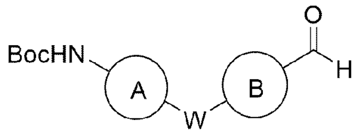
Compound c

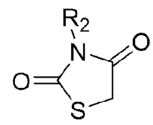
Compound d

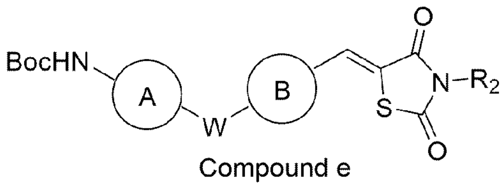
Compound e

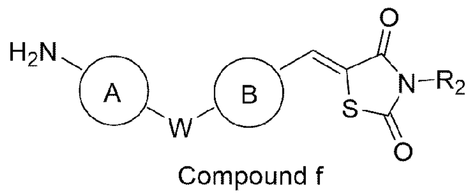
Compound f

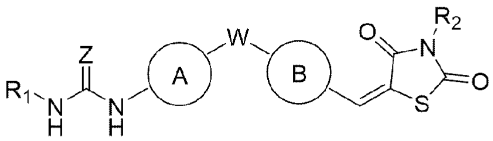
Compound g

$R_1-NH_2$
Compound w

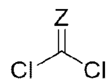
Compound af

;

wherein Q in the compound a is selected from the group consisting of H, hydroxyl, amino, sulfhydryl, carboxyl, and acyl chloride; and X in the compound b is selected from the group consisting of H, hydroxyl, halogen, and haloalkyl;

(2) under the condition that the compound has a structure shown in Formula II, the method comprises:

subjecting the compound a and a compound h to a first substitution to obtain a compound i;
subjecting the compound i to a first hydrolysis to obtain a compound j;
subjecting the compound j and a compound aa to a second substitution to obtain a compound k;
subjecting the compound k to a second hydrolysis to obtain a compound l; and
subjecting the compound l, the compound w, and the compound af to nucleophilic substitution to obtain the compound having the structure shown in Formula II; wherein
structural formulas of the compound h, the compound i, the compound j, the compound k, the compound l, and the compound aa are as follows:

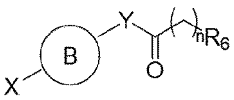
Compound h

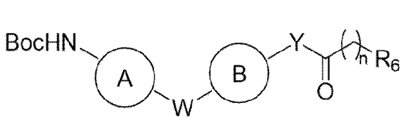
Compound i

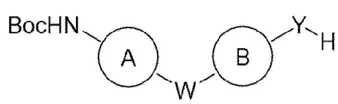
Compound j

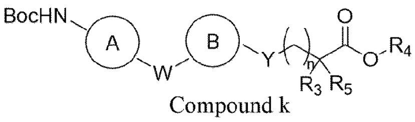
Compound k
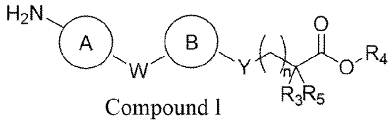
Compound l
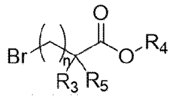
Compound aa
;

X in the compound h is selected from the group consisting of H, hydroxyl, halogen, and haloalkyl;

(3) under the condition that the compound has a structure shown in Formula III, the method comprises:

subjecting a compound m and the compound b to a first substitution to obtain a compound n;
subjecting the compound n and the compound d to a first condensation to obtain a compound o;
subjecting the compound o to a first hydrolysis to obtain a compound p;
subjecting the compound p, the compound w, and the compound af to a first nucleophilic substitution to obtain a compound q; wherein the compound q is the compound having the structure shown in Formula III where a thiazolidinedione group is linked to a double bond; and
subjecting the compound q to a first reduction to obtain the compound having the structure shown in Formula III where the thiazolidinedione group is linked to a single bond; wherein
structural formulas of the compound m, the compound n, the compound o, the compound p, and the compound q are as follows:

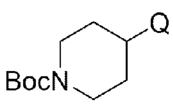
Compound m
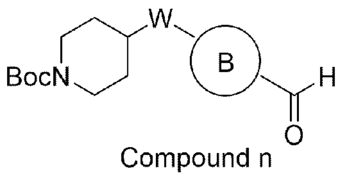
Compound n
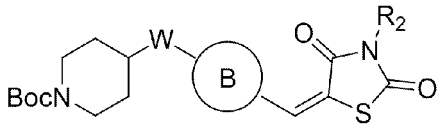
Compound o

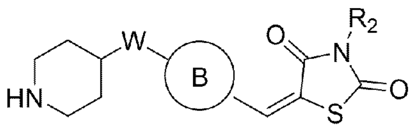
Compound p
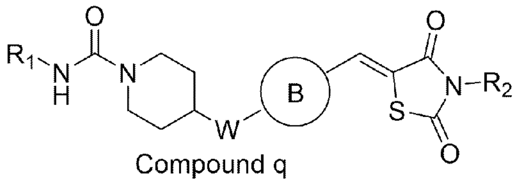
Compound q
;

wherein Q in the compound m is selected from the group consisting of H, hydroxyl, amino, sulfhydryl, carboxyl, and acyl chloride;

(4) under the condition that the compound has a structure shown in Formula IV, the method comprises:

subjecting the compound m and the compound h to a first substitution to obtain a compound r;
subjecting the compound r to a first hydrolysis to obtain a compound s;
subjecting the compound s and the compound aa to a second substitution to obtain a compound t;
subjecting the compound t to a second hydrolysis to obtain a compound u; and
subjecting the compound u, the compound w, and the compound af to a first nucleophilic substitution to obtain the compound having the structure shown in Formula IV; wherein
structural formulas of the compound r, the compound s, the compound t, and the compound u are as follows:

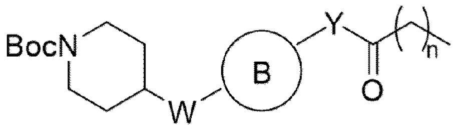
Compound r
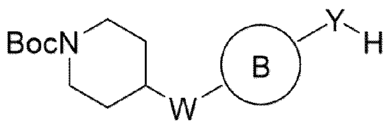
Compound s

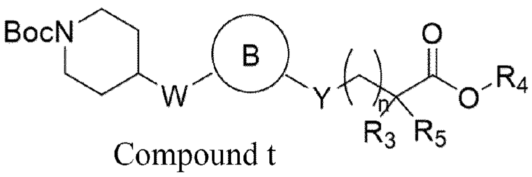

Compound t

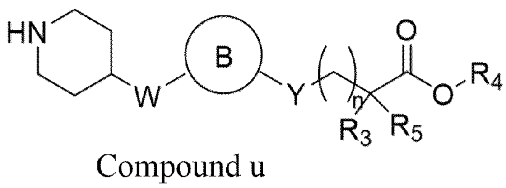

Compound u

;

(5) under the condition that the compound has a structure shown in Formula V, the method comprises:

subjecting the compound a and the compound h to a first substitution to obtain a compound i;
subjecting the compound i to a first hydrolysis to obtain a compound v; and
subjecting the compound v, the compound w, and the compound af to a first nucleophilic substitution to obtain the compound having the structure shown in Formula V; wherein
structural formulas of the compound a, the compound h, the compound i, and the compound v are as follows:

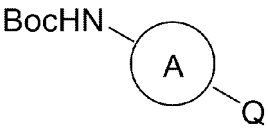

Compound a

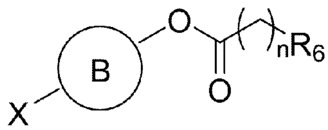

Compound h

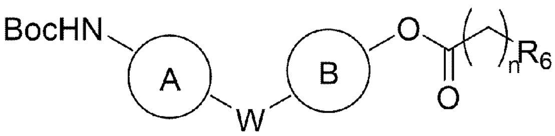

Compound i

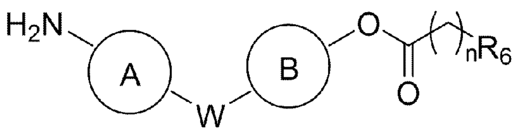

Compound v

;

(6) under the condition that the compound has a structure shown in Formula VI, the method comprises:

subjecting the compound a and a compound x to a first substitution to obtain a compound y;
subjecting the compound y to a first hydrolysis to obtain a compound z; and
subjecting the compound z, the compound w, and the compound af to a first nucleophilic substitution to obtain the compound having the structure shown in Formula V; wherein
structural formulas of the compound a, the compound x, the compound y, and the compound z are as follows:

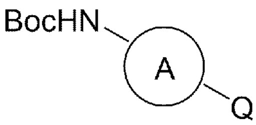

Compound a

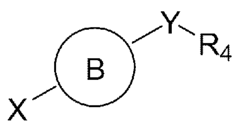

Compound x

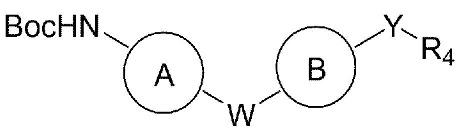

Compound y

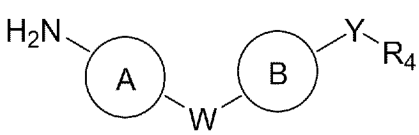

Compound z

;

wherein X in the compound x is selected from the group consisting of H, hydroxyl, halogen, and haloalkyl; and

(7) under the condition that the compound has a structure shown in Formula V or VI where Y is -NH-, the method comprises:

subjecting the compound a and a compound ab to a first substitution to obtain a compound ac;
subjecting the compound ac to a first hydrolysis to obtain a compound ad;
subjecting the compound ad, the compound w, and the compound af to nucleophilic substitution to obtain a compound ae;
subjecting the compound ae to a first reduction to obtain the compound having the structure shown in Formula VI; and
subjecting the compound having the structure shown in Formula VI and a compound ag to condensation to obtain the compound having the structure shown in Formula V,

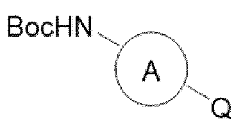
Compound a

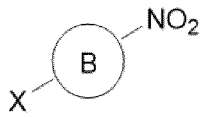
Compound ab

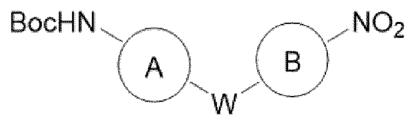
Compound ac

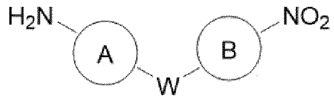
Compound ad

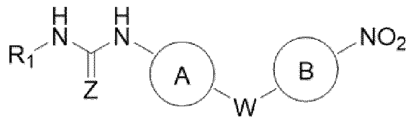
Compound ae

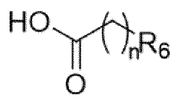
Compound ag

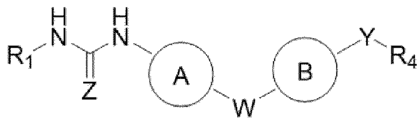
Formula VI

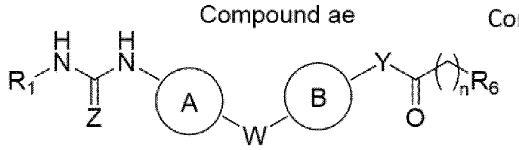
Formula V

;

wherein X in the compound ab is selected from the group consisting of H, hydroxyl, halogen, and haloalkyl.

**8.** Use of the compound as claimed in any one of claims 1 to 6 and a pharmaceutically acceptable deuterated product, salt, or hydrate thereof, or the compound prepared by the method as claimed in claim 7 in preparation of a peroxisome proliferator-activated receptors (PPARs) agonist and/or a soluble epoxide hydrolase (sEH) inhibitor.

**9.** The use as claimed in claim 8, wherein the PPARs agonist and the sEH inhibitor are used to treat sEH-mediated and PPAR-mediated diseases; and
the sEH-mediated and PPARs-mediated diseases comprise one selected from the group consisting of an inflammatory disease, a pain, sepsis, a cardiovascular disease, a neurodegenerative disease, diabetes, a diabetic complication, depression, liver fibrosis, renal failure, a chronic obstructive pulmonary disease, and pulmonary arterial hypertension.

**10.** The use as claimed in claim 9, wherein the inflammatory disease comprises one selected from the group consisting of nonalcoholic steatohepatitis and chronic nephritis; and
the pain comprises a neuropathic pain.

**11.** Use of the compound as claimed in any one of claims 1 to 6 and a pharmaceutically acceptable deuterated product, salt, or hydrate thereof in treatment of sEH-mediated and PPARs-mediated diseases; wherein
the sEH-mediated and PPARs-mediated diseases comprise one selected from the group consisting of an inflammatory disease, a pain, sepsis, a cardiovascular disease, a neurodegenerative disease, diabetes, a diabetic complication, depression, liver fibrosis, renal failure, a chronic obstructive pulmonary disease, and pulmonary arterial hypertension.

**12.** A drug, comprising the compound as claimed in any one of claims 1 to 6 and a pharmaceutically acceptable deuterated product, salt, or hydrate thereof, or the compound prepared by the method as claimed in claim 7.

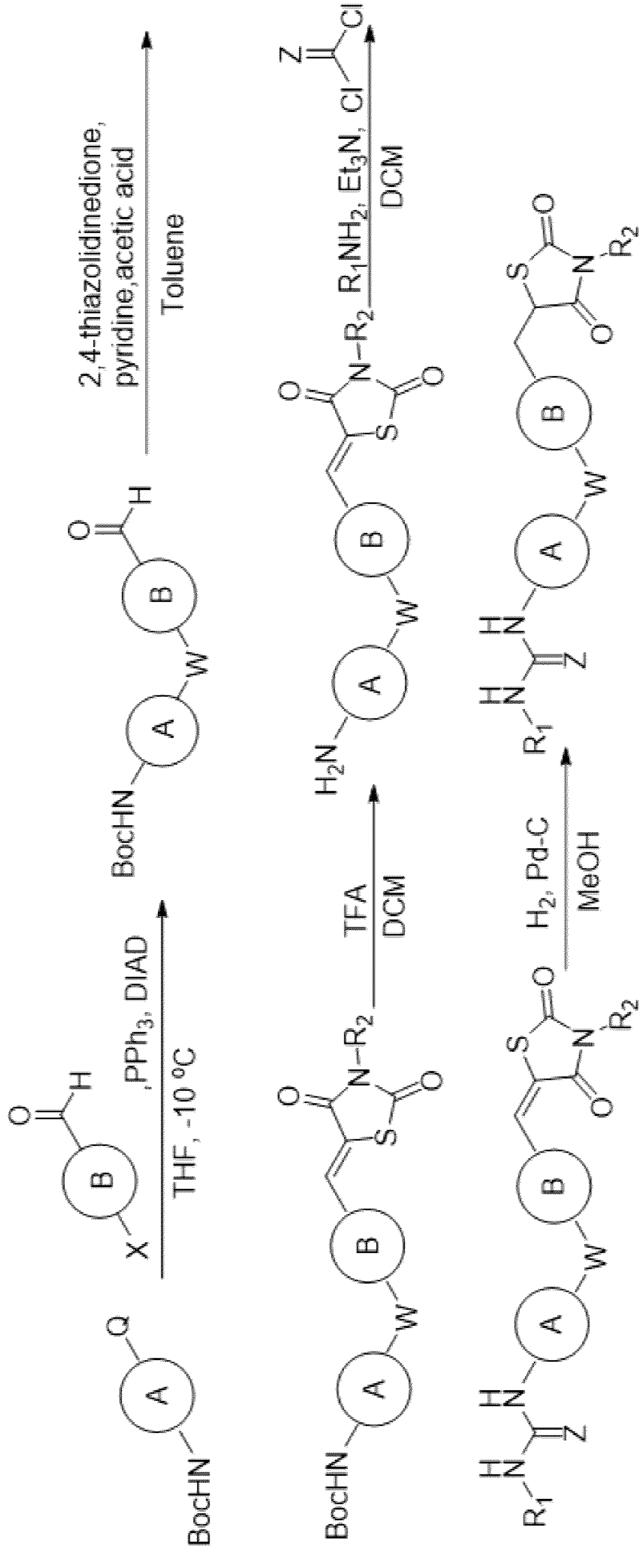
PPh$_3$, DIAD
THF, -10 ºC
2,4-thiazolidinedione, pyridine, acetic acid
Toluene
TFA
DCM
R$_1$NH$_2$, Et$_3$N,
DCM
H$_2$, Pd-C
MeOH

FIG. 1

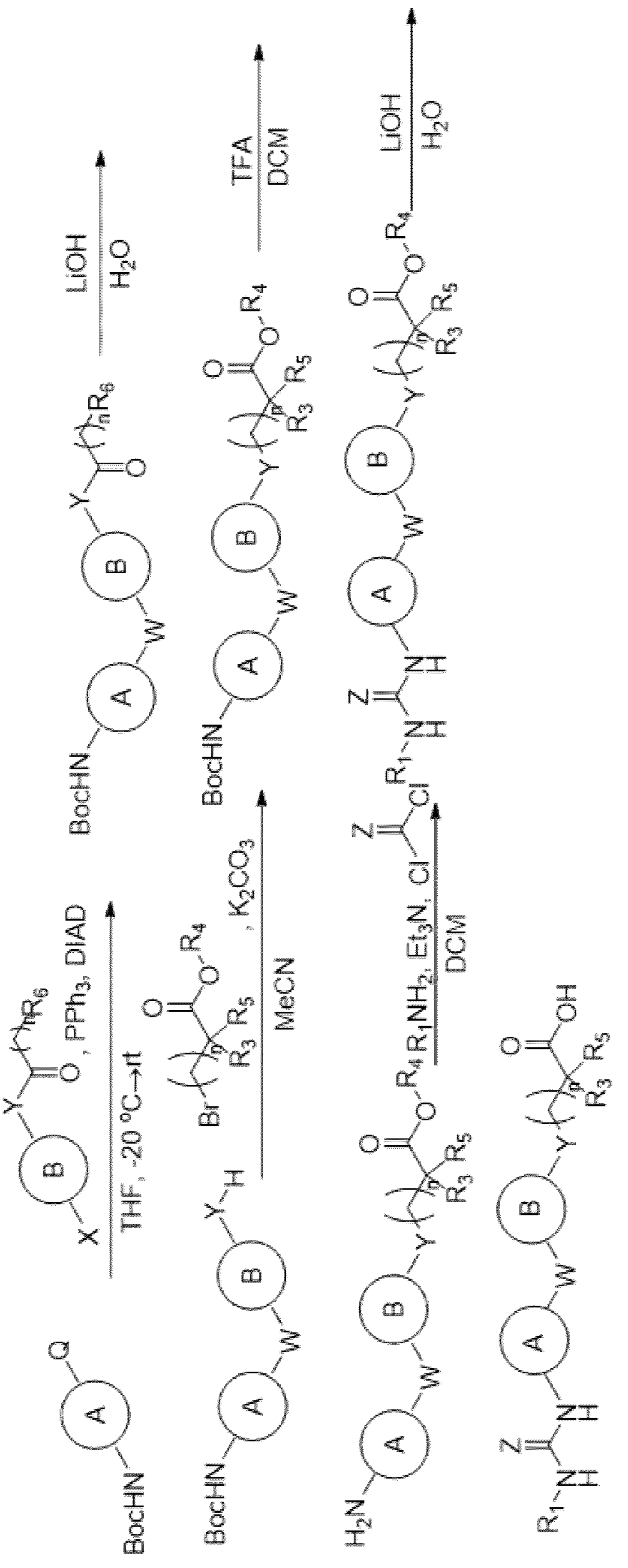
PPh$_3$, DIAD
THF, -20 °C→rt
LiOH
H$_2$O
K$_2$CO$_3$
MeCN
TFA
DCM
R$_1$NH$_2$, Et$_3$N, Cl
DCM
LiOH
H$_2$O

FIG. 2

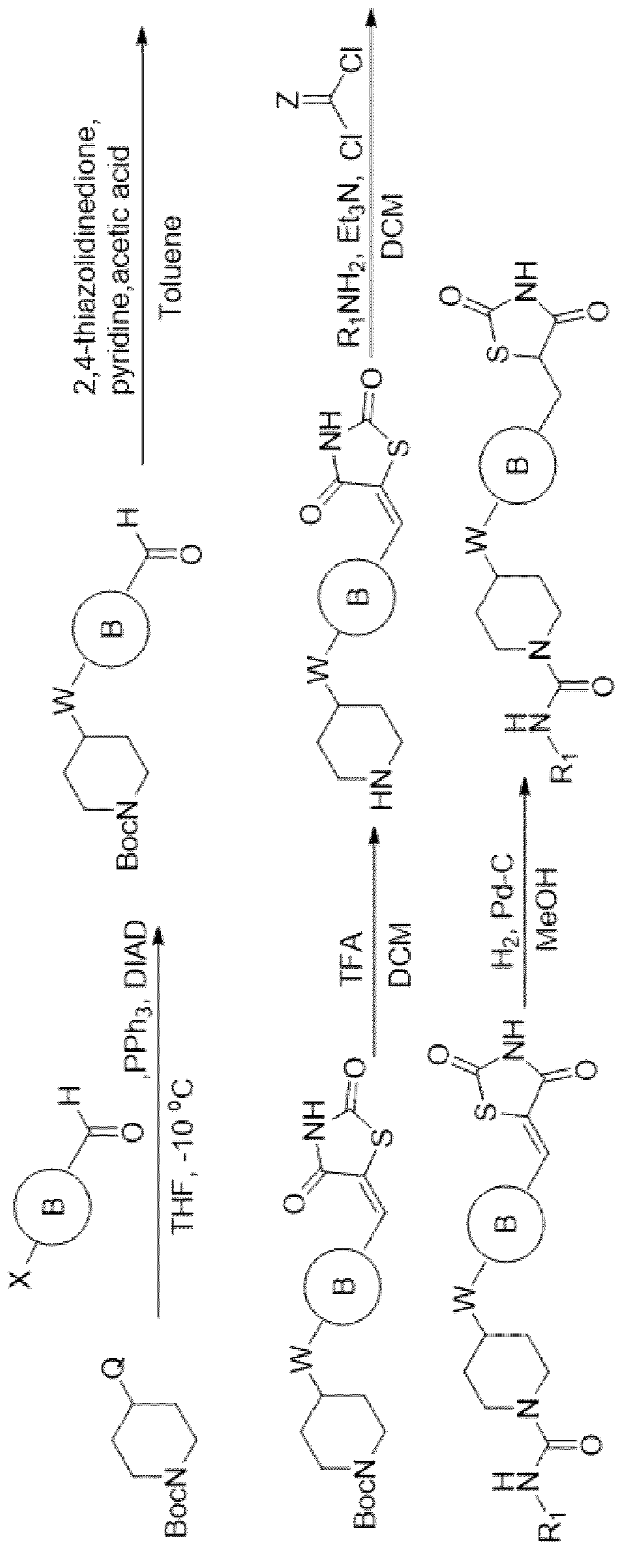

,PPh$_3$, DIAD
THF, -10 °C
2,4-thiazolidinedione,
pyridine,acetic acid
Toluene
TFA
DCM
$R_1NH_2$, $Et_3N$,
DCM
$H_2$, Pd-C
MeOH


FIG. 3

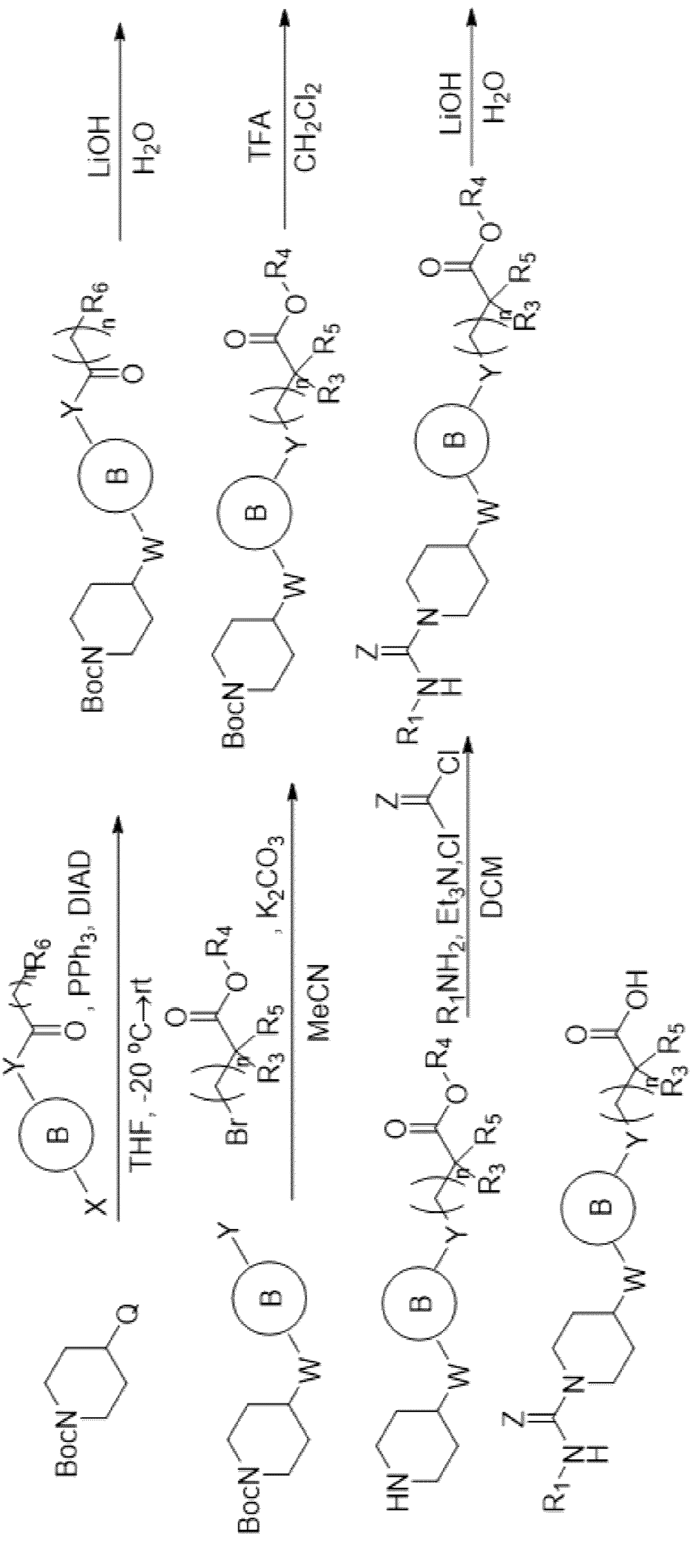
PPh$_3$, DIAD
THF, -20 °C→rt
K$_2$CO$_3$
MeCN
R$_1$NH$_2$, Et$_3$N,
DCM
LiOH
H$_2$O
TFA
CH$_2$Cl$_2$
LiOH
H$_2$O

FIG. 4

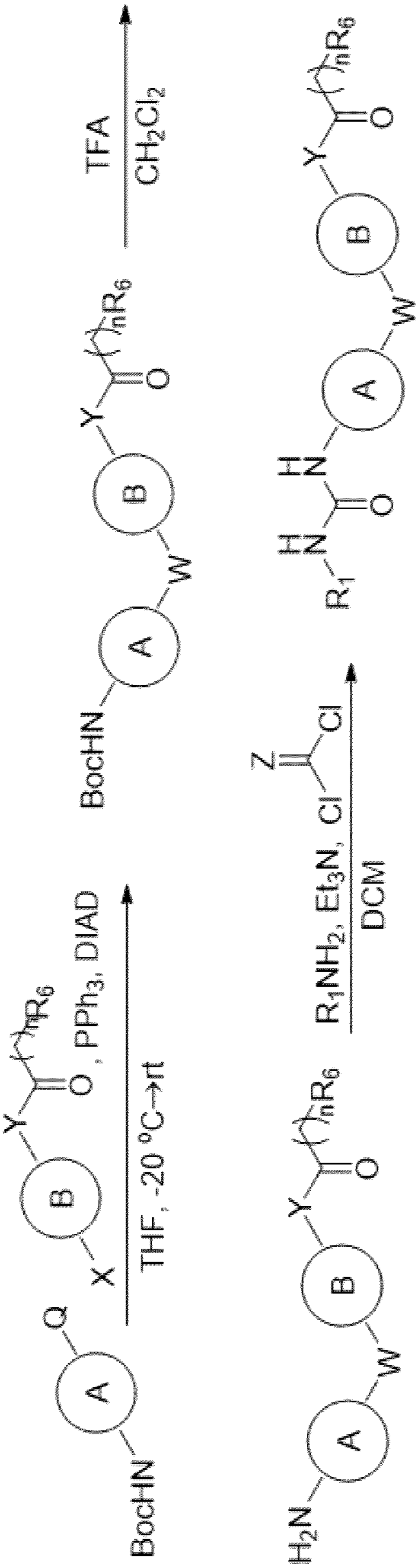


FIG. 5

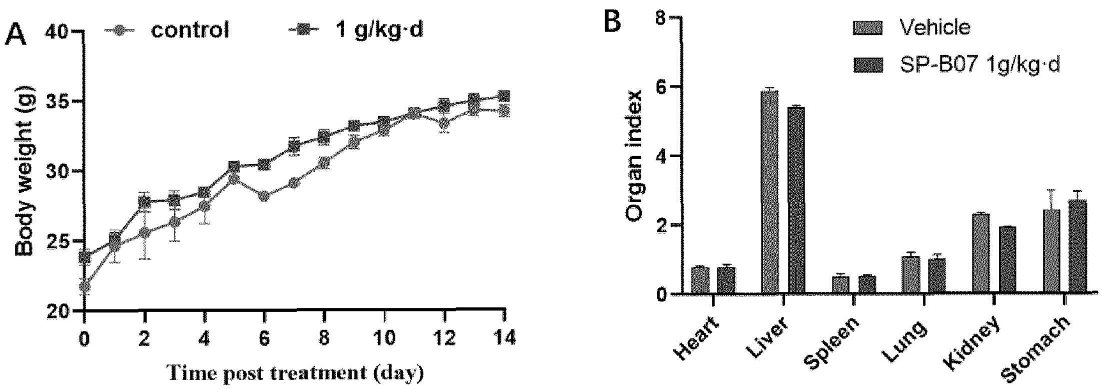


FIG. 6

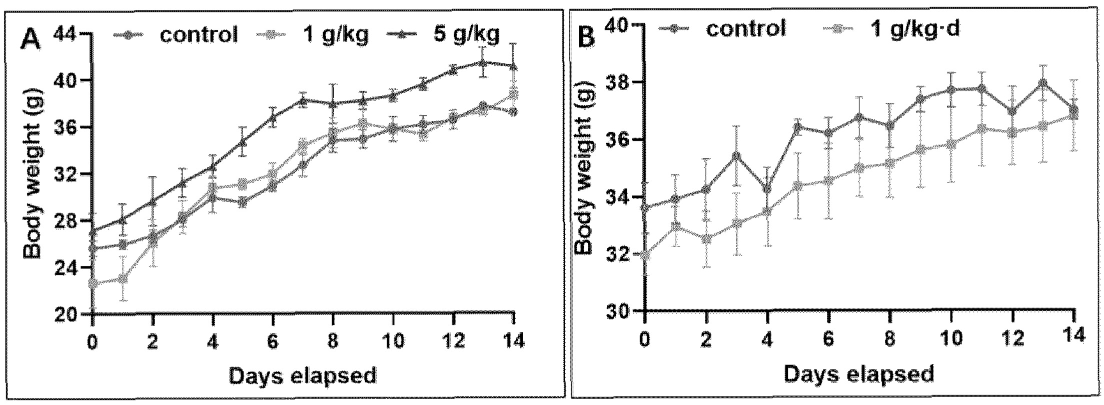


FIG. 7

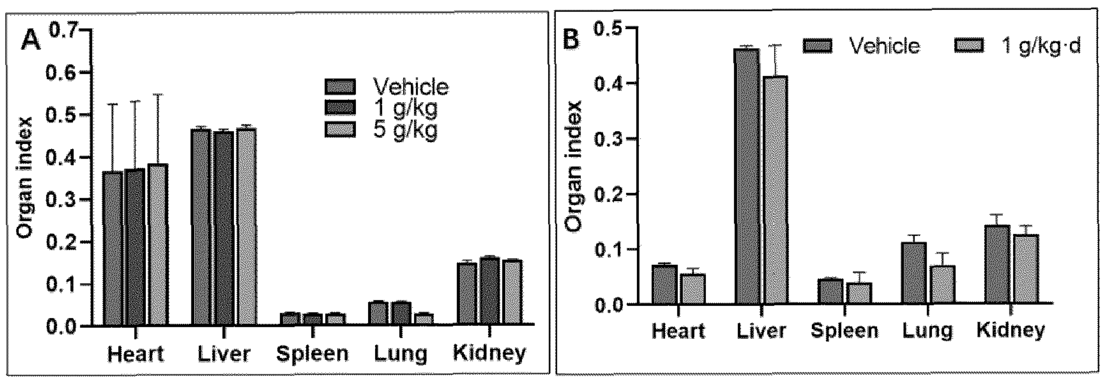


FIG. 8

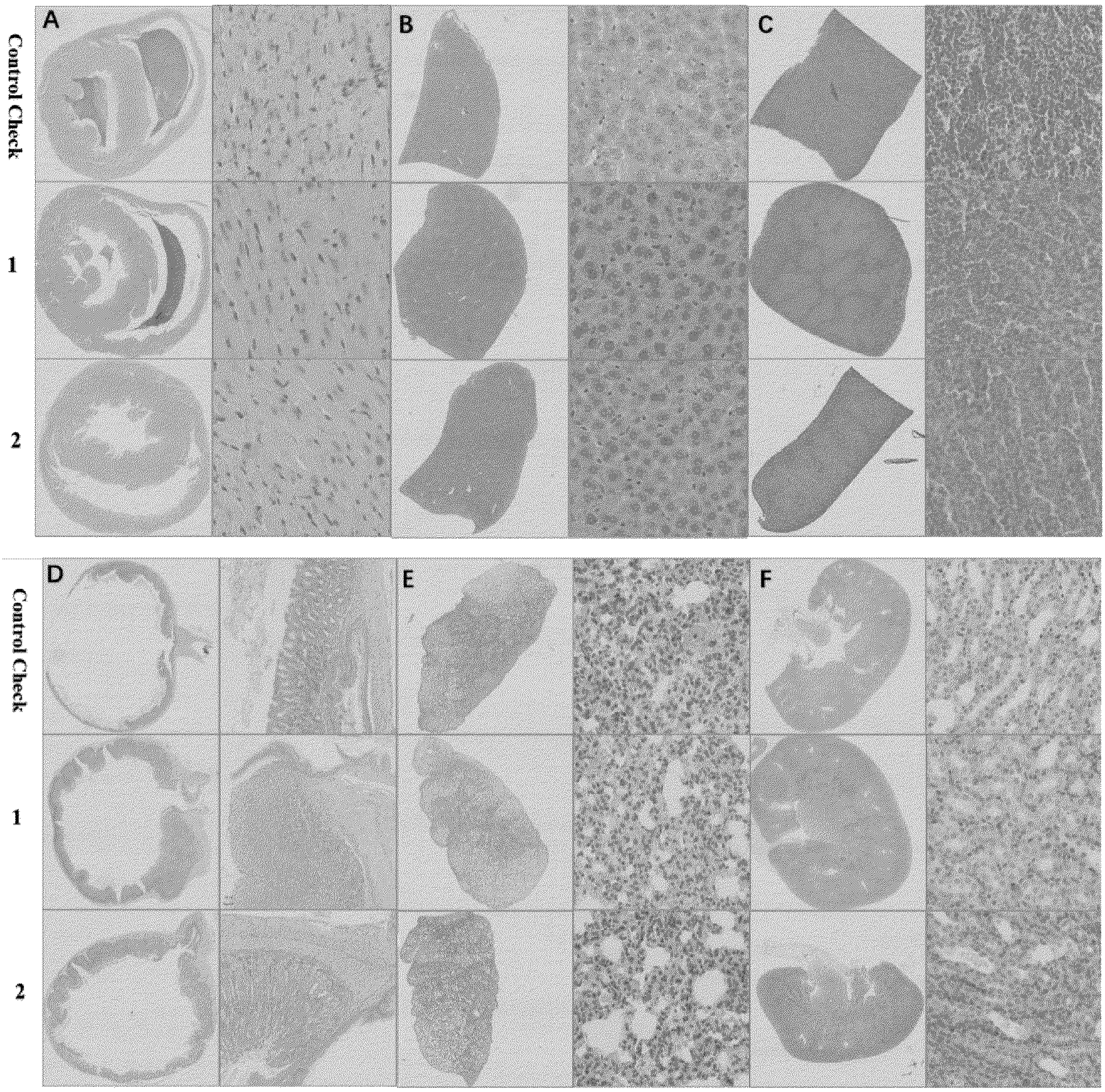


FIG. 9

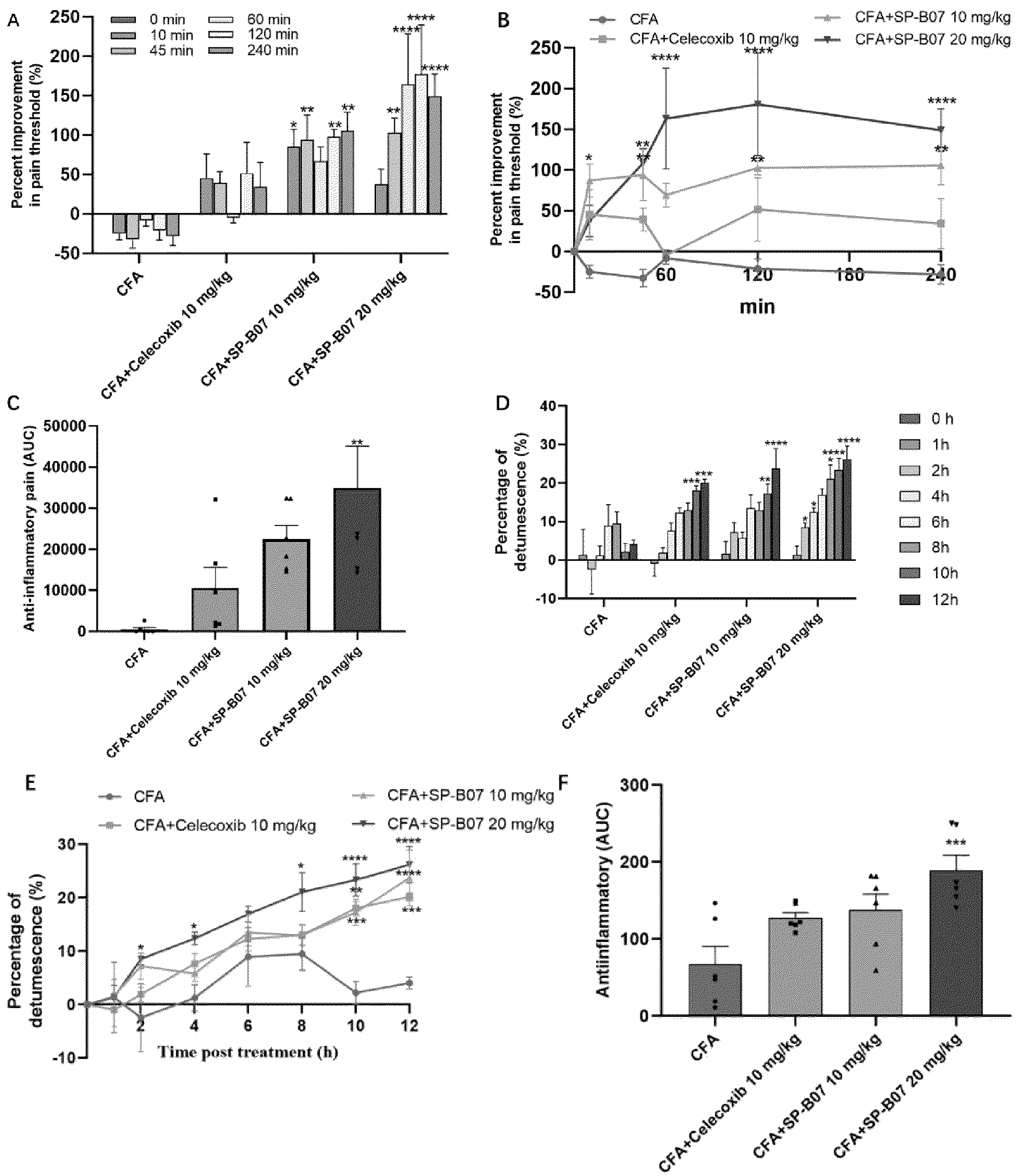
A
Percent improvement in pain threshold (%)
0 min
10 min
45 min
60 min
120 min
240 min
CFA
CFA+Celecoxib 10 mg/kg
CFA+SP-B07 10 mg/kg
CFA+SP-B07 20 mg/kg
B
CFA
CFA+Celecoxib 10 mg/kg
CFA+SP-B07 10 mg/kg
CFA+SP-B07 20 mg/kg
min
C
Anti-inflammatory pain (AUC)
D
Percentage of detumescence (%)
0 h
1h
2h
4h
6h
8h
10h
12h
E
Time post treatment (h)
F
Antiinflammatory (AUC)

FIG. 10

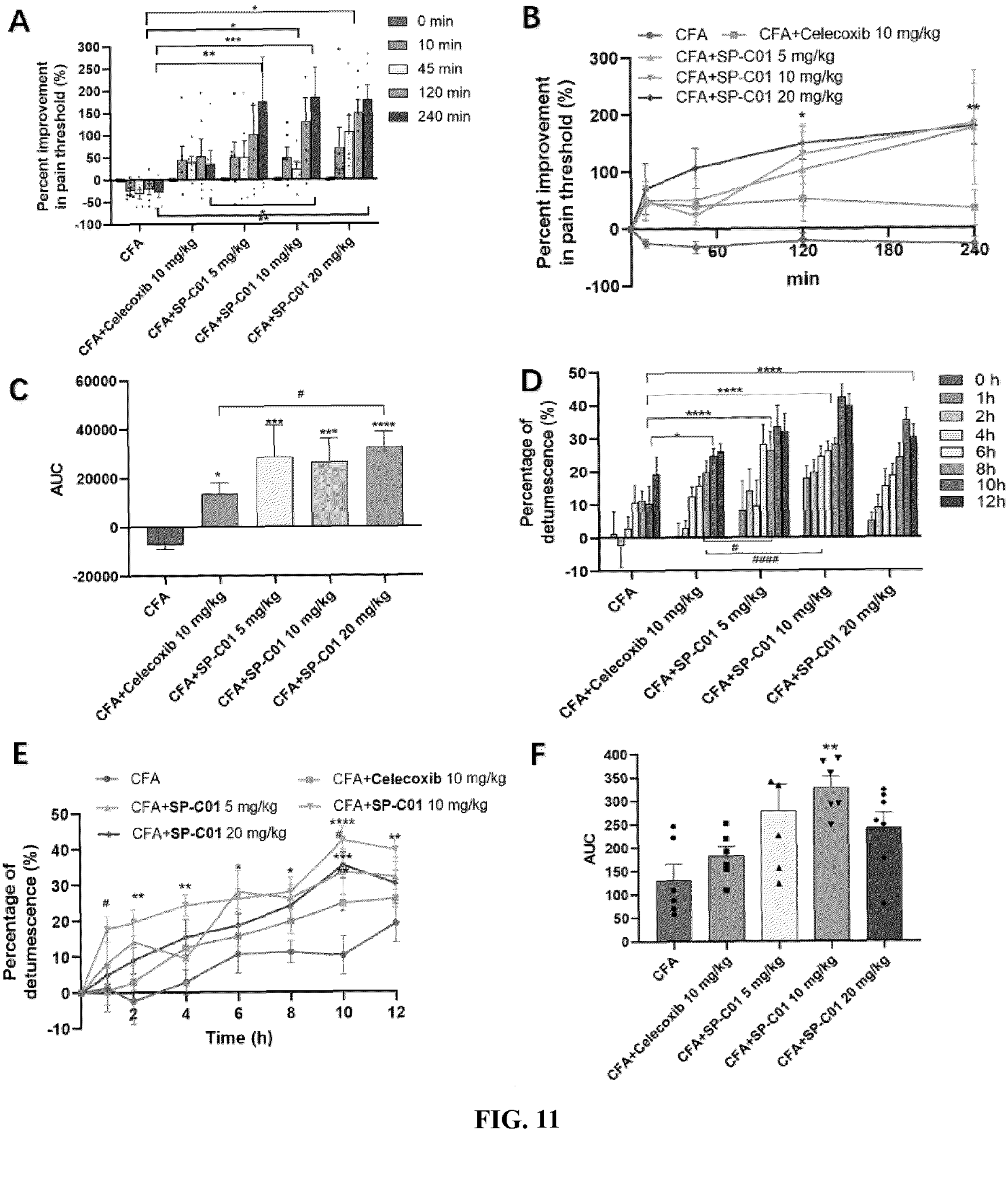
A
0 min
10 min
45 min
120 min
240 min
Percent improvement in pain threshold (%)
CFA
CFA+Celecoxib 10 mg/kg
CFA+SP-C01 5 mg/kg
CFA+SP-C01 10 mg/kg
CFA+SP-C01 20 mg/kg
B
CFA
CFA+Celecoxib 10 mg/kg
CFA+SP-C01 5 mg/kg
CFA+SP-C01 10 mg/kg
CFA+SP-C01 20 mg/kg
Percent improvement in pain threshold (%)
min
C
AUC
D
0 h
1h
2h
4h
6h
8h
10h
12h
Percentage of detumescence (%)
E
CFA
CFA+Celecoxib 10 mg/kg
CFA+SP-C01 5 mg/kg
CFA+SP-C01 10 mg/kg
CFA+SP-C01 20 mg/kg
Percentage of detumescence (%)
Time (h)
F
AUC

FIG. 11

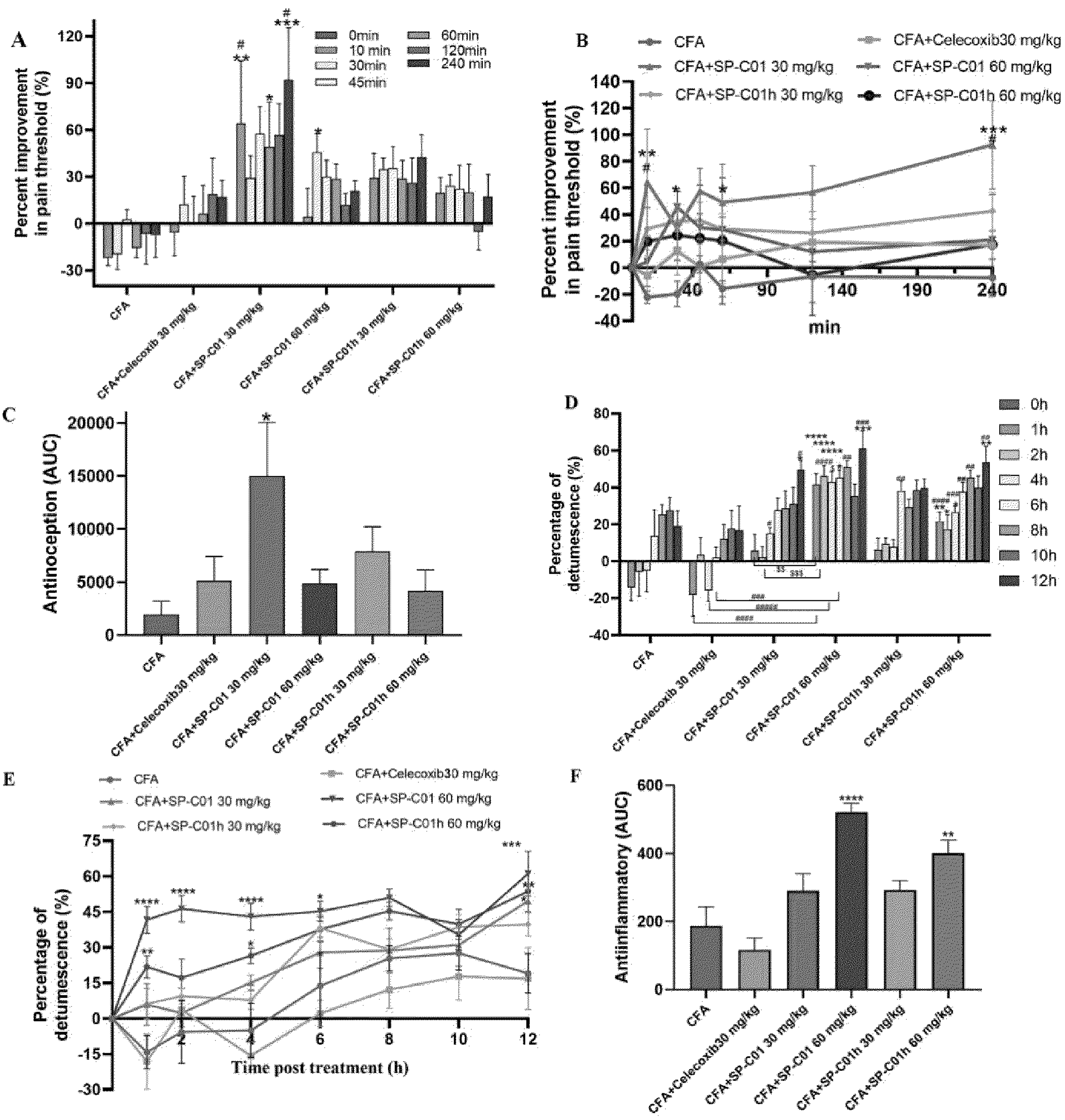
A
0min
10 min
30min
45min
60min
120min
240 min
Percent improvement in pain threshold (%)
CFA
CFA+Celecoxib 30 mg/kg
CFA+SP-C01 30 mg/kg
CFA+SP-C01 60 mg/kg
CFA+SP-C01h 30 mg/kg
CFA+SP-C01h 60 mg/kg
B
CFA
CFA+SP-C01 30 mg/kg
CFA+SP-C01h 30 mg/kg
CFA+Celecoxib30 mg/kg
CFA+SP-C01 60 mg/kg
CFA+SP-C01h 60 mg/kg
Percent improvement in pain threshold (%)
min
C
Antinociception (AUC)
CFA
CFA+Celecoxib30 mg/kg
CFA+SP-C01 30 mg/kg
CFA+SP-C01 60 mg/kg
CFA+SP-C01h 30 mg/kg
CFA+SP-C01h 60 mg/kg
D
0h
1h
2h
4h
6h
8h
10h
12h
Percentage of detumescence (%)
CFA
CFA+Celecoxib 30 mg/kg
CFA+SP-C01 30 mg/kg
CFA+SP-C01 60 mg/kg
CFA+SP-C01h 30 mg/kg
CFA+SP-C01h 60 mg/kg
E
CFA
CFA+SP-C01 30 mg/kg
CFA+SP-C01h 30 mg/kg
CFA+Celecoxib30 mg/kg
CFA+SP-C01 60 mg/kg
CFA+SP-C01h 60 mg/kg
Percentage of detumescence (%)
Time post treatment (h)
F
Antiinflammatory (AUC)
CFA
CFA+Celecoxib30 mg/kg
CFA+SP-C01 30 mg/kg
CFA+SP-C01 60 mg/kg
CFA+SP-C01h 30 mg/kg
CFA+SP-C01h 60 mg/kg

**FIG. 12**

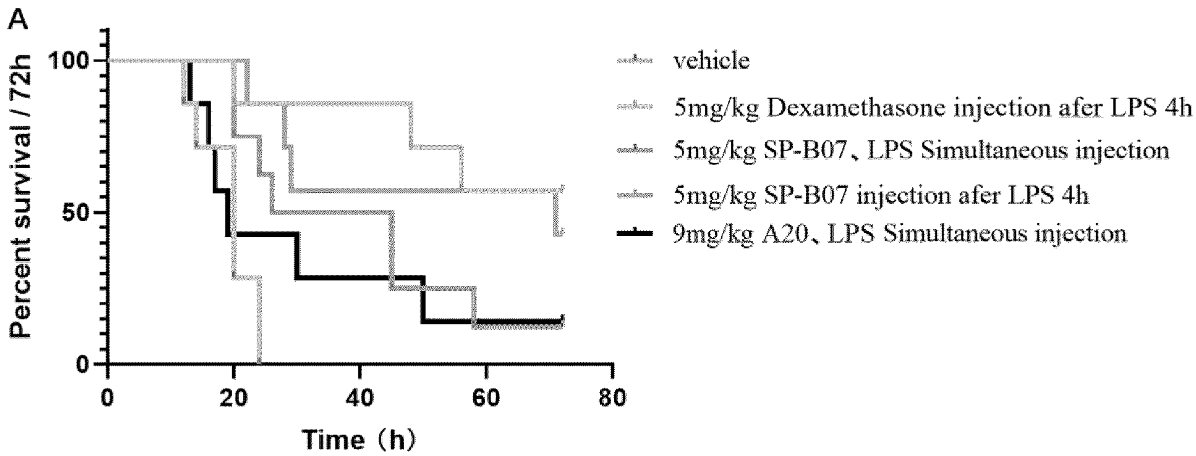
A
Percent survival / 72h
Time (h)
vehicle
5mg/kg Dexamethasone injection afer LPS 4h
5mg/kg SP-B07、LPS Simultaneous injection
5mg/kg SP-B07 injection afer LPS 4h
9mg/kg A20、LPS Simultaneous injection

**FIG. 13**

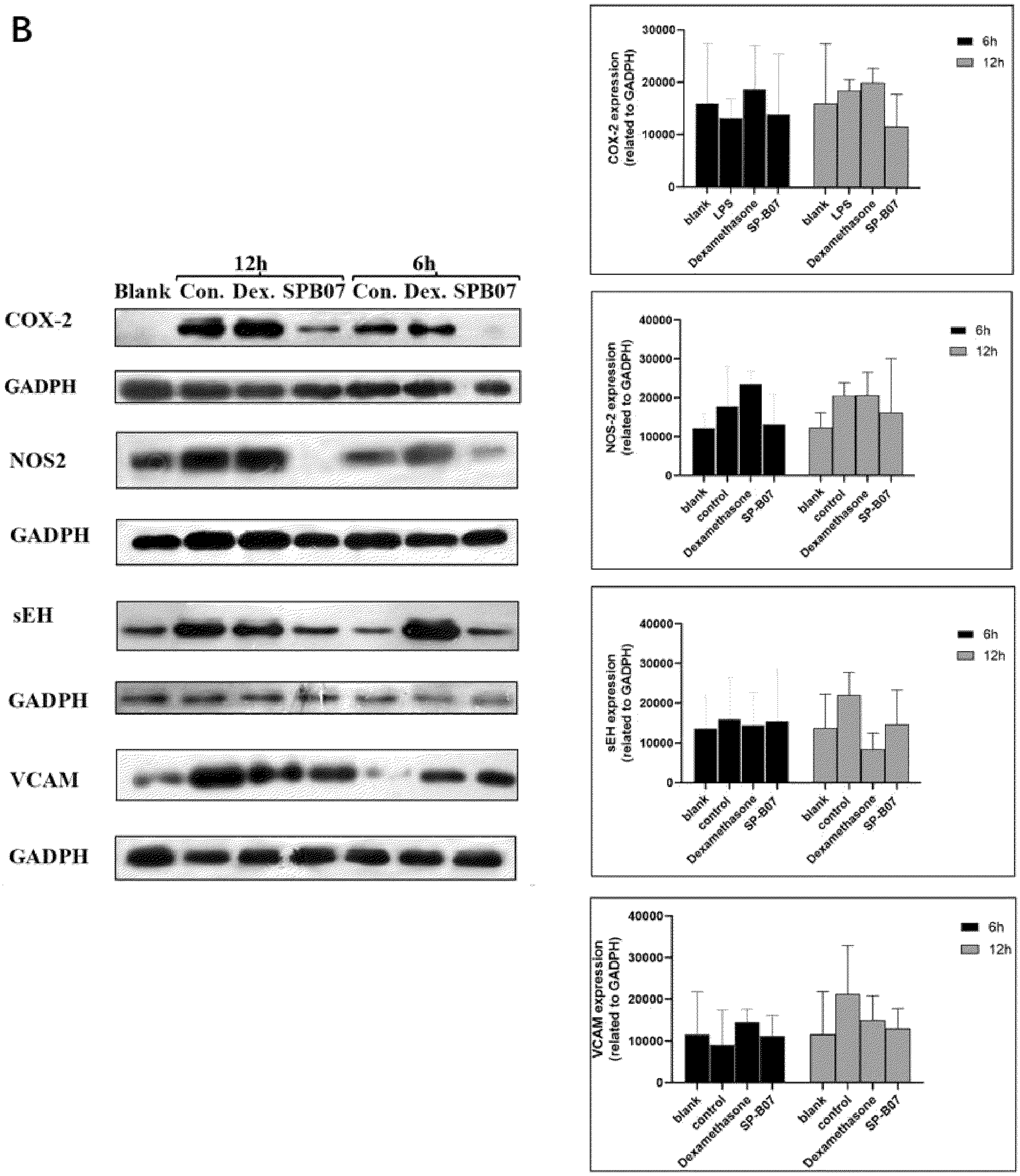
B
12h
6h
Blank Con. Dex. SPB07 Con. Dex. SPB07
COX-2
GADPH
NOS2
GADPH
sEH
GADPH
VCAM
GADPH
COX-2 expression (related to GADPH)
NOS-2 expression (related to GADPH)
sEH expression (related to GADPH)
VCAM expression (related to GADPH)
blank
LPS
control
Dexamethasone
SP-B07
6h
12h

FIG. 14

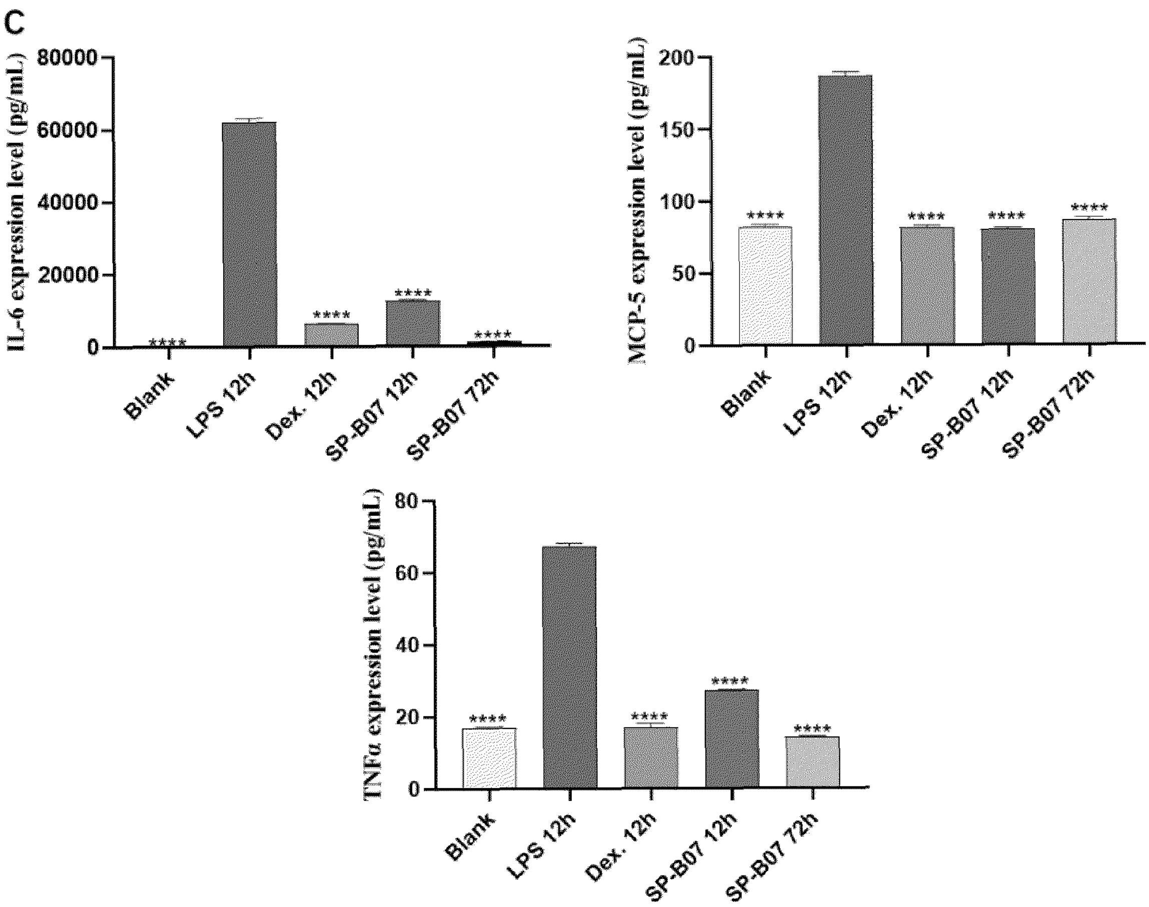
C
IL-6 expression level (pg/mL)
MCP-5 expression level (pg/mL)
TNFα expression level (pg/mL)
Blank
LPS 12h
Dex. 12h
SP-B07 12h
SP-B07 72h
****

FIG. 15

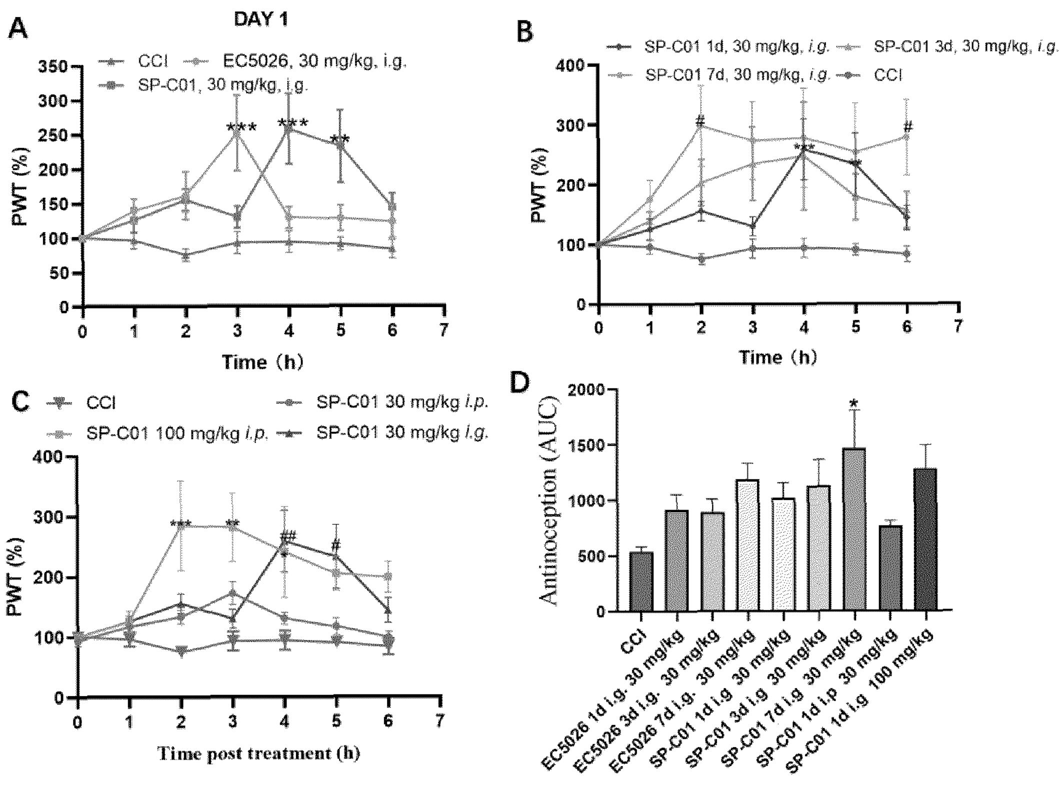
A
DAY 1
CCI
EC5026, 30 mg/kg, i.g.
SP-C01, 30 mg/kg, i.g.
PWT (%)
Time (h)
B
SP-C01 1d, 30 mg/kg, i.g.
SP-C01 3d, 30 mg/kg, i.g.
SP-C01 7d, 30 mg/kg, i.g.
CCI
C
SP-C01 30 mg/kg i.p.
SP-C01 100 mg/kg i.p.
SP-C01 30 mg/kg i.g.
Time post treatment (h)
D
Antinoception (AUC)
EC5026 1d i.g. 30 mg/kg
EC5026 3d i.g. 30 mg/kg
EC5026 7d i.g. 30 mg/kg
SP-C01 1d i.g. 30 mg/kg
SP-C01 3d i.g. 30 mg/kg
SP-C01 7d i.g. 30 mg/kg
SP-C01 1d i.p. 30 mg/kg
SP-C01 1d i.g. 100 mg/kg

FIG. 16

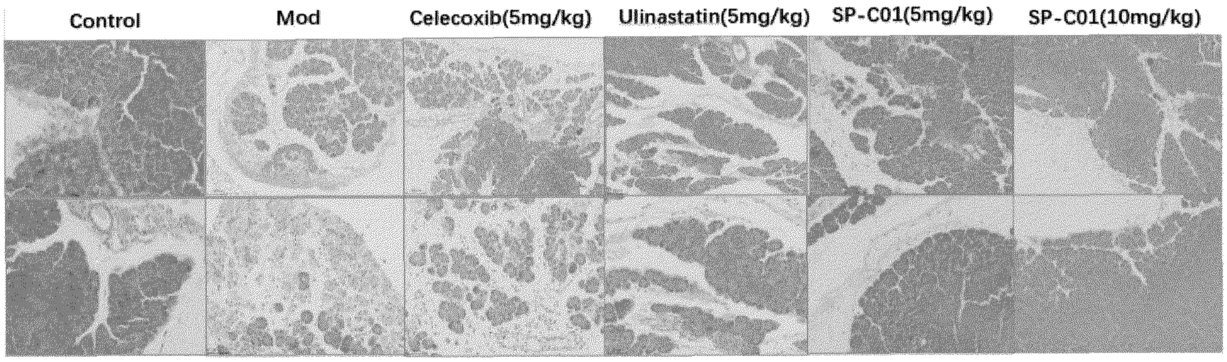
Control
Mod
Celecoxib(5mg/kg)
Ulinastatin(5mg/kg)
SP-C01(5mg/kg)
SP-C01(10mg/kg)

FIG. 17

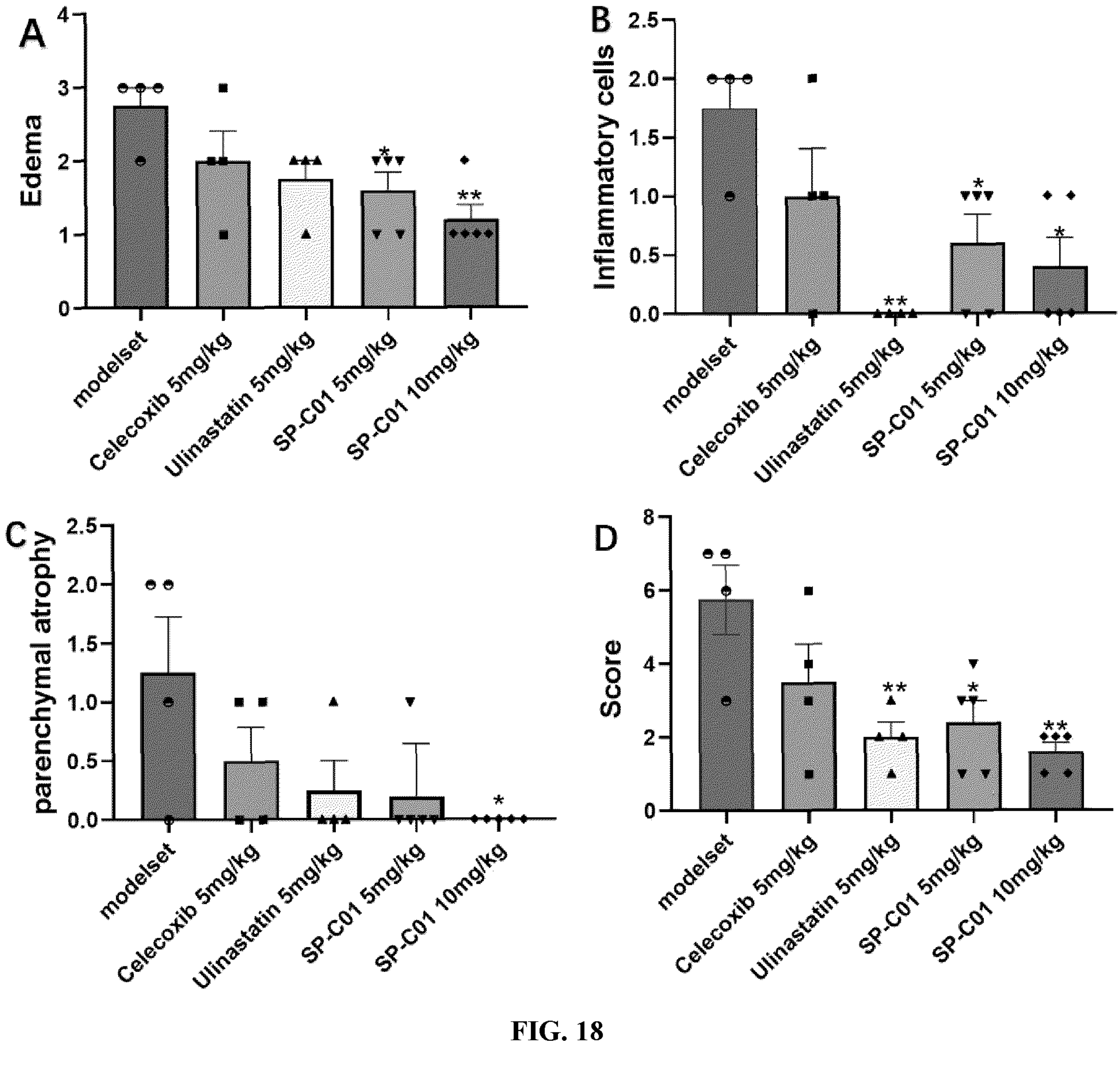
A
Edema
B
Inflammatory cells
C
parenchymal atrophy
D
Score
modelset
Celecoxib 5mg/kg
Ulinastatin 5mg/kg
SP-C01 5mg/kg
SP-C01 10mg/kg

FIG. 18

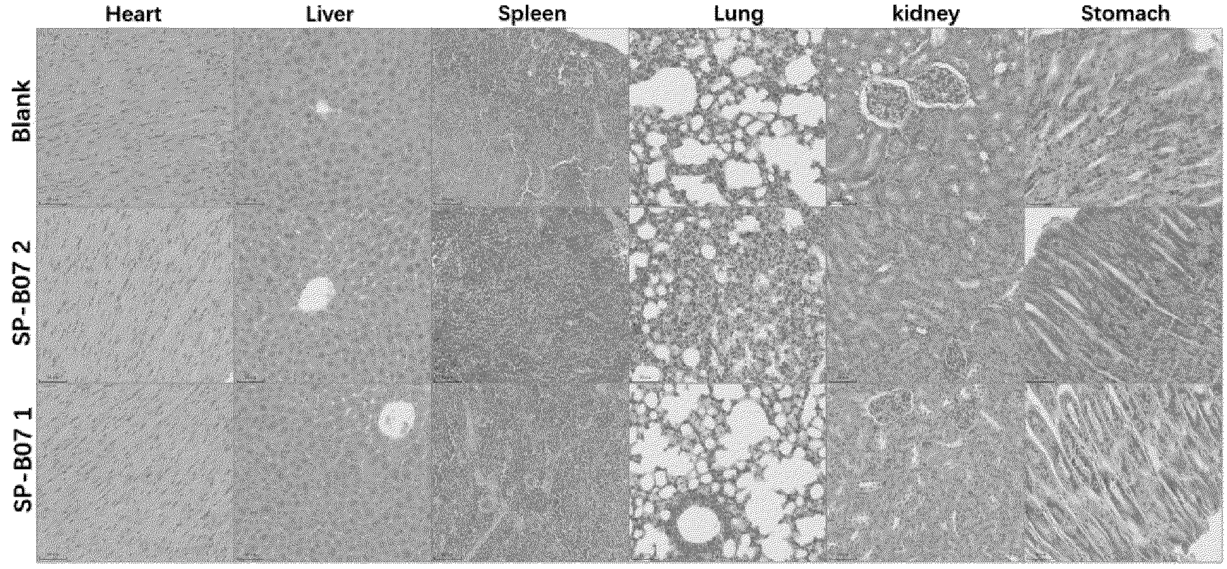


FIG. 19

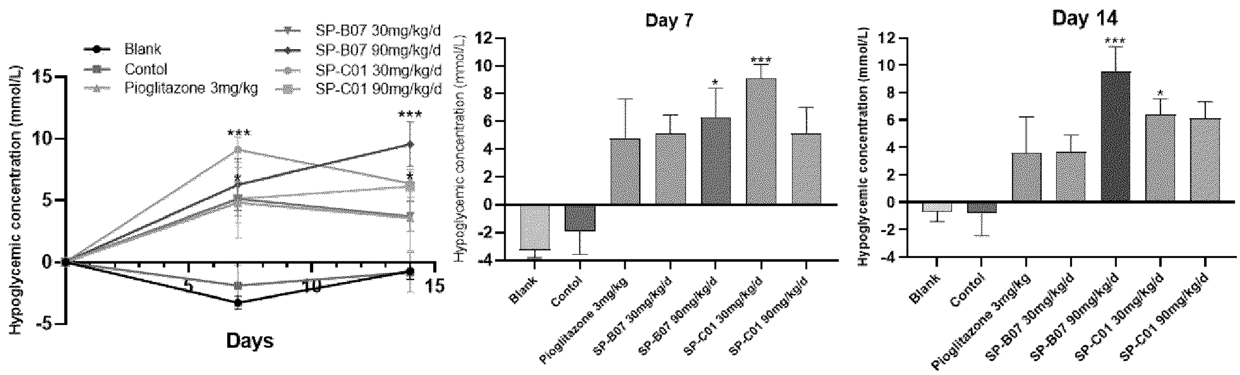


FIG. 20

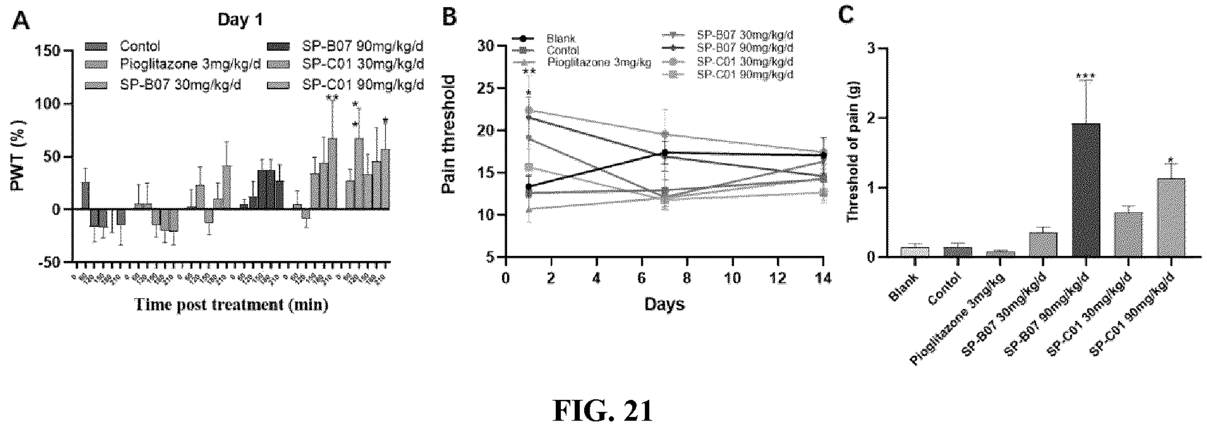


FIG. 21

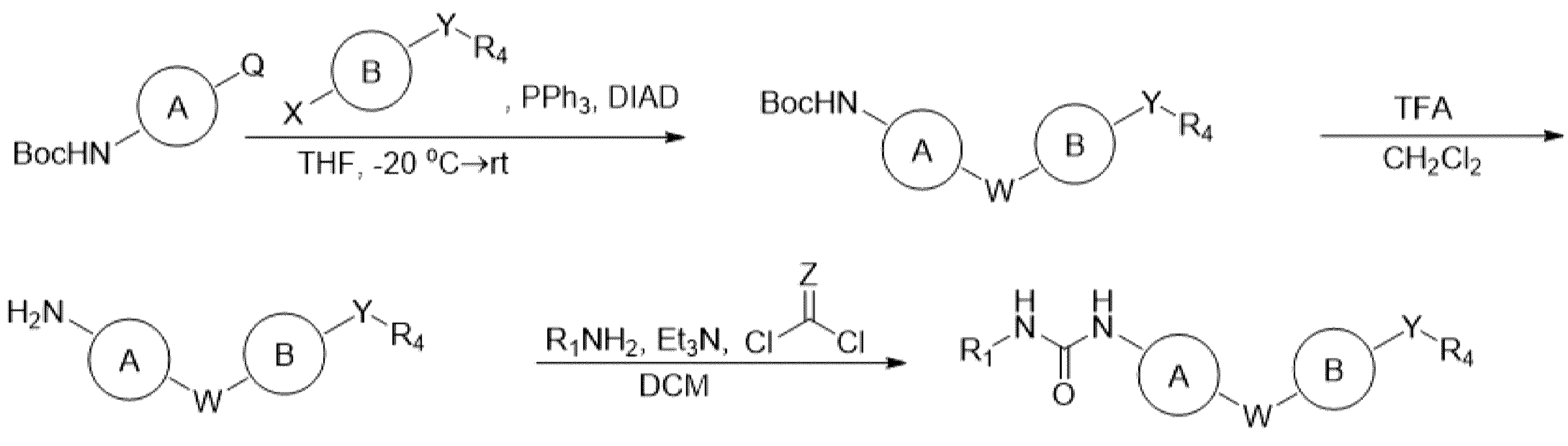


FIG. 22

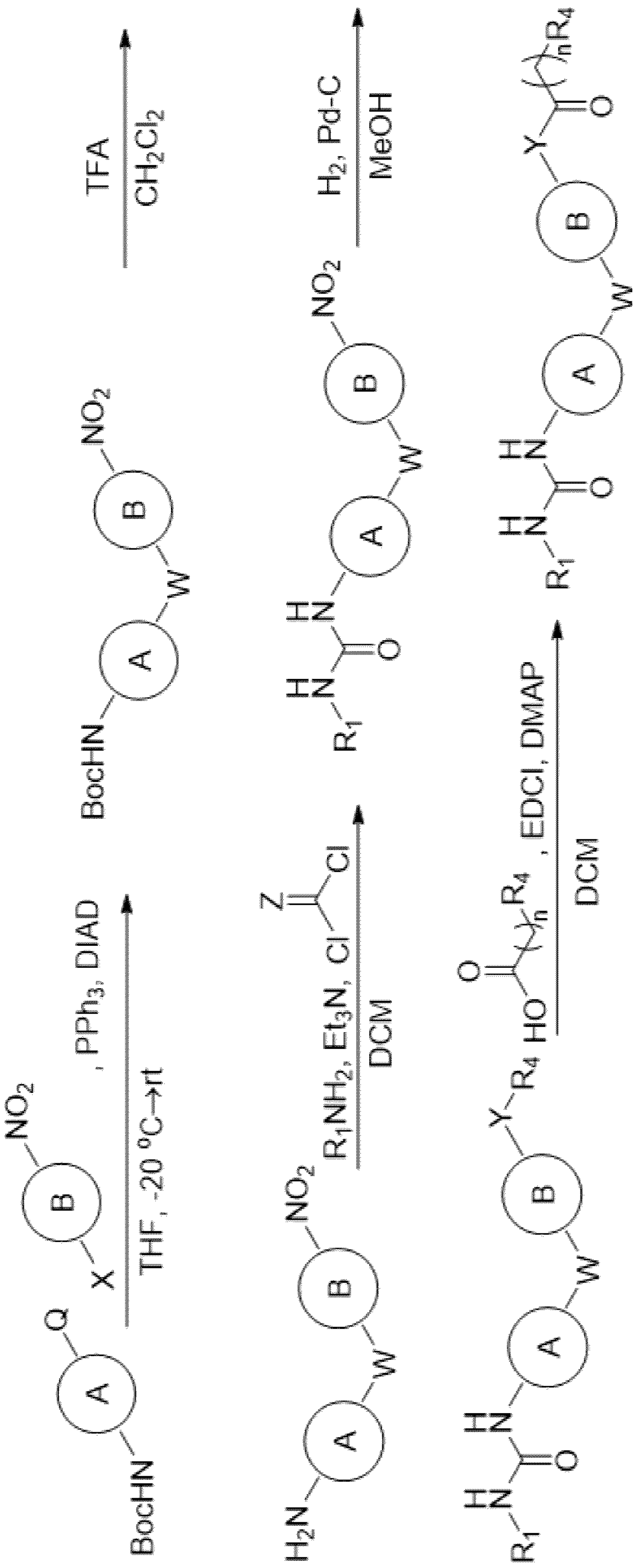
, PPh3, DIAD
THF, -20 °C→rt
TFA
CH2Cl2
R1NH2, Et3N,
DCM
H2, Pd-C
MeOH
, EDCI, DMAP
DCM

FIG. 23

**INTERNATIONAL SEARCH REPORT**

International application No.
**PCT/CN2023/139144**

**A. CLASSIFICATION OF SUBJECT MATTER**

C07D277/34(2006.01)i; C07D417/12(2006.01)i; C07D401/12(2006.01)i; C07C275/34(2006.01)i; C07C275/26(2006.01)i; C07C273/18(2006.01)i; A61P29/00(2006.01)i; A61P25/28(2006.01)i; A61P25/24(2006.01)i; A61P1/16(2006.01)i; A61P13/12(2006.01)i; A61P11/00(2006.01)i; A61P9/12(2006.01)i; A61P3/10(2006.01)i; A61P31/00(2006.01)i

According to International Patent Classification (IPC) or to both national classification and IPC

**B. FIELDS SEARCHED**

Minimum documentation searched (classification system followed by classification symbols)

IPC: C07D277/-, C07D417/-, C07D401/-, A61P/-

Documentation searched other than minimum documentation to the extent that such documents are included in the fields searched

Electronic data base consulted during the international search (name of data base and, where practicable, search terms used)

CNTXT; ENTXTC; VEN; CNKI; Caplus, REG: 脲, urea, sEH, PPAR, 根据权利要求1和6进行了结构式检索, structural formula search according to claims 1 and 6

**C. DOCUMENTS CONSIDERED TO BE RELEVANT**

| Category* | Citation of document, with indication, where appropriate, of the relevant passages | Relevant to claim No. |
|---|---|---|
| PX | CN 116217511 A (SHENYANG PHARMACEUTICAL UNIVERSITY) 06 June 2023 (2023-06-06)<br>claims 1-10 | 1-10, 12 |
| X | CN 1382129 A (KYORIN PHARMACEUTICAL CO., LTD.) 27 November 2002 (2002-11-27)<br>description, embodiments 11, 24 and 25, description, last paragraph, and claims 7 and 10-12 | 1-5, 7-10, 12 |
| X | CN 101068810 A (OTSUKA PHARMA CO., LTD.) 07 November 2007 (2007-11-07)<br>description, table 65, embodiment 226, and pages 153-155, and claim 26 | 1, 2, 12 |
| X | WO 0102394 A1 (GERON CORP. et al.) 11 January 2001 (2001-01-11)<br>description, embodiment 49, and claim 30 | 1, 2, 12 |
| X | WO 9741119 A1 (DR. REDDY'S RESEARCH FOUNDATION et al.) 06 November 1997 (1997-11-06)<br>description, embodiment 9, compound, and claims 1-15 | 1-3, 9, 10, 12 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 April 2024** | **03 April 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No. PCT/CN2023/139144

C. DOCUMENTS CONSIDERED TO BE RELEVANT

| Category* | Citation of document, with indication, where appropriate, of the relevant passages | Relevant to claim No. |
|---|---|---|
| X | WO 2017160861 A1 (THE UNIVERSITY OF CALIFORNIA) 21 September 2017 (2017-09-21) description, background art, compound t-TUCB, embodiments 10, 12 and 14, and table 3, compounds A-19, A-21 and A-23, and claims 29-36 | 1-10, 12 |
| X | WO 2012112570 A1 (UNIV CALIFORNIA et al.) 23 August 2012 (2012-08-23) description, table 1, compounds 1686 and 1728, and claims 1-15 | 1-10, 12 |
| X | WO 2022076881 A1 (UNIV CALIFORNIA et al.) 14 April 2022 (2022-04-14) description, tables 1 and 2, compound 1.003, and claims 1-16 | 1-6, 8-10, 12 |
| X | JP 2007291079 A (OTSUKA PHARMACEUTICAL CO., LTD.) 08 November 2007 (2007-11-08) description, table 65, embodiment 226, and claim 26 | 1, 2, 12 |
| X | BURMISTROV, Vladimir et al. "Effects of Adamantane Alterations on Soluble Epoxide Hydrolase Inhibition Potency, Physical Properties and Metabolic Stability" *Bioorganic Chemistry,* Vol. vol. 76, 30 December 2017 (2017-12-30), pages 510-527, table 5, compound 2b | 1-5, 8-10, 12 |
| X | Chemical Catalog. "CAS RN 2650912-17-5" *Aurora Fine Chemicals LLC,* 08 July 2021 (2021-07-08), STN Files: CHEMCATS | 1-5 |
| X | Chemical Catalog. "CAS RN 2650324-02-8" *Aurora Fine Chemicals LLC,* 07 July 2021 (2021-07-07), STN Files: CHEMCATS | 1-5 |

Form PCT/ISA/210 (second sheet) (July 2022)

**INTERNATIONAL SEARCH REPORT**

International application No. **PCT/CN2023/139144**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **11**
because they relate to subject matter not required to be searched by this Authority, namely:
Claim 11 relates to a method for treatment of the human or animal body by therapy. (PCT Rule 39.1(iv))

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The present application comprises the following six inventions:

(1) Claims 1-12 relate to a compound of formula I, a preparation method therefor, a use thereof, and a drug.

(2) Claims 1-12 relate to a compound of formula II, a preparation method therefor, a use thereof, and a drug.

(3) Claims 1-12 relate to a compound of formula III, a preparation method therefor, a use thereof, and a drug.

(4) Claims 1-12 relate to a compound of formula IV, a preparation method therefor, a use thereof, and a drug.

(5) Claims 1-12 relate to a compound of formula V, a preparation method therefor, a use thereof, and a drug.

(6) Claims 1-12 relate to a compound of formula VI, a preparation method therefor, a use thereof, and a drug.

The above listed inventions do not have a single general inventive concept, by means of which the inventions are linked with each other (PCT Rule 13.1), and therefore lack unity of invention. The reasoning therefor is as follows: $R_1$-$R_6$, A, B, W, Y and Z in general formula I-VI are all variable groups, and terminal groups connected to a B terminal or a Y segment are also different; and common structure -NH-C-N- cannot constitute a special technical feature that makes a contribution over the prior art, that is, the six groups of inventions do not have a same or corresponding special technical feature.

Form PCT/ISA/210 (continuation of first sheet) (July 2022)

**INTERNATIONAL SEARCH REPORT**

International application No.
**PCT/CN2023/139144**

**Box No. III Observations where unity of invention is lacking (Continuation of item 3 of first sheet)**

1. ☑ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☑ No protest accompanied the payment of additional search fees.

Form PCT/ISA/210 (continuation of first sheet) (July 2022)

**INTERNATIONAL SEARCH REPORT**
**Information on patent family members**

International application No.
**PCT/CN2023/139144**

| Patent document cited in search report | Publication date (day/month/year) | Patent family member(s) | Publication date (day/month/year) |
|---|---|---|---|
| CN 116217511 A | 06 June 2023 | None | |
| CN 1382129 A | 27 November 2002 | ATE 296294 T1 | 15 June 2005 |
| | | HUP 0202520 A2 | 28 November 2002 |
| | | HUP 0202520 A3 | 29 November 2004 |
| | | EP 1213287 A1 | 12 June 2002 |
| | | EP 1213287 A4 | 18 September 2002 |
| | | EP 1213287 B1 | 25 May 2005 |
| | | WO 0114349 A1 | 01 March 2001 |
| | | KR 20020040774 A | 30 May 2002 |
| | | KR 100738245 B1 | 12 July 2007 |
| | | CA 2382574 A1 | 01 March 2001 |
| | | US 6730687 B1 | 04 May 2004 |
| | | AU 6594500 A | 19 March 2001 |
| | | AU 778720 B2 | 16 December 2004 |
| | | DE 60020381 D1 | 30 June 2005 |
| | | DE 60020381 T2 | 26 January 2006 |
| CN 101068810 A | 07 November 2007 | BRPI 0516219 A | 26 August 2008 |
| | | EP 2426128 A1 | 07 March 2012 |
| | | ES 2393197 T3 | 19 December 2012 |
| | | ZA 200702228 B | 24 June 2009 |
| | | CA 2580811 A1 | 06 April 2006 |
| | | CA 2580811 C | 08 April 2014 |
| | | AR 055193 A1 | 08 August 2007 |
| | | EP 1797082 A1 | 20 June 2007 |
| | | EP 1797082 A4 | 23 September 2009 |
| | | EP 1797082 B1 | 29 August 2012 |
| | | WO 2006035954 A1 | 06 April 2006 |
| | | KR 20070061902 A | 14 June 2007 |
| | | KR 100823414 B1 | 17 April 2008 |
| | | US 2007179173 A1 | 02 August 2007 |
| | | US 7777038 B2 | 17 August 2010 |
| | | HK 1109901 A1 | 27 June 2008 |
| | | AU 2005288080 A1 | 06 April 2006 |
| | | AU 2005288080 B2 | 13 October 2011 |
| | | IL 181630 A0 | 04 July 2007 |
| | | IL 210756 A0 | 31 March 2011 |
| | | RU 2007116147 A | 10 November 2008 |
| | | RU 2430920 C2 | 10 October 2011 |
| | | US 2010261705 A1 | 14 October 2010 |
| | | US 8362252 B2 | 29 January 2013 |
| | | RU 2011123036 A | 20 December 2012 |
| | | TW 200616961 A | 01 June 2006 |
| | | TWI 353353 B | 01 December 2011 |
| | | KR 20070072632 A | 04 July 2007 |
| | | KR 100840465 B1 | 20 June 2008 |
| | | JP 3906471 B1 | 18 April 2007 |
| | | JP 2007512220 A | 17 May 2007 |
| | | AU 2011202454 A1 | 16 June 2011 |
| | | AU 2011202454 B2 | 08 March 2012 |
| | | MX 2007003735 A | 23 April 2007 |

Form PCT/ISA/210 (patent family annex) (July 2022)

**INTERNATIONAL SEARCH REPORT**
**Information on patent family members**

International application No.
**PCT/CN2023/139144**

| Patent document cited in search report | | | Publication date (day/month/year) | Patent family member(s) | | | Publication date (day/month/year) |
|---|---|---|---|---|---|---|---|
| WO | 0102394 | A1 | 11 January 2001 | EP | 1109808 | A1 | 27 June 2001 |
| | | | | US | 2002115700 | A1 | 22 August 2002 |
| | | | | AU | 6062900 | A | 22 January 2001 |
| | | | | US | 6372742 | B1 | 16 April 2002 |
| WO | 9741119 | A1 | 06 November 1997 | DE | 69727837 | D1 | 01 April 2004 |
| | | | | DE | 69727837 | T2 | 30 December 2004 |
| | | | | EP | 0981526 | A1 | 01 March 2000 |
| | | | | EP | 0981526 | B1 | 25 February 2004 |
| | | | | AU | 2930797 | A | 19 November 1997 |
| | | | | JP | 2001518069 | A | 09 October 2001 |
| | | | | CN | 1221415 | A | 30 June 1999 |
| WO | 2017160861 | A1 | 21 September 2017 | US | 2019077785 | A1 | 14 March 2019 |
| | | | | US | 10858338 | B2 | 08 December 2020 |
| | | | | US | 2021155601 | A1 | 27 May 2021 |
| | | | | US | 11873288 | B2 | 16 January 2024 |
| WO | 2012112570 | A1 | 23 August 2012 | EP | 2675274 | A1 | 25 December 2013 |
| | | | | EP | 2675274 | A4 | 06 August 2014 |
| | | | | EP | 2675274 | B1 | 03 May 2017 |
| | | | | CA | 2826277 | A1 | 23 August 2012 |
| | | | | US | 2014088156 | A1 | 27 March 2014 |
| | | | | US | 9029401 | B2 | 12 May 2015 |
| WO | 2022076881 | A1 | 14 April 2022 | None | | | |
| JP | 2007291079 | A | 08 November 2007 | JP | 5191155 | B2 | 24 April 2013 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310282598


### Non-patent literature cited in the description


- *Nature*, 2001, vol. 413 (6852), 203-210
- *Neurotherapeutics*, 2020, vol. 17, 900-916
- *Cell Physiol Biochem*, 2015, vol. 36, 474-486